# EUROPEAN PATENT APPLICATION

(11) **EP 3 556 215 A1**
(43) Date of publication of application: **23.10.2019**
(21) Application number: 19165534.9
(22) Date of filing: 13.12.2013
(51) Int. Cl.: A01N 63/00, C12N 15/00, C12N 5/02

(54) **ANOIKIS RESISTANT PLACENTAL STEM CELLS AND USES THEREOF**

(30) Priority: 14.12.2012 US 201261737498 P
(62) Divisional of application: 13862498.6
(71) Applicant: Celularity, Inc., Warren, New Jersey 07059 (US)
(72) Inventor: ZHANG, Xiaokui, Warren, NJ 07059 (US); ABBOT, Stewart, Warren, NJ 07059 (US); VOSKINARIAN-BERSE, Vanessa, Millington, NJ 07946 (US)
(74) Representative: Mewburn Ellis LLP

(57) **Abstract**

The invnetion provides an isolated placental stem cell, wherein said placental stem cell is resistant to anoikis, wherein said anokis resistant placental stem cell expresses at least one anoikis associated gene at a decreased level as compared to the expression of the same anoikis associated gene in an unmodified placental stem cell. The invention further provides an isolated population of said anoikis resistant cells as well as a method of producing said anoikis resistant cells. Further provided is a composition comprising said isolated anoikis resistant placental stem cells or cell populations.

## Description

This application claims priority to U.S. Provisional Patent Application No. 61/737,498, filed December 14, 2012, the disclosure of which is incorporated herein by reference in its entirety.

### 1. FIELD

Provided herein are anoikis-resistant placental cells and compositions thereof as well as methods of using such cells and compositions.

### 2. BACKGROUND

Because mammalian placentas are plentiful and are normally discarded as medical waste, they represent a unique source of medically-useful cells, e.g., placental stem cells. Placental stem cells, typically adhere (attach) to culture surfaces, such as tissue culture plates and extracellular matrix. Anoikis is a form of programmed cell death (apoptosis) that occurs in attachment-dependent cells when they are cultured/present in low attachment environments. There exists a need for populations of placental stem cells that are resistant to anoikis, and thus survive for longer periods of time in a non-adherent state. Provided herein are such improved placental stem cells, populations of such placental stem cells, and methods of using the same.

### 3. SUMMARY

In one aspect, provided herein is a method of modifying placental stem cells to make them anoikis resistant. The anoikis resistant placental stem cells (arPSCs) provided herein demonstrate increased survival in low-attachment environments, and thus can advantageously be used, e.g., in therapies that utilize administration of placental stem cells (e.g., systemic administration of placental stem cells) based on their ability to persist for longer durations of time in an unattached state, e.g., as compared to unmodified placental stem cells (e.g., placental stem cells that have not been modified to be anoikis resistant). In certain embodiments, placental stem cells are anoikis resistant if they are capable of surviving in conditions in which placental stem cells would normally undergo anoikis. In certain embodiments, placental stem cells are anoikis resistant if they are capable of surviving for a longer duration of time relative to unmodified placental stem cells in conditions in which placental stem cells would normally undergo anoikis.

In one embodiment, provided herein is a method of modifying placental stem cells to make them anoikis resistant, comprising contacting the placental stem cells with an effective amount of oligomeric or polymeric molecules, such that one or more genes associated with anoikis of the placental stem cells is inhibited (e.g., downregulated as compared to placental stem cells that have not been modified, *e*.*g*., that have not been contacted with said molecules). Such modified placental stem cells described herein are referred to herein as "anoikis resistant placental stem cells" ("arPSCs"). In certain embodiments, said oligomeric or polymeric molecules are modulatory RNA molecules. In specific embodiments, the modulatory RNA molecules are small interfering RNAs (siRNAs), microRNA inhibitors (miR inhibitors), miR mimics, antisense RNAs, small hairpin RNAs (shRNAs), microRNA-adapted shRNA (shRNAmirs), or any combination thereof.

In certain embodiments, the modulatory RNA molecules used in the methods described herein for generating arPSCs target one or more placental stem cell genes ("anoikis-associated genes") identified herein as being associated with anoikis in the placental stem cells. In a specific embodiment, said one or more anoikis-associated genes targeted in the methods described herein to produce arPSCs comprise one or more of the genes listed in Table 1, below:

**Table 1: Human Placental Stem Cell Anoikis Associated Genes**

| **Gene ID (NCBI)** | **Gene Symbol** | **Gene Description** |
|---|---|---|
| 57463 | AMIGO1 | adhesion molecule with Ig-like domain 1 |
| 57569 | ARHGAP20 | Rho GTPase activating protein 20 |
| 952 | CD38 | CD38 molecule |
| 23155 | CLCC1 | chloride channel CLIC-like 1 |
| 1270, 386607 | CNTF, ZFP91-CNTF | ciliary neurotrophic factor\| ZFP91 -CNTF readthrough transcript |
| 1351 | COX8A | cytochrome c oxidase subunit 8A (ubiquitous) |
| 9704 | DHX34 | DEAH (Asp-Glu-Ala-His) box polypeptide 34 |
| 51023, 84142 | FAM175A, MRPS18C | mitochondrial ribosomal protein S18C\| family with sequence similarity 175, member A |
| 284257 | FAM44C | family with sequence similarity 44, member C |
| 8789 | FBP2 | fructose-1,6-bisphosphatase 2 |
| 2313 | FLI1 | Friend leukemia virus integration 1 |
| 166752 | FREM3 | FRAS1 related extracellular matrix 3 |
| 24138 | IFIT5 | interferon-induced protein with tetratricopeptide repeats 5 |
| 399851 | LOC399851 | hypothetical gene supported by AY129010 |
| 400713 | LOC400713 | zinc finger-like |
| 651610 | LOC651610 | serine-protein kinase ATM-like |
| 51227 | PIGP | phosphatidylinositol glycan anchor biosynthesis, class P |
| 79628 | SH3TC2 | SH3 domain and tetratricopeptide repeats 2 |
| 6515 | SLC2A3 | solute carrier family 2 (facilitated glucose transporter), member 3 |
| 27067 | STAU2 | staufen, RNA binding protein, homolog 2 (Drosophila) |
| 8577 | TMEFF1 | transmembrane protein with EGF-like and two follistatin-like domains 1 |
| 221468 | TMEM217 | transmembrane protein 217 |
| 84283 | TMEM79 | transmembrane protein 79 |
| 83878 | USHBP1 | Usher syndrome 1C binding protein 1 |
| 83464 | APH1B | anterior pharynx defective 1 homolog B (C. elegans) |
| 491 | ATP2B2 | ATPase, Ca++ transporting, plasma membrane 2 |
| 196541 | C13orf39 | chromosome 13 open reading frame 39 |
| 84103 | C4orf17 | chromosome 4 open reading frame 17 |
| 201725 | C4orf46 | chromosome 4 open reading frame 46 |
| 51428 | DDX41 | DEAD (Asp-Glu-Ala-Asp) box polypeptide 41 |
| 84237 | DKFZp547J222 | hypothetical LOC84237 |
| 2260 | FGFR1 | fibroblast growth factor receptor 1 |
| 85462 | FHDC1 | FH2 domain containing 1 |
| 2771 | GNAI2 | guanine nucleotide binding protein (G protein), alpha inhibiting activity polypeptide 2 |
| 2814 | GP5 | glycoprotein V (platelet) |
| 3557 | IL1RN | interleukin 1 receptor antagonist |
| 347240 | KIF24 | kinesin family member 24 |
| 85442 | KNDC1 | kinase non-catalytic C-lobe domain (KIND) containing 1 |
| 100132598 | LOC100132598 | similar to hCG2001192 |
| 151760 | LOC151760 | hypothetical LOC151760 |
| 152024 | LOC152024 | hypothetical protein LOC152024 |
| 339833 | LOC339833 | hypothetical protein LOC339833 |
| 2846 | LPAR4 | lysophosphatidic acid receptor 4 |
| 55341 | LSG1 | large subunit GTPase 1 homolog (S. cerevisiae) |
| 4217 | MAP3K5 | mitogen-activated protein kinase kinase kinase 5 |
| 5165 | PDK3 | pyruvate dehydrogenase kinase, isozyme 3 |
| 57161 | PELI2 | pellino homolog 2 (Drosophila) |
| 7844 | RNF103 | ring finger protein 103 |
| 169166 | SNX31 | sorting nexin 31 |
| 25828 | TXN2 | thioredoxin 2 |
| 343702 | XKR7 | XK, Kell blood group complex subunit-related family, member 7 |

In one embodiment, the modulatory RNA molecules used in the methods described herein for generating arPSCs are small interfering RNAs (siRNAs). In a specific embodiment, said siRNAs target one or more of the anoikis-associated genes listed in Table 1, above. In another specific embodiment, said siRNAs are double-stranded, wherein one strand of said siRNAs have a sequence at least about 70%, 80%, 90%, 95%, 98% or 100% complementary to the sequence of one of the genes identified in Table 1, above (as identified based on the Gene ID of the gene provided in the table).

In another specific embodiment, the siRNAs used in the methods described herein for generating arPSCs target the placental stem cell anoikis associated gene FHDC1 (NCBI GENE ID NO:85462). In another specific embodiment, the siRNAs used in the methods described herein for generating arPSCs target the placental stem cell anoikis associated gene GNAI2 (NCBI GENE ID NO:2771). In another specific embodiment, the siRNAs used in the methods described herein for generating arPSCs target the placental stem cell anoikis associated gene KNDC1 (NCBI GENE ID NO:85442). In another specific embodiment, the siRNAs used in the methods described herein for generating arPSCs target the placental stem cell anoikis associated gene LPAR4 (NCBI GENE ID NO:2846). In another specific embodiment, the siRNAs used in the methods described herein for generating arPSCs target the placental stem cell anoikis associated gene MAP3K5 (NCBI GENE ID NO:4217). In another specific embodiment, the siRNAs used in the methods described herein for generating arPSCs target the placental stem cell anoikis associated gene SLC2A3 (NCBI GENE ID NO:6515). In another specific embodiment, the siRNAs used in the methods described herein for generating arPSCs target the placental stem cell anoikis associated gene STAU2 (NCBI GENE ID NO:27067).

In another specific embodiment, the siRNAs used in the methods described herein for generating arPSCs target one, two, three, or more of the following placental stem cell anoikis-associated genes: FHDC1 (NCBI GENE ID NO:85462), GNAI2 (NCBI GENE ID NO:2771), KNDC1 (NCBI GENE ID NO:85442), LPAR4 (NCBI GENE ID NO:2846), MAP3K5 (NCBI GENE ID NO:4217), SLC2A3 (NCBI GENE ID NO:6515), and STAU2 (NCBI GENE ID NO:27067). In another specific embodiment, the siRNAs used in the methods described herein for generating arPSCs target one, two, three, or more of the following placental stem cell anoikis-associated genes: FHDC1 (NCBI GENE ID NO:85462), GNAI2 (NCBI GENE ID NO:2771), KNDC1 (NCBI GENE ID NO:85442), LPAR4 (NCBI GENE ID NO:2846), MAP3K5 (NCBI GENE ID NO:4217), SLC2A3 (NCBI GENE ID NO:6515), and STAU2 (NCBI GENE ID NO:27067), and target at least one additional anoikis associated gene recited in Table 1.

In another embodiment, the modulatory RNA molecules used in the methods described herein for generating arPSCs are small hairpin RNAs (shRNAs). In a specific embodiment, said shRNAs target one or more of the anoikis-associated genes listed in Table 1, above. In another specific embodiment, said shRNAs have a sequence at least about 70%, 80%, 90%, 95%, 98% or 100% complementary to the sequence of one of the genes identified in Table 1, above (as identified based on the Gene ID of the gene provided in the table).

In another specific embodiment, the shRNAs used in the methods described herein for generating arPSCs target the placental stem cell anoikis associated gene FHDC1 (NCBI GENE ID NO:85462). In another specific embodiment, the shRNAs used in the methods described herein for generating arPSCs target the placental stem cell anoikis associated gene GNAI2 (NCBI GENE ID NO:2771). In another specific embodiment, the shRNAs used in the methods described herein for generating arPSCs target the placental stem cell anoikis associated gene KNDC1 (NCBI GENE ID NO:85442). In another specific embodiment, the shRNAs used in the methods described herein for generating arPSCs target the placental stem cell anoikis associated gene LPAR4 (NCBI GENE ID NO:2846). In another specific embodiment, the shRNAs used in the methods described herein for generating arPSCs target the placental stem cell anoikis associated gene MAP3K5 (NCBI GENE ID NO:4217). In another specific embodiment, the shRNAs used in the methods described herein for generating arPSCs target the placental stem cell anoikis associated gene SLC2A3 (NCBI GENE ID NO:6515). In another specific embodiment, the shRNAs used in the methods described herein for generating arPSCs target the placental stem cell anoikis associated gene STAU2 (NCBI GENE ID NO:27067).

In another specific embodiment, the shRNAs used in the methods described herein for generating arPSCs target one, two, three, or more of the following placental stem cell anoikis-associated genes: FHDC1 (NCBI GENE ID NO:85462), GNAI2 (NCBI GENE ID NO:2771), KNDC1 (NCBI GENE ID NO:85442), LPAR4 (NCBI GENE ID NO:2846), MAP3K5 (NCBI GENE ID NO:4217), SLC2A3 (NCBI GENE ID NO:6515), and STAU2 (NCBI GENE ID NO:27067). In another specific embodiment, the shRNAs used in the methods described herein for generating arPSCs target one, two, three, or more of the following placental stem cell anoikis-associated genes: FHDC1 (NCBI GENE ID NO:85462), GNAI2 (NCBI GENE ID NO:2771), KNDC1 (NCBI GENE ID NO:85442), LPAR4 (NCBI GENE ID NO:2846), MAP3K5 (NCBI GENE ID NO:4217), SLC2A3 (NCBI GENE ID NO:6515), and STAU2 (NCBI GENE ID NO:27067), and target at least one additional anoikis associated gene recited in Table 1.

In another embodiment, the modulatory RNA molecules used in the methods described herein for generating arPSCs are antisense RNAs. In a specific embodiment, said antisense RNAs target one or more of the anoikis-associated genes listed in Table 1, above. In another specific embodiment, said antisense RNAs have a sequence at least about 70%, 80%, 90%, 95%, 98% or 100% complementary to the sequence of one of the genes identified in Table 1, above (as identified based on the Gene ID of the gene provided in the table).

In another specific embodiment, the antisense RNAs used in the methods described herein for generating arPSCs target the placental stem cell anoikis associated gene FHDC1 (NCBI GENE ID NO:85462). In another specific embodiment, the antisense RNAs used in the methods described herein for generating arPSCs target the placental stem cell anoikis associated gene GNAI2 (NCBI GENE ID NO:2771). In another specific embodiment, the antisense RNAs used in the methods described herein for generating arPSCs target the placental stem cell anoikis associated gene KNDC1 (NCBI GENE ID NO:85442). In another specific embodiment, the antisense RNAs used in the methods described herein for generating arPSCs target the placental stem cell anoikis associated gene LPAR4 (NCBI GENE ID NO:2846). In another specific embodiment, the antisense RNAs used in the methods described herein for generating arPSCs target the placental stem cell anoikis associated gene MAP3K5 (NCBI GENE ID NO:4217). In another specific embodiment, the antisense RNAs used in the methods described herein for generating arPSCs target the placental stem cell anoikis associated gene SLC2A3 (NCBI GENE ID NO:6515). In another specific embodiment, the antisense RNAs used in the methods described herein for generating arPSCs target the placental stem cell anoikis associated gene STAU2 (NCBI GENE ID NO:27067).

In another specific embodiment, the antisense RNAs used in the methods described herein for generating arPSCs target one, two, three, or more of the following placental stem cell anoikis-associated genes: FHDC1 (NCBI GENE ID NO:85462), GNAI2 (NCBI GENE ID NO:2771), KNDC1 (NCBI GENE ID NO:85442), LPAR4 (NCBI GENE ID NO:2846), MAP3K5 (NCBI GENE ID NO:4217), SLC2A3 (NCBI GENE ID NO:6515), and STAU2 (NCBI GENE ID NO:27067). In another specific embodiment, the antisense RNAs used in the methods described herein for generating arPSCs target one, two, three, or more of the following placental stem cell anoikis-associated genes: FHDC1 (NCBI GENE ID NO:85462), GNAI2 (NCBI GENE ID NO:2771), KNDC1 (NCBI GENE ID NO:85442), LPAR4 (NCBI GENE ID NO:2846), MAP3K5 (NCBI GENE ID NO:4217), SLC2A3 (NCBI GENE ID NO:6515), and STAU2 (NCBI GENE ID NO:27067), and target at least one additional anoikis associated gene recited in Table 1.

In another embodiment, the modulatory RNA molecules used in the methods described herein for generating arPSCs target one or more microRNAs (miRNAs) in placental cells that act to modulate the production of one or more anoikis-associated genes. In one embodiment, said modulatory RNA molecules are miR inhibitors. In another embodiment, said modulatory RNA molecules are miR mimics. In a specific embodiment, the miRNA targeted is an miRNA that modulates one or more of the anoikis-associated genes listed in Table 1, above. In certain embodiments, said miR inhibitors or said miR mimics have a sequence at least about 70%, 80%, 90%, 95%, 98% or 100% complementary to the sequence an miRNA that modulates the production of one of the genes identified in Table 1.

In another specific embodiment, the miR inhibitors or miR mimics used in the methods described herein for generating arPSCs target a miRNA that modulates the production of the placental stem cell anoikis associated gene FHDC1 (NCBI GENE ID NO:85462). In another specific embodiment, the miR inhibitors or miR mimics used in the methods described herein for generating arPSCs target a miRNA that modulates the production of the placental stem cell anoikis associated gene GNAI2 (NCBI GENE ID NO:2771). In another specific embodiment, the miR inhibitors or miR mimics used in the methods described herein for generating arPSCs target a miRNA that modulates the production of the placental stem cell anoikis associated gene KNDC1 (NCBI GENE ID NO:85442). In another specific embodiment, the miR inhibitors or miR mimics used in the methods described herein for generating arPSCs target a miRNA that modulates the production of the placental stem cell anoikis associated gene LPAR4 (NCBI GENE ID NO:2846). In another specific embodiment, the miR inhibitors or miR mimics used in the methods described herein for generating arPSCs target a miRNA that modulates the production of the placental stem cell anoikis associated gene MAP3K5 (NCBI GENE ID NO:4217). In another specific embodiment, the miR inhibitors or miR mimics used in the methods described herein for generating arPSCs target a miRNA that modulates the production of the placental stem cell anoikis associated gene SLC2A3 (NCBI GENE ID NO:6515). In another specific embodiment, the miR inhibitors or miR mimics used in the methods described herein for generating arPSCs target a miRNA that modulates the production of the placental stem cell anoikis associated gene STAU2 (NCBI GENE ID NO:27067).

In another specific embodiment, the miR inhibitors or miR mimics used in the methods described herein for generating arPSCs target one, two, three, or more miRNAs, wherein said miRNAs modulate the production of one, two, three, or more of the following placental stem cell anoikis-associated genes: FHDC1 (NCBI GENE ID NO:85462), GNAI2 (NCBI GENE ID NO:2771), KNDC1 (NCBI GENE ID NO:85442), LPAR4 (NCBI GENE ID NO:2846), MAP3K5 (NCBI GENE ID NO:4217), SLC2A3 (NCBI GENE ID NO:6515), and STAU2 (NCBI GENE ID NO:27067). In another specific embodiment, the miR inhibitors or miR mimics used in the methods described herein for generating arPSCs target one, two, three, or more miRNAs, wherein said miRNAs modulate the production of one, two, three, or more of the following placental stem cell anoikis-associated genes: FHDC1 (NCBI GENE ID NO:85462), GNAI2 (NCBI GENE ID NO:2771), KNDC1 (NCBI GENE ID NO:85442), LPAR4 (NCBI GENE ID NO:2846), MAP3K5 (NCBI GENE ID NO:4217), SLC2A3 (NCBI GENE ID NO:6515), and STAU2 (NCBI GENE ID NO:27067), and target at least one miRNA that modulates the production of at least one additional anoikis associated gene recited in Table 1.

In another aspect, provided herein are isolated anoikis resistant placental stem cells (arPSCs), and compositions thereof, produced according to the methods described herein, e.g., placental stem cells that have been modified by contacting said placental stem cells with an effective amount of oligomeric or polymeric molecules (*e*.*g*., modulatory RNA molecules), to render them anoikis resistant. Such anoikis resistant placental stem cells demonstrate increased survival in low-attachment environments as compared to, e.g., unmodified placental stem cells (e.g., placental stem cells that have not been contacted with an effective amount of oligomeric or polymeric molecules (*e*.*g*., modulatory RNA molecules)).

In one embodiment, the isolated arPSCs provided herein express at least one anoikis associated gene at a decreased level as compared to the expression of the same anoikis associated gene in an unmodified placental stem cell. In a specific embodiment, provided herein is an isolated arPSC, or population thereof, wherein said isolated arPSC expresses at least one gene from those listed in Table 1 at a decreased level as compared to the expression of the same anoikis associated gene in an unmodified placental stem cell. In another specific embodiment, provided herein is an isolated arPSC, or population thereof, wherein said isolated arPSC expresses at more than one gene from those listed in Table 1 at a decreased level as compared to the expression of the same anoikis associated gene in an unmodified placental stem cell, e.g., the isolated arPSC expresses, two, three, four, five, six, seven, eight, nine, ten, or greater than ten genes from those listed in Table 1 at a decreased level as compared to the expression of the same anoikis associated gene in an unmodified placental stem cell.

In another specific embodiment, provided herein is an isolated arPSC, wherein said arPSC expresses the anoikis associated gene FHDC1 (NCBI GENE ID NO:85462) at a decreased level as compared to the expression of the anoikis associated gene FHDC1 (NCBI GENE ID NO:85462) in an unmodified placental stem cell. In another specific embodiment, provided herein is an isolated arPSC, wherein said arPSC expresses the anoikis associated gene GNAI2 (NCBI GENE ID NO:2771) at a decreased level as compared to the expression of the anoikis associated gene GNAI2 (NCBI GENE ID NO:2771) in an unmodified placental stem cell. In another specific embodiment, provided herein is an isolated arPSC, wherein said arPSC expresses the anoikis associated gene KNDC1 (NCBI GENE ID NO:85442) at a decreased level as compared to the expression of the anoikis associated gene KNDC1 (NCBI GENE ID NO:85442) in an unmodified placental stem cell. In another specific embodiment, provided herein is an isolated arPSC, wherein said arPSC expresses the anoikis associated gene LPAR4 (NCBI GENE ID NO:2846) at a decreased level as compared to the expression of the anoikis associated gene LPAR4 (NCBI GENE ID NO:2846) in an unmodified placental stem cell. In another specific embodiment, provided herein is an isolated arPSC, wherein said arPSC expresses the anoikis associated gene MAP3K5 (NCBI GENE ID NO:4217) at a decreased level as compared to the expression of the anoikis associated gene MAP3K5 (NCBI GENE ID NO:4217) in an unmodified placental stem cell. In another specific embodiment, provided herein is an isolated arPSC, wherein said arPSC expresses the anoikis associated gene SLC2A3 (NCBI GENE ID NO:6515) at a decreased level as compared to the expression of the anoikis associated gene SLC2A3 (NCBI GENE ID NO:6515) in an unmodified placental stem cell. In another specific embodiment, provided herein is an isolated arPSC, wherein said arPSC expresses the anoikis associated gene STAU2 (NCBI GENE ID NO:27067) at a decreased level as compared to the expression of the anoikis associated gene STAU2 (NCBI GENE ID NO:27067) in an unmodified placental stem cell. Further provided herein are populations of cells comprising such arPSCs and compositions comprising such arPSCs.

In another specific embodiment, provided herein is an isolated arPSC, wherein said arPSC expresses one, two, three, or more of the following placental stem cell anoikis-associated genes at a decreased level as compared to the expression of the same anoikis associated gene(s) in an unmodified placental stem cell: FHDC1 (NCBI GENE ID NO:85462), GNAI2 (NCBI GENE ID NO:2771), KNDC1 (NCBI GENE ID NO:85442), LPAR4 (NCBI GENE ID NO:2846), MAP3K5 (NCBI GENE ID NO:4217), SLC2A3 (NCBI GENE ID NO:6515), and STAU2 (NCBI GENE ID NO:27067). In another specific embodiment, provided herein is an isolated arPSC, wherein said arPSC (i) expresses one, two, three, or more of the following placental stem cell anoikis-associated genes at a decreased level as compared to the expression of the same anoikis associated gene(s) in an unmodified placental stem cell: FHDC1 (NCBI GENE ID NO:85462), GNAI2 (NCBI GENE ID NO:2771), KNDC1 (NCBI GENE ID NO:85442), LPAR4 (NCBI GENE ID NO:2846), MAP3K5 (NCBI GENE ID NO:4217), SLC2A3 (NCBI GENE ID NO:6515), and STAU2 (NCBI GENE ID NO:27067); and (ii) expresses at least one additional anoikis associated gene recited in Table 1 at a decreased level as compared to the expression of the same anoikis associated gene(s) in an unmodified placental stem cell. Further provided herein are populations of cells comprising such arPSCs and compositions comprising such arPSCs.

In a specific embodiment, the arPSCs described herein are CD10⁺, CD34⁻, CD105⁺, and CD200⁺. In another specific embodiment, the arPSCs described herein express CD200 and do not express HLA-G; or express CD73, CD105, and CD200; or express CD200 and OCT-4; or express CD73 and CD105 and do not express HLA-G; or express CD73 and CD105 and facilitate the formation of one or more embryoid-like bodies in a population of placental cells comprising said stem cell when said population is cultured under conditions that allow for the formation of an embryoid-like body; or express OCT-4 and facilitate the formation of one or more embryoid-like bodies in a population of placental cells comprising said stem cell when said population is cultured under conditions that allow for the formation of an embryoid-like body. In another specific embodiment, the arPSCs described herein are additionally CD90⁺ and CD45⁻. In another specific embodiment, the arPSCs described herein are additionally CD80⁻ and CD86⁻. In yet other embodiments, the arPSCs described herein express one or more of CD44, CD90, HLA-A,B,C or ABC-p, and/or do not express one or more of CD45, CD117, CD133, KDR, CD80, CD86, HLA-DR, SSEA3, SSEA4, or CD38. In certain embodiments, the arPSCs described herein suppress the activity of an immune cell, *e*.*g*., suppress proliferation of a T cell to a detectably greater degree than unmodified placental stem cells (e.g., placental cells that have not been contacted with an effective amount of oligomeric or polymeric molecules (*e*.*g*., modulatory RNA molecules)), as determinable by, e.g., a mixed leukocyte reaction assay, regression assay, or bead T cell assay.

In another aspect, provided herein is a method for an immune response, e.g., modulating the immune response of a subject, e.g., a human subject, or modulating an immune response in vitro, comprising contacting immune cells with the arPSCs described herein, or a composition thereof. In a specific embodiment, the arPSCs provided herein are capable of modulating an immune response to the same degree as an equivalent amount of unmodified placental stem cells (e.g., placental stem cells that are not resistant to anoikis). Assays for measuring the ability of cells (e.g., placental stem cells, including arPSCs) to modulate an immune response are known in the art (see, e.g., U.S. Patent No. 7,682,803, the disclosure of which is herein incorporated by reference in its entirety) and described herein, e.g., mixed lymphocyte reaction, regression assay.

In another aspect, provided herein is a method for promoting angiogenesis. In a specific embodiment, provided herein is a method for promoting angiogenesis in a subject, e.g., a human subject, comprising administering to said subject the arPSCs described herein, or a composition thereof. In another specific embodiment, the arPSCs provided herein are capable of promoting angiogenesis to the same degree as an equivalent amount of unmodified placental stem cells (e.g., placental stem cells that are not resistant to anoikis). Assays for measuring the ability of cells (e.g., placental stem cells, including arPSCs) to promote angiogenesis are known in the art (see, e.g., U.S. Patent Application Publication No. 2011/0250182, the disclosure of which is herein incorporated by reference in its entirety), e.g., assaying the ability of cells to promote tube formation by endothelial cells, assaying the ability of cells to promote endothelial cell migration and/or proliferation, and assaying the ability of cells to secrete factors that promote angiogenesis.

### 3.1 DEFINITIONS

As used herein, the term "amount," when referring to placental stem cells, *e*.*g*., anoikis resistant placental stem cells described herein, means a particular number of placental stem cells (e.g., anoikis resistant placental stem cells).

As used herein, the term "derived" means isolated from or otherwise purified. For example, placental derived adherent cells are isolated from placenta. The term "derived" encompasses cells that are cultured from cells isolated directly from a tissue, *e*.*g*., the placenta, and cells cultured or expanded from primary isolates.

As used herein, "immunolocalization" means the detection of a compound, *e*.*g*., a cellular marker, using an immune protein, *e*.*g*., an antibody or fragment thereof in, for example, flow cytometry, fluorescence-activated cell sorting, magnetic cell sorting, *in situ* hybridization, immunohistochemistry, or the like.

As used herein, the term "SH2" refers to an antibody that binds an epitope on the marker CD105. Thus, cells that are referred to as SH2⁺ are CD105⁺.

As used herein, the terms "SH3" and SH4" refer to antibodies that bind epitopes present on the marker CD73. Thus, cells that are referred to as SH3⁺ and/or SH4⁺ are CD73⁺.

As used herein, a stem cell is "isolated" if at least 50%, 60%, 70%, 80%, 90%, 95%, or at least 99% of the other cells with which the stem cell is naturally associated are removed from the stem cell, *e*.*g*., during collection and/or culture of the stem cell. A population of "isolated" cells means a population of cells that is substantially separated from other cells of the tissue, *e*.*g*., placenta, from which the population of cells is derived. In some embodiments, a population of, *e*.*g*., stem cells is "isolated" if at least 50%, 60%, 70%, 80%, 90%, 95%, or at least 99% of the cells with which the population of stem cells are naturally associated are removed from the population of stem cells, *e*.*g*., during collection and/or culture of the population of stem cells.

As used herein, the term "placental stem cell" refers to a stem cell or progenitor cell that is derived from, *e*.*g*., isolated from, a mammalian placenta, regardless of the number of passages after a primary culture, which adheres to a tissue culture substrate (*e*.*g*., tissue culture plastic or a fibronectin-coated tissue culture plate) in its unmodified state. The term "placental stem cell" as used herein does not, however, refer to a trophoblast, a cytotrophoblast, embryonic germ cell, or embryonic stem cell, as those cells are understood by persons of skill in the art. The terms "placental stem cell" and "placenta-derived stem cell" may be used interchangeably. Unless otherwise noted herein, the term "placental" includes the umbilical cord. The placental stem cells disclosed herein are, in certain embodiments, multipotent *in vitro* (that is, the cells differentiate *in vitro* under differentiating conditions), multipotent *in vivo* (that is, the cells differentiate *in vivo*), or both.

As used herein, a cell is "positive" for a particular marker when that marker is detectable. For example, a placental stem cell is positive for, *e*.*g*., CD73 because CD73 is detectable on placental stem cells in an amount detectably greater than background (in comparison to, *e*.*g*., an isotype control or an experimental negative control for any given assay). A cell is also positive for a marker when that marker can be used to distinguish the cell from at least one other cell type, or can be used to select or isolate the cell when present or expressed by the cell.

As used herein, the term "stem cell" defines a cell that retains at least one attribute of a stem cell, *e*.*g*., a marker or gene expression profile associated with one or more types of stem cells; the ability to replicate at least 10-40 times in culture; multipotency, *e*.*g*., the ability to differentiate, either *in vitro*, *in vivo* or both, into cells of one or more of the three germ layers; the lack of adult (*i*.*e*., differentiated) cell characteristics, or the like.

As used herein, "immunomodulation" and "immunomodulatory" mean causing, or having the capacity to cause, a detectable change in an immune response, and the ability to cause a detectable change in an immune response.

As used herein, "immunosuppression" and "immunosuppressive" mean causing, or having the capacity to cause, a detectable reduction in an immune response, and the ability to cause a detectable suppression of an immune response.

As used herein, the term "oligomeric or polymeric molecule" refers to a biomolecule that is capable of targeting a gene, RNA, or protein of interest (*e*.*g*., by binding or hybridizing to a region of a gene, RNA, or protein of interest). This term includes, for example, oligonucleotides, oligonucleosides, oligonucleotide analogs, oligonucleotide mimetics, oligopeptides or polypeptides, and any combinations (*e*.*g*., chimeric combinations) thereof. As such, these compounds may be single-stranded, double-stranded, circular, branched or have hairpins and can comprise structural elements such as internal or terminal bulges or loops. Oligomeric or polymeric double-stranded molecules can be two strands hybridized to form double-stranded compounds or a single strand with sufficient self complementarity to allow for hybridization and formation of a fully or partially double-stranded molecule.

As used herein, the term "modulatory RNA molecule" refers to an RNA molecule that modulates, (*e*.*g*., up-regulates or down-regulates) directly or indirectly, the expression or activity of the selectable target(s) (*e*.*g*., a target gene, RNA, or protein). In certain embodiments, a "modulatory RNA molecule" is a siRNA, miR inhibitor, miR mimic, antisense RNA, shRNA, shRNAmir, or a hybrid or a combination thereof that modulates the expression of the selectable target in a host cell. In certain embodiments, the modulatory RNA molecules provided herein comprise about 1 to about 100, from about 8 to about 80, 10 to 50, 13 to 80, 13 to 50, 13 to 30, 13 to 24, 18 to 22, 19 to 23, 20 to 80, 20 to 50, 20 to 30, or 20 to 24 nucleobases (*i.e.* from about 1 to about 100 linked nucleosides).

As used herein, the phrase "increased survival," when describing the survival of anoikis resistant placental stem cells as compared to unmodified placental stem cells refers to the ability of the anoikis resistant placental stem cells to remain viable under conditions that cause the death (e.g., by apoptosis) of unmodified placental stem cells, e.g., conditions wherein the placental stem cells cannot adhere to a substrate (e.g., a tissue culture plate or a biological substrate such as extracellular matrix) or have a diminished ability to adhere to a substrate, i.e., low-attachment conditions. In certain embodiments, increased survival of the arPSCs described herein relative to unmodified placental stem cells refers to the ability of the arPSCs to exhibit at least a 1.5-fold, 2-fold, 2.5-fold, 3-fold, 3.5-fold, 4-fold, 4.5-fold, 5-fold, 6-fold, 7-fold, 8-fold, 9-fold, or 10-fold increase in survival time when cultured under low-attachment conditions relative to an equivalent amount of unmodified placental stem cells cultured under the same conditions. In certain embodiments, increased survival of the arPSCs described herein relative to unmodified placental stem cells refers to the ability of the arPSCs to exhibit at least a 1.5-fold to 2.5-fold, a 2-fold to 3-fold, a 2.5-fold to 3.5-fold, a 3-fold to 4-fold, a 3.5-fold to 4.5-fold, a 4-fold to 5-fold, a 5-fold to 6-fold, a 6-fold to 7-fold, a 7-fold to 8-fold, an 8-fold to 9-fold, or a 9-fold to 10-fold increase in survival time when cultured under low-attachment conditions relative to an equivalent amount of unmodified placental stem cells cultured under the same conditions. Survival of arPSCs and unmodified placental stem cells can be assessed using methods known in the art, e.g., trypan blue exclusion assay, fluorescein diacetate uptake assay, propidium iodide uptake assay; thymidine uptake assay, and MTT (3-(4,5-Dimethylthiazol-2-yl)-2,5-diphenyltetrazolium bromide) assay.

As used herein, the phrase "decreased level," when referring to the level of expression of a given gene in an anoikis resistant placental stem cell as compared to the expression of the same gene in an unmodified placental stem cell means that the expression of the gene in the anoikis resistant placental stem cell is downregulated or inhibited, resulting in, e.g., a reduction in the mRNA transcript produced by the gene and/or the protein resulting from the expression of the gene. Determination of whether or not a given gene is expressed at a decreased level can be accomplished by any art-recognized method for detection of protein production or nucleic acid production by cells, *e.g.* nucleic acid-based methods, *e*.*g*., northern blot analysis, reverse transcriptase polymerase chain reaction (RT-PCR), real-time PCR, quantitative PCR, and the like. Expression of proteins can be assessed using antibodies that bind to the protein of interest, *e*.*g*., in an ELISA, Western blot, sandwich assay, or the like. In certain embodiments, a gene in an anoikis resistant placental stem cell (e.g., an anoikis associated gene) is expressed at a decreased level if its expression is decreased by at least a 1.5-fold, 2-fold, 2.5-fold, 3-fold, 3.5-fold, 4-fold, 4.5-fold, 5-fold, 6-fold, 7-fold, 8-fold, 9-fold, or 10-fold as compared to the expression of the gene in an unmodified placental stem cell. In certain embodiments, a gene in an anoikis resistant placental stem cell (e.g., an anoikis associated gene) is expressed at a decreased level if its expression is decreased by at least 1.5-fold to 2.5-fold, 2-fold to 3-fold, 2.5-fold to 3.5-fold, 3-fold to 4-fold, 3.5-fold to 4.5-fold, 4-fold to 5-fold, 5-fold to 6-fold, 6-fold to 7-fold, 7-fold to 8-fold, 8-fold to 9-fold, or 9-fold to 10-fold as compared to the expression of the gene in an unmodified placental stem cell.

### 4. BRIEF DESCRIPTION OF THE FIGURES

FIG. 1 depicts growth of placental stem cells on plates that allow cell adherence (Corning Cellbind) and under low-attachment conditions on plates that do not allow cell adherence (low attachment plates: Corning Ultra-Low Attachment; Nunc Hydrocell; and Nunc Low Cell Binding).
FIG. 2 depicts microscopic images of placental stem cells cultures on plates that allow cell adherence (Corning Cellbind) and under low-attachment conditions on a culture plate that does not allow cell adherence (Corning Ultra-Low Attachment).
FIG. 3 depicts growth of placental stem cells transduced with GFP-expressing lentiviral shRNA in tissue culture plate wells. Bright spots in the tissue plate wells correspond to GFP expression by the transduced placental stem cells.
FIG. 4 depicts the results of an MTS assay performed on placental stem cells in which specified anoikis associated genes (listed on the x-axis) were targeted with siRNA. Non-treated control indicates unmodified placental stem cells; NTP control indicates placental stem cells treated with non-specific siRNA.
FIG. 5 depicts the results of a CyQuant Direct viability assay performed on placental stem cells in which specified anoikis associated genes (listed on the y-axis) were targeted with siRNA. Non-treated control indicates unmodified placental stem cells; NTP control indicates placental stem cells treated with non-specific siRNA.
FIG 6 depicts the results of a CyQuant Direct viability assay performed on placental stem cells in which specified anoikis associated genes (listed on the x-axis) were targeted with siRNA. NTP control indicates placental stem cells treated with non-specific siRNA.
FIG 7: cell growth. A) depicts growth of a population of anoikis resistant stem cells under low attachment conditions (on plates that do not allow cell adherence). B) depicts growth of a population of unmodified placental stem cells under low attachment conditions (on plates that do not allow cell adherence).

### 5. DETAILED DESCRIPTION

### 5.1 PRODUCTION OF ANOIKIS RESISTANT PLACENTAL STEM CELLS

In one aspect, provided herein are methods of modifying placental stem cells to make them resistant to anoikis. Such methods comprise contacting the placental stem cells with an effective amount of one or more oligomeric or polymeric molecules, such that one or more genes that confer anoikis in the placental stem cells is inhibited, i.e., the expression of the gene in the placental stem cells contacted with the oligomeric or polymeric molecules is lessened as compared to the expression of the gene in placental stem cells that have not been contacted with the same oligomeric or polymeric molecules. The anoikis resistant placental stem cells (arPSCs) produced by the methods described herein are placental stem cells that demonstrate an increased survival in low-attachment conditions as compared to unmodified placental stem cells. In certain embodiments, the oligomeric or polymeric molecules used in the methods described herein comprise nucleotides (*e*.*g*., DNA or RNA molecules), nucleosides, nucleotide analogs, nucleotide mimetics, polypeptides, nucleotide analogs, nucleotide mimetics, and any combinations (*e*.*g*., chimeric combinations) thereof.

In one embodiment, the nucleotide analog is an RNA analog, for example, an RNA analog that has been modified in the 2'-OH group, *e.g.* by substitution with a group, for example -O-CH₃, -O-CH₂-CH₂-O-CH₃, -O-CH₂-CH₂-CH₂-NH₂, -O-CH₂-CH₂-CH₂-OH or -F.

In certain embodiments, the oligomeric or polymeric molecules used in the methods described herein comprise one or more modifications (*e*.*g*., chemical modifications) in the sugars, bases, or internucleoside linkages. As used herein, the term "internucleoside linkage group" refers to a group capable of covalently coupling together two nucleotides, such as between RNA units. Examples include phosphate, phosphodiester groups and phosphorothioate groups. In one embodiment, the oligomeric or polymeric molecules used in the methods described herein comprise at least one phosphate internucleoside linkage group. In one embodiment, the oligomeric or polymeric molecules used in the methods described herein comprise at least one phosphodiester internucleoside linkage group.

In certain embodiments, the oligomeric or polymeric molecules used in the methods described herein are single-stranded oligonucleotides or polynucleotides. In certain embodiments, the oligomeric or polymeric molecules used in the methods described herein are double-stranded oligonucleotides or polynucleotides. In certain embodiments, the oligonucleotides or polynucleotides used in the methods described herein comprise one or more modifications (*e*.*g*., chemical modifications) in the sugars, bases, or internucleoside linkages.

In a specific embodiment, the oligomeric molecules used in the methods described herein are modulatory RNA molecules. In certain embodiments, the modulator RNA molecules are small interfering RNAs (siRNAs), microRNA inhibitors (anti-miRs), other modulatory RNA molecules such as antisense RNAs, miR mimics, small hairpin RNAs (shRNAs), microRNA-adapted shRNA (shRNAmirs), or any combination thereof.

### 5.1.1 siRNAs

In certain embodiments, the methods provided herein for the production of anoikis resistant placental stem cells comprise contacting placental stem cells with an effective amount of small interfering RNAs (siRNAs), such that the resistance to anoikis in said placental stem cells is conferred, *e*.*g*., as compared to placental stem cells that have not been modified, *e*.*g*., that have not been contacted with siRNAs. As used herein, the term "small interfering RNA" or "siRNA" refers to an RNA molecule that interferes with the expression of a specific gene.

The siRNAs used in the methods described herein can be single-stranded or double-stranded, and can be modified or unmodified. In one embodiment, the siRNAs used in the methods described herein have one or more 2'-deoxy or 2'-O-modified bases. In some embodiments, the siRNAs used in the methods described herein have one or more base substitutions and inversions (*e*.*g*., 3-4 nucleobases inversions).

In some embodiments, the siRNAs used in the methods described herein are double-stranded. In one embodiment, one strand of the siRNA is antisense to the target nucleic acid, while the other strand is complementary to the first strand. In certain embodiments, said siRNAs comprise a central complementary region between the first and second strands and terminal regions that are optionally complementary between said first and second strands or with the target RNA.

In certain embodiments, the siRNAs used in the methods described herein have a length of about 2 to about 50 nucleobases. In some embodiments, the siRNAs used in the methods described herein are double-stranded, and have a length of about 5 to 45, about 7 to 40, or about 10 to about 35 nucleobases. In some embodiments, the siRNAs used in the methods described herein are double-stranded, and are about 10, 11, 12, 13, 14, 15, 16, 17, 18, 19, 20, 21, 22, 23, 24, 25, 26, 27, 28, 29, 30, 31, 32, 33, 34, or 35 nucleobases in length.

In certain embodiments, one or both ends of the first and/or second strands of the siRNAs used in the methods described herein are modified by adding one or more natural or modified nucleobases to form an overhang. In certain embodiments, one or both ends of the first and/or second strands of the siRNAs used in the methods described herein are blunt. It is possible for one end of the first and/or second strands of the siRNAs used in the methods described herein to be blunt and the other to have overhanging nucleobases. In one embodiment, said overhangs are about 1 to about 10, about 2 to about 8, about 3 to about 7, about 4 to about 6 nucleobase(s) in length. In another embodiment, said overhangs are about 1, 2, 3, 4, 5, 6, 7, 8, 9, or 10 nucleobase(s) in length. In a specific embodiment, the siRNAs used in the methods described herein are double-stranded, and have a length of about 21 nucleobases. In another specific embodiment, the siRNAs are double-stranded, and have a length of about 21 nucleobases comprising dinucleotide 3' overhangs (*e*.*g*., dinucleotide 3' DNA overhangs such as UU or TT 3'-overhangs) such that there is a 19 nt complementary region between the sense and anti-sense strands.

In a specific embodiment, provided herein is a method of producing arPSCs, comprising contacting a placental stem cell, or population thereof, with one or more siRNAs that target one or more genes identified herein as being associated with anoikis in placental stem cells, i.e., the method comprises the targeting of one or more anoikis-associated genes with one or more siRNAs. The anoikis-associated genes that can be targeted by siRNA in accordance with the methods described herein include the genes listed in Table 1, above.

In a specific embodiment, provided herein is a method of producing arPSCs, comprising contacting a placental stem cell, or population thereof, with siRNAs that target one or more of the anoikis associated genes listed in Table 1, above. In one embodiment, said siRNAs are double-stranded. In a specific embodiment, one strand (*e*.*g*., sense strand) of said double-stranded siRNAs has a sequence at least about 70%, 80%, 90%, 95%, 98% or 100% complementary to the sequence of one of the genes identified in Table 1, above (as identified based on the Gene ID of the gene provided in the table).

In another specific embodiment, the siRNAs used in the methods described herein for generating arPSCs target the placental stem cell anoikis associated gene FHDC1 (NCBI GENE ID NO:85462). In another specific embodiment, the siRNAs used in the methods described herein for generating arPSCs target the placental stem cell anoikis associated gene GNAI2 (NCBI GENE ID NO:2771). In another specific embodiment, the siRNAs used in the methods described herein for generating arPSCs target the placental stem cell anoikis associated gene KNDC1 (NCBI GENE ID NO:85442). In another specific embodiment, the siRNAs used in the methods described herein for generating arPSCs target the placental stem cell anoikis associated gene LPAR4 (NCBI GENE ID NO:2846). In another specific embodiment, the siRNAs used in the methods described herein for generating arPSCs target the placental stem cell anoikis associated gene MAP3K5 (NCBI GENE ID NO:4217). In another specific embodiment, the siRNAs used in the methods described herein for generating arPSCs target the placental stem cell anoikis associated gene SLC2A3 (NCBI GENE ID NO:6515). In another specific embodiment, the siRNAs used in the methods described herein for generating arPSCs target the placental stem cell anoikis associated gene STAU2 (NCBI GENE ID NO:27067).

In another specific embodiment, the siRNAs used in the methods described herein for generating arPSCs target one, two, three, or more of the following placental stem cell anoikis-associated genes: FHDC1 (NCBI GENE ID NO:85462), GNAI2 (NCBI GENE ID NO:2771), KNDC1 (NCBI GENE ID NO:85442), LPAR4 (NCBI GENE ID NO:2846), MAP3K5 (NCBI GENE ID NO:4217), SLC2A3 (NCBI GENE ID NO:6515), and STAU2 (NCBI GENE ID NO:27067). In another specific embodiment, the siRNAs used in the methods described herein for generating arPSCs target one, two, three, or more of the following placental stem cell anoikis-associated genes: FHDC1 (NCBI GENE ID NO:85462), GNAI2 (NCBI GENE ID NO:2771), KNDC1 (NCBI GENE ID NO:85442), LPAR4 (NCBI GENE ID NO:2846), MAP3K5 (NCBI GENE ID NO:4217), SLC2A3 (NCBI GENE ID NO:6515), and STAU2 (NCBI GENE ID NO:27067), and target at least one additional anoikis associated gene recited in Table 1.

In another specific embodiment, contacting of an anoikis-associated gene of a placental stem cell with siRNAs results in a decrease in the mRNA level of said gene in said placental stem cell, e.g., the mRNA level of the anoikis-associated gene in the resulting arPSCs is decreased relative to the mRNA level of the same gene in unmodified placental stem cells (i.e., placental stem cells not contacted with an siRNA). In certain embodiments, the mRNA level of an anoikis-associated gene in an arPSC produced according to the methods described herein is decreased about, up to, or no more than, 5%, 10%, 15%, 20%, 25%, 30%, 35%, 40%, 45%, 50%, 55%, 60%, 65%, 70%, 75%, 80%, 85%, 90%, 95%, 98% or 99%, e.g., as compared to the expression of said gene (mRNA level) in unmodified placental stem cells.

The siRNAs used in the methods described herein can be supplied by a commercial vendor (*e*.*g*., Ambion; Dharmacon), or be synthesized by, *e*.*g*., solid phase synthesis, or according to the procedures as described in, *e*.*g*., Protocols for Oligonucleotides and Analogs, Ed. Agrawal (1993), Humana Press; Scaringe, Methods (2001), 23, 206-217. Gait et al., Applications of Chemically synthesized RNA in RNA: Protein Interactions, Ed. Smith (1998), 1-36. Gallo et al., Tetrahedron (2001), 57, 5707-5713).

siRNAs useful for the production of anoikis resistant placental stem cells can be identified by a variety of methods known in the art. In certain embodiments, such siRNAs are identified and obtained from one or more siRNA libraries, *e*.*g*., a commercially available library (*e*.*g*., Ambion, Silencer® Select Human Nuclear Hormone Receptor (HNR) siRNA Library V4; Dharmacon, siRNA library Human ON-TARGETplus siRNA Nuclear Receptors Sub-Library), optionally by a screening method, *e*.*g*., medium or high-throughput screening. In one embodiment, such a library can encompass a wide range of genes (*e*.*g*., human genome-wide siRNA library), or pre-defined to encompass specific target genes or gene families (*e*.*g*., human nuclear receptor siRNA library, phosphatase siRNA library, *etc*.). The screening method can be carried out, for example, using automated robotics, liquid handling equipments, data processing software, and/or sensitive detectors, *e*.*g*., Precision XS Automated Pipettor System, EL406 liquid handling system, or synergy plate reader.

### 5.1.2 miR inhibitors and miR mimics

In certain embodiments, the methods provided herein for the production of anoikis resistant placental stem cells comprise contacting placental stem cells with an effective amount of microRNA inhibitors (miR inhibitors), such that the resistance to anoikis in said placental stem cells is conferred, *e*.*g*., as compared to placental stem cells that have not been modified, *e*.*g*., that have not been contacted with miR inhibitors. As used herein, the term "microRNA," "miRNA," or "miR" refers to short ribonucleic acid (RNA) molecules, including, but not limited to, mature single stranded miRNAs, precursor miRNAs (pre-miR), and variants thereof. As used herein, the term "microRNA inhibitor," "miRNA inhibitor," "miR inhibitor" or "anti-miR" refer to a ribonucleic acid molecule designed to inhibit miRNAs (*e*.*g*., endogenous miRNAs). In some embodiments, the miR inhibitors downregulate (*e*.*g*., inhibit) a target gene by inhibition of one or more endogenous miRs. In one embodiment, the microRNAs are naturally occurring. In certain embodiments, the microRNAs are post-transcriptional regulators that bind to complementary sequences on target messenger RNA transcripts (mRNAs) and result in translational repression and gene silencing. In certain embodiments, a single precursor contains more than one mature miRNA sequence. In other embodiments, multiple precursor miRNAs contain the same mature sequence. In some embodiments, when the relative abundances clearly indicate which is the predominantly expressed miRNA, the term "microRNA," "miRNA," or "miR" refers to the predominant product, and the term "microRNA*," "miRNA*," or "miR*" refers to the opposite arm of the precursor. In one embodiment, miRNA is the "guide" strand that eventually enters RNA-Induced Silencing Complex (RISC), and miRNA* is the other "passenger" strand. In another embodiment, the level of miRNA* present in the cell at a lower level (*e*.*g*., ≤15%) relative to the corresponding miRNA. In some embodiments where there is a higher proportion of passenger strand present in the cell, the nomenclature miRNA-3p (*i*.*e*., miRNA derived from the 3' arm of the precursor miRNA) and miRNA-5p (*i*.*e*., miRNA-5p is the miRNA derived from the 5' arm of the precursor miRNA) is used instead of miRNA/miRNA*.

As used herein, the term "microRNA mimic(s)" or "miR mimic(s)" refers to molecules that can be used to imitate or mimic the gene silencing ability of one or more miRNAs. In one embodiment, the miR mimics down-regulate (e.g., inhibit) a target gene by imitating one or more endogenous miRs. In certain embodiments, miRNA mimics are synthetic non-coding RNAs (*i*.*e*., the miRNA is not obtained by purification from a source of the endogenous miRNA). In certain embodiments, the miRNA mimics are capable of entering the RNAi pathway and regulating gene expression. In certain embodiments, miRNA mimics can be designed as mature molecules (*e*.*g*. single stranded) or mimic precursors (*e*.*g*., pri- or pre-miRNAs).

In some embodiments, the miR inhibitors or miRNA mimics provided herein comprise nucleic acid (modified or modified nucleic acids) including oligonucleotides comprising, *e*.*g*., RNA, DNA, modified RNA, modified DNA, locked nucleic acids, or 2'-O,4'-C-ethylene-bridged nucleic acids (ENA), or any combination of thereof.

The miR inhibitors or miR mimics can be single-stranded or double-stranded, and can be modified or unmodified. In certain embodiments, the miR inhibitors or miR mimics have a length of about 2 to about 30 nucleobases. In certain embodiments, the miR inhibitors or miR mimics are single-stranded, and have a length of about 15 to about 30 nucleobases. In some embodiments, the miR inhibitors are single-stranded, and are about 15, 16, 17, 18, 19, 20, 21, 22, 23, 24, 25, 26, 27, 28, 29 or 30 nucleobases in length.

In a specific embodiment, provided herein is a method of producing arPSCs, comprising contacting a placental stem cell, or population thereof, with one or more miR inhibitors or miR mimics that target one or more miRs in said placental stem cells that modulate the activity of one or more genes identified herein as being associated with anoikis in placental stem cells. The miRs that can be targeted by miR inhibitors and/or miR mimics in accordance with the methods described herein include miRs associated with the modulation of the anoikis associated genes listed in Table 1, above.

In another specific embodiment, provided herein is a method of producing arPSCs, comprising contacting a placental stem cell, or population thereof, with a miR inhibitor or miR mimic that targets a miR in said placental stem cells that modulates the production of an anoikis associated gene in said placental stem cell (e.g., an anoikis associated gene listed in Table 1, above), such that the production of the anoikis associated gene by said placental stem cells is decreased, e.g., as compared to an equivalent number of unmodified placental stem cells. In certain embodiments, said miR inhibitors or said miR mimics have a sequence at least about 70%, 80%, 90%, 95%, 98% or 100% complementary to the sequence an miRNA that modulates the production of one of the genes identified in Table 1.

In another specific embodiment, the miR inhibitors or miR mimics used in the methods described herein for generating arPSCs target a miRNA that modulates the production of the placental stem cell anoikis associated gene FHDC1 (NCBI GENE ID NO:85462). In another specific embodiment, the miR inhibitors or miR mimics used in the methods described herein for generating arPSCs target a miRNA that modulates the production of the placental stem cell anoikis associated gene GNAI2 (NCBI GENE ID NO:2771). In another specific embodiment, the miR inhibitors or miR mimics used in the methods described herein for generating arPSCs target a miRNA that modulates the production of the placental stem cell anoikis associated gene KNDC1 (NCBI GENE ID NO:85442). In another specific embodiment, the miR inhibitors or miR mimics used in the methods described herein for generating arPSCs target a miRNA that modulates the production of the placental stem cell anoikis associated gene LPAR4 (NCBI GENE ID NO:2846). In another specific embodiment, the miR inhibitors or miR mimics used in the methods described herein for generating arPSCs target a miRNA that modulates the production of the placental stem cell anoikis associated gene MAP3K5 (NCBI GENE ID NO:4217). In another specific embodiment, the miR inhibitors or miR mimics used in the methods described herein for generating arPSCs target a miRNA that modulates the production of the placental stem cell anoikis associated gene SLC2A3 (NCBI GENE ID NO:6515). In another specific embodiment, the miR inhibitors or miR mimics used in the methods described herein for generating arPSCs target a miRNA that modulates the production of the placental stem cell anoikis associated gene STAU2 (NCBI GENE ID NO:27067).

In another specific embodiment, the miR inhibitors or miR mimics used in the methods described herein for generating arPSCs target one, two, three, or more miRNAs, wherein said miRNAs modulate the production of one, two, three, or more of the following placental stem cell anoikis-associated genes: FHDC1 (NCBI GENE ID NO:85462), GNAI2 (NCBI GENE ID NO:2771), KNDC1 (NCBI GENE ID NO:85442), LPAR4 (NCBI GENE ID NO:2846), MAP3K5 (NCBI GENE ID NO:4217), SLC2A3 (NCBI GENE ID NO:6515), and STAU2 (NCBI GENE ID NO:27067). In another specific embodiment, the miR inhibitors or miR mimics used in the methods described herein for generating arPSCs target one, two, three, or more miRNAs, wherein said miRNAs modulate the production of one, two, three, or more of the following placental stem cell anoikis-associated genes: FHDC1 (NCBI GENE ID NO:85462), GNAI2 (NCBI GENE ID NO:2771), KNDC1 (NCBI GENE ID NO:85442), LPAR4 (NCBI GENE ID NO:2846), MAP3K5 (NCBI GENE ID NO:4217), SLC2A3 (NCBI GENE ID NO:6515), and STAU2 (NCBI GENE ID NO:27067), and target at least one miRNA that modulates the production of at least one additional anoikis associated gene recited in Table 1.

In another specific embodiment, contacting of miRNA that modulates the production of an anoikis-associated gene of a placental stem cell with a miR inhibitor or miR mimic results in a decrease in the mRNA level of said gene in said placental stem cell, e.g., the mRNA level of the anoikis-associated gene in the resulting arPSCs is decreased relative to the mRNA level of the same gene in unmodified placental stem cells (i.e., placental stem cells not contacted with a miR inhibitor or miR mimic). In certain embodiments, the mRNA level of an anoikis-associated gene in an arPSC produced according to the methods described herein is decreased about, up to, or no more than, 5%, 10%, 15%, 20%, 25%, 30%, 35%, 40%, 45%, 50%, 55%, 60%, 65%, 70%, 75%, 80%, 85%, 90%, 95%, 98% or 99%, e.g., as compared to the expression of said gene (mRNA level) in unmodified placental stem cells.

The miR inhibitors and miR mimics used in the methods described herein can be supplied by a commercial vendor (*e*.*g*., Ambion; Dharmafect), or can be synthesized by, *e*.*g*., solid phase synthesis, or according to the procedures as described in, *e*.*g*., Protocols for Oligonucleotides and Analogs, Ed. Agrawal (1993), Humana Press; Scaringe, Methods (2001), 23, 206-217. Gait et al., Applications of Chemically synthesized RNA in RNA: Protein Interactions, Ed. Smith (1998), 1-36. Gallo et al., Tetrahedron (2001), 57, 5707-5713).

The miR inhibitors and miR mimics used in the methods described herein can be identified by a variety of methods known in the art. In certain embodiments, such miR inhibitors and/or miR mimics are identified and obtained from one or more miR inhibitors or miR mimics libraries, *e*.*g*., a commercially available library (*e*.*g*., Ambion, Anti-miR miRNA Precursor Library Human V13), optionally by a screening method, *e*.*g*., medium or high-throughput screening. In one embodiment, such a library can encompass a wide range of target miRs (*e*.*g*., human genome-wide siRNA library), or pre-defined to encompass specific target genes or gene families (*e*.*g*., nuclear receptor siRNA library, phosphatase siRNA library *etc*.). The screening method can be carried out, for example, using automated robotics, liquid handling equipments, data processing software, and/or sensitive detectors, *e*.*g*., Precision XS Automated Pipettor System, EL406 liquid handling system, or synergy plate reader.

### 5.1.3 Other modulatory RNA molecules

Other modulatory RNA molecules useful for the production of arPSCs comprise antisense RNAs, shRNAs, and shRNAmirs. These RNA molecules can be used in any combination and can be used in combination with siRNAs, miR mimics and/or miR inhibitors to produce the arPSCs as described herein.

As used herein, the term "antisense RNA" is an antisense ribonucleic acid molecule. By illustration only and without limitation, the antisense RNAs hybridize to a target nucleic acid (e.g., a gene) and modulate expression activities of the target nucleic acid, such as transcription or translation.

As used herein, the term "small hairpin RNA" or "shRNA" refers to an RNA molecule comprising a stem-loop structure; the term "shRNAmir" refers to "microRNA-adapted shRNA." In certain embodiments, said shRNA comprises a first and second region of complementary sequence, the degree of complementarity and orientation of the regions being sufficient such that base pairing occurs between the regions, the first and second regions being joined by a loop region, the loop resulting from a lack of base pairing between nucleotides (or nucleotide analogs) within the loop region. The shRNA hairpin structure can be, for example, cleaved by the cellular machinery into siRNA, which is then bound to the RNA-induced silencing complex (RISC). This complex binds to and cleaves mRNAs which match the siRNA that is bound to it.

In some embodiments, shRNAmirs or microRNA-adapted shRNA provided herein are shRNA constructs that mimic naturally occurring primary transcript miRNA with the addition of an miRNA loop and a miRNA flanking sequence to a shRNA. Without wishing to be bound by any theory, the shRNAmir is first cleaved to produce shRNA by Drosha, and then cleaved again by Dicer to produce siRNA. The siRNA is then incorporated into the RISC for target mRNA degradation. This allows the shRNAmir to be cleaved by Drosha thereby allowing for a greater increase in knockdown efficiency. The addition of a miR30 loop and 125 nt of miR30 flanking sequence on either side of the shRNA hairpin has been reported to result in greater than 10-fold increase in Drosha and Dicer processing of the expressed hairpins when compared with conventional shRNA constructs without microRNA.

In a specific embodiment, provided herein is a method of producing arPSCs, comprising contacting a placental stem cell, or population thereof, with one or more antisense RNAs, shRNAs, and shRNAmirs that target one or more genes identified herein as being associated with anoikis in placental stem cells, i.e., the method comprises the targeting of one or more anoikis-associated genes with one or more antisense RNAs, shRNAs, and shRNAmirs. The anoikis-associated genes that can be targeted by antisense RNAs, shRNAs, and shRNAmirs in accordance with the methods described herein include the genes listed in Table 1, above.

In another specific embodiment, the modulatory RNA molecules used in the methods described herein for generating arPSCs are small hairpin RNAs or shRNAs. In a specific embodiment, said shRNAs target one or more of the anoikis-associated genes listed in Table 1, above. In another specific embodiment, said shRNAs have a sequence at least about 70%, 80%, 90%, 95%, 98% or 100% complementary to the sequence of one of the genes identified in Table 1, above (as identified based on the Gene ID of the gene provided in the table).

In another embodiment, the modulatory RNA molecules used in the methods described herein for generating arPSCs are antisense RNAs. In a specific embodiment, said antisense RNAs target one or more of the anoikis-associated genes listed in Table 1, above. In another specific embodiment, said antisense RNAs have a sequence at least about 70%, 80%, 90%, 95%, 98% or 100% complementary to the sequence of one of the genes identified in Table 1, above (as identified based on the Gene ID of the gene provided in the table).

In another specific embodiment, the shRNAs used in the methods described herein for generating arPSCs target the placental stem cell anoikis associated gene FHDC1 (NCBI GENE ID NO:85462). In another specific embodiment, the shRNAs used in the methods described herein for generating arPSCs target the placental stem cell anoikis associated gene GNAI2 (NCBI GENE ID NO:2771). In another specific embodiment, the shRNAs used in the methods described herein for generating arPSCs target the placental stem cell anoikis associated gene KNDC1 (NCBI GENE ID NO:85442). In another specific embodiment, the shRNAs used in the methods described herein for generating arPSCs target the placental stem cell anoikis associated gene LPAR4 (NCBI GENE ID NO:2846). In another specific embodiment, the shRNAs used in the methods described herein for generating arPSCs target the placental stem cell anoikis associated gene MAP3K5 (NCBI GENE ID NO:4217). In another specific embodiment, the shRNAs used in the methods described herein for generating arPSCs target the placental stem cell anoikis associated gene SLC2A3 (NCBI GENE ID NO:6515). In another specific embodiment, the shRNAs used in the methods described herein for generating arPSCs target the placental stem cell anoikis associated gene STAU2 (NCBI GENE ID NO:27067).

In another specific embodiment, the shRNAs used in the methods described herein for generating arPSCs target one, two, three, or more of the following placental stem cell anoikis-associated genes: FHDC1 (NCBI GENE ID NO:85462), GNAI2 (NCBI GENE ID NO:2771), KNDC1 (NCBI GENE ID NO:85442), LPAR4 (NCBI GENE ID NO:2846), MAP3K5 (NCBI GENE ID NO:4217), SLC2A3 (NCBI GENE ID NO:6515), and STAU2 (NCBI GENE ID NO:27067). In another specific embodiment, the shRNAs used in the methods described herein for generating arPSCs target one, two, three, or more of the following placental stem cell anoikis-associated genes: FHDC1 (NCBI GENE ID NO:85462), GNAI2 (NCBI GENE ID NO:2771), KNDC1 (NCBI GENE ID NO:85442), LPAR4 (NCBI GENE ID NO:2846), MAP3K5 (NCBI GENE ID NO:4217), SLC2A3 (NCBI GENE ID NO:6515), and STAU2 (NCBI GENE ID NO:27067), and target at least one additional anoikis associated gene recited in Table 1.

In another specific embodiment, the antisense RNAs used in the methods described herein for generating arPSCs target the placental stem cell anoikis associated gene FHDC1 (NCBI GENE ID NO:85462). In another specific embodiment, the antisense RNAs used in the methods described herein for generating arPSCs target the placental stem cell anoikis associated gene GNAI2 (NCBI GENE ID NO:2771). In another specific embodiment, the antisense RNAs used in the methods described herein for generating arPSCs target the placental stem cell anoikis associated gene KNDC1 (NCBI GENE ID NO:85442). In another specific embodiment, the antisense RNAs used in the methods described herein for generating arPSCs target the placental stem cell anoikis associated gene LPAR4 (NCBI GENE ID NO:2846). In another specific embodiment, the antisense RNAs used in the methods described herein for generating arPSCs target the placental stem cell anoikis associated gene MAP3K5 (NCBI GENE ID NO:4217). In another specific embodiment, the antisense RNAs used in the methods described herein for generating arPSCs target the placental stem cell anoikis associated gene SLC2A3 (NCBI GENE ID NO:6515). In another specific embodiment, the antisense RNAs used in the methods described herein for generating arPSCs target the placental stem cell anoikis associated gene STAU2 (NCBI GENE ID NO:27067).

In another specific embodiment, the antisense RNAs used in the methods described herein for generating arPSCs target one, two, three, or more of the following placental stem cell anoikis-associated genes: FHDC1 (NCBI GENE ID NO:85462), GNAI2 (NCBI GENE ID NO:2771), KNDC1 (NCBI GENE ID NO:85442), LPAR4 (NCBI GENE ID NO:2846), MAP3K5 (NCBI GENE ID NO:4217), SLC2A3 (NCBI GENE ID NO:6515), and STAU2 (NCBI GENE ID NO:27067). In another specific embodiment, the antisense RNAs used in the methods described herein for generating arPSCs target one, two, three, or more of the following placental stem cell anoikis-associated genes: FHDC1 (NCBI GENE ID NO:85462), GNAI2 (NCBI GENE ID NO:2771), KNDC1 (NCBI GENE ID NO:85442), LPAR4 (NCBI GENE ID NO:2846), MAP3K5 (NCBI GENE ID NO:4217), SLC2A3 (NCBI GENE ID NO:6515), and STAU2 (NCBI GENE ID NO:27067), and target at least one additional anoikis associated gene recited in Table 1.

In another specific embodiment, contacting of an anoikis-associated gene of a placental stem cell with an shRNA or antisense RNA results in a decrease in the mRNA level of said gene in said placental stem cell, e.g., the mRNA level of the anoikis-associated gene in the resulting arPSCs is decreased relative to the mRNA level of the same gene in unmodified placental stem cells (i.e., placental stem cells not contacted with an shRNA or antisense RNA). In certain embodiments, the mRNA level of an anoikis-associated gene in an arPSC produced according to the methods described herein is decreased about, up to, or no more than, 5%, 10%, 15%, 20%, 25%, 30%, 35%, 40%, 45%, 50%, 55%, 60%, 65%, 70%, 75%, 80%, 85%, 90%, 95%, 98% or 99%, e.g., as compared to the expression of said gene (mRNA level) in unmodified placental stem cells.

The antisense RNAs, shRNAs and shRNAmirs used in the methods described herein can be supplied by a commercial vendor (*e*.*g*., Ambion; Dharmafect), or can be synthesized by, *e*.*g*., solid phase synthesis, or according to the procedures as described in, *e*.*g*., Protocols for Oligonucleotides and Analogs, Ed. Agrawal (1993), Humana Press; Scaringe, Methods (2001), 23, 206-217. Gait et al., Applications of Chemically synthesized RNA in RNA: Protein Interactions, Ed. Smith (1998), 1-36. Gallo et al., Tetrahedron (2001), 57, 5707-5713).

Antisense RNAs, shRNAs, shRNAmirs and other modulatory RNA molecules useful for the production of anoikis resistant placental stem cells can be identified by a variety of methods known in the art. In certain embodiments, such antisense RNAs, shRNAs, shRNAmirs and other modulatory RNA molecules are identified and obtained from one or more libraries, *e*.*g*., a commercially available library (Thermo Scientific, shRNAmir libraries), optionally by a screening method, *e*.*g*., medium or high-throughput screening. In one embodiment, such a library can encompass a wide range of genes (*e*.*g*., human genome targeted library), or pre-defined to encompass specific target genes or gene families (*e*.*g*., human nuclear receptor targeted library, phosphatase targeted library, *etc*.). The screening method can be carried out, for example, using automated robotics, liquid handling equipments, data processing software, and/or sensitive detectors, *e*.*g*., Precision XS Automated Pipettor System, EL406 liquid handling system, or synergy plate reader.

In certain embodiments, the antisense RNAs, shRNAs and shRNAmirs used in the methods described herein comprise about 1 to about 100, from about 8 to about 80, 10 to 50, 13 to 80, 13 to 50, 13 to 30, 13 to 24, 18 to 22, 19 to 23, 20 to 80, 20 to 50, 20 to 30, or 20 to 24 nucleobases (nucleobases (*i.e.* from about 1 to about 100 linked nucleosides).

The antisense RNAs, shRNAs and shRNAmirs used in the methods described herein can be single-stranded or double-stranded, modified or unmodified. In certain embodiments, said antisense RNAs, miR mimics, shRNAs, shRNAmirs and other modulatory RNA molecules comprise about 1 to about 100, from about 8 to about 80, 10 to 50, 13 to 80, 13 to 50, 13 to 30, 13 to 24, 18 to 22, 19 to 23, 20 to 80, 20 to 50, 20 to 30, or 20 to 24 nucleobases (*i.e.* from about 1 to about 100 linked nucleosides). In certain embodiment, the antisense RNAs, shRNAs and shRNAmirs used in the methods described herein are single-stranded, comprising from about 12 to about 35 nucleobases (*i.e.* from about 12 to about 35 linked nucleosides). In one embodiment, the antisense RNAs, miR mimics, shRNAs and shRNAmirs used in the methods described herein are about 10, 11, 12, 13, 14, 15, 16, 17, 18, 19, 20, 21, 22, 23, 24, 25, 26, 27, 28, 29, 30, 31, 32, 33, 34, or 35 nucleobases in length.

The shRNAmirs used in the methods described herein can be delivered to the cells by any known method. In a specific embodiment, an shRNAmir used in the methods described herein is incorporated into a eukaryotic expression vector. In another specific embodiment, an shRNAmir used in the methods described herein is incorporated into a viral vector for gene expression. Such viral vectors include, but are not limited to, retroviral vectors, e.g., lentivirus, and adenoviruses. In a specific embodiment, an shRNAmir used in the methods described herein is incorporated into a lentiviral vector.

### 5.1.4 Delivery Of Modulatory RNA Molecules To Placental Stem Cells

The modulatory RNA molecules used in the methods described herein can be delivered to placental stem cells by transfection (*e*.*g*., transient or stable transfection) or other means known in the art. In certain embodiments, said transfection can be carried out, *e*.*g*., using lipids (*e*.*g*., liposomes), calcium phosphate, cyclodextrin, dendrimers, or polymers (*e*.*g*., cationic polymers); by electroporation, optical transfection, gene electrotransfer, impalefection, gene gun, or magnetofection; via viruses (*e*.*g*., viral carriers); or a combination thereof. In one embodiment, said transfection is performed using commercially available transfection reagents or kits (*e*.*g*., Ambion, siPORT™ Amine, siPORT NeoFX's; Dharmafect, Dharmafect 3 Transfection Reagent or Dharmafect 1 Transfection Reagent; Invitrogen, Lipofectamine RNAiMAX; Integrated DNA Technologies, Transductin; Mirus Bio LLC, TransIT-siQUEST, TransIT-TKO). In a specific embodiment, said transfection can be carried out using Dharmacon ON-TARGET plus SMARTpool® siRNA reagents with the Dharmafect 1 Transfection Reagent. In some embodiments, said transfection can be set up in a medium or high-throughput manner, including, but not limited to, use of microtiter plate (*e*.*g*., 96-well plate) and microplate reader (*e*.*g*., synergy plate reader), or automation system, for example, Precision XS Automated Pipettor System, EL406 liquid handling system. In other embodiments, said transfection is set up in a large scale, including, but not limited to, the use of tissue culture dishes or culture flasks (*e*.*g*., T25, T75, or T225 flasks). Placental stem cells can be plated and cultured in tissue culture containers, *e*.*g*., dishes, flasks, multiwell plates, or the like, for a sufficient time for the placental stem cells to proliferate to about 20-80% confluence, or about 30-70% confluence at the time of transfection. For example, there can be about 2000, 2500, 3000, 3500, or 4000 placental stem cells per well in a 96-well plate at the time of transfection. In one embodiment, placental stem cells are about 50% confluence at the time of transfection. In another embodiment, there are about 3000 or 3500 placental stem cells per well in a 96-well plate at the time of direct transfection. In another embodiment, there are about 3500 placental stem cells per well in a 96-well plate at the time of reverse transfection.

The modulatory RNA molecules used in the methods described herein can be administered to the cells by transient transfection, or can be stably transfected to the cell for long-term modulation (*e*.*g*., suppression) of genes to which the modulatory RNA molecules (e.g., siRNAs) are targeted. In one embodiment, stable transfection of modulatory RNA molecules can be carried out, for example, by the use of plasmids or expression vectors that express functional modulatory RNA molecules. In one embodiment, such plasmids or expression vectors comprise a selectable marker (*e*.*g*., an antibiotic selection marker). In another embodiment, such plasmids or expression vectors comprise a cytomegalovirus (CMV) promoter, an RNA polymerase III (RNA pol III) promoter (*e*.*g*., U6 or H1), or an RNA polymerase II (RNA pol II) promoter. In another embodiment, such plasmids or expression vectors are commercially available (*e*.*g*., Ambion, pSilencer™ 4.1-CMV vector).

Other examples of mammalian expression vectors include pLOC (Open Biosystems), which contains a cytomegalovirus promoter; pCDM8 (Seed, Nature 329:840 (1987)) and pMT2PC (Kaufman et al., EMBO J. 6:187-195 (1987)). Other example expression vectors that may be used include pFN10A (ACT) FLEXI® Vector (Promega), pFN11A (BIND) FLEXI® Vector (Promega), pGL4.31[*luc2P*/*GAL4*UAS/Hygro] (Promega), pFC14K (HALOTAG® 7) MCV FLEXI® Vector (Promega), pFC15A (HALOTAG® 7) MCV FLEXI® Vector (Promega), and the like.

When used in mammalian cells, an expression vector's control functions can be provided by viral regulatory elements. For example, commonly used promoters are derived from polyoma virus, adenovirus 2, cytomegalovirus, and simian virus 40. Other suitable expression systems for both prokaryotic and eukaryotic cells are described, *e*.*g*., in chapters 16 and 17 of Sambrook et al., eds., Molecular Cloning: A Laboratory Manual, 2nd, ed., Cold Spring Harbor Laboratory, Cold Spring Harbor Laboratory Press, Cold Spring Harbor, N.Y. (1989).

Recombinant expression vectors can include one or more control sequences that can be, for example, operably linked to the nucleic acid sequence encoding the gene to be expressed. Such control sequences are described, for example, in Goeddel, Gene Expression Technology: Methods in Enzymology 185, Academic Press, San Diego, Calif. (1990). In certain embodiments, the vector includes a control sequence that directs constitutive expression of the nucleotide sequence in the placental stem cells. In certain other embodiments, the control sequence directs expression of the nucleotide sequence only in cells of certain tissues in a recipient of the arPSCs, *e*.*g*., in lung, neural, muscle, skin, vascular system, or other tissues, within said recipient. In certain other embodiments, said vector comprises a control sequence that is inducible, *e*.*g*., by contact with a chemical agent, *e*.*g*., tetracycline.

The modulatory RNA molecules can be administered to the cells by any technique known to those of skill in the art, *e*.*g*., by direct transfection. For example, said direct transfection can involve the step of pre-plating the cells prior to transfection, allowing them to reattach and resume growth for a period of time (*e*.*g*., 24 hours) before exposure to transfection complexes. The modulatory RNA molecules can also be administered to the cells by reverse transfection. For example, said reverse transfection can involve the step of adding transfection complexes to the cells while they are in suspension, prior to plating.

In various embodiments, the effects of the modulatory RNA molecules on placental stem cells, *e*.*g*., downregulation of one or more anoikis-associated genes in said placental stem cells so as to generate arPSCs from said placental stem cells, can last for up to, about, or no more than, 1, 2, 3, 4, 5, 6, 7, 8, 9, 10, 11, 12, 13, 14, 15, 16, 17, 18, 19, 20, 21, 22 or 23 hours, or 1, 2, 3, 4, 5, 6, 7, 8, 9, 10, 11, 12, 13, 14, 15, 16, 17, 18, 19, 20, 21, 22, 23, 24, 25, 26, 27, or 28 days, or more. In certain embodiments, the arPSCs generated using the methods described herein are used (e.g., administered to a subject) within no more than 1, 2, 3, 4, 5, 6, 7, 8, 9, 10, 11, 12, 13, 14, 15, 16, 17, 18, 19, 20, 21, 22 or 23 hours, or 1, 2, 3, 4, 5, 6, 7, 8, 9, 10, 11, 12, 13, 14, 15, 16, 17, 18, 19, 20, 21, 22, 23, 24, 25, 26, 27, or 28 days of the time the arPSCs are produced. In certain embodiments, the arPSCs generated using the methods described herein are preserved, e.g., cryopreserved, before use (e.g., before administration to a subject). In certain embodiments, the effects of the modulatory RNA molecules on the arPSCs are inducible. In certain other embodiments, no, or substantially no, cellular expansion (culturing of the arPSCs, proliferation, etc.) is performed between the time the placental stem cells are modified to produce the arPSCs and the time the arPSCs are administered or cryopreserved.

Assessment of the function (*e*.*g*., silencing of anoikis-associated genes) of the modulatory RNA molecules used in the methods described herein, *e*.*g*., the level or degree of gene silencing, can be accomplished by any art-recognized method for detection of protein production or nucleic acid production by cells. For example, assessment can be performed by determining the mRNA or protein level of a gene of interest in a sample of arPSCs (*e*.*g*., a sample of 10 x 10⁵ to 10 x 10⁷ arPSCs, or 1%, 2%, 3%, 4%, 5%, 6%, 7%, 8%, 9%, or 10% of said arPSCs) as compared to equivalent placental stem cells that have not been transfected or transformed with such a nucleic acid sequence. Such assessment can be performed using, *e*.*g*. nucleic acid-based methods, e.g., northern blot analysis, reverse transcriptase polymerase chain reaction (RT-PCR), real-time PCR, quantitative PCR, and the like. In other embodiments, expression of protein can be assessed using antibodies that bind to the protein of interest, *e*.*g*., in an ELISA, sandwich assay, or the like. In a specific embodiment, the anoikis resistant placental stem cells generated using the methods described herein produce 5%, 10%, 15%, 20%, 25%, 30%, 35%, 40%, 45%, 50%, 55%, 60%, 65%, 70%, 75%, 80%, 85%, 90%, 95%, or 100% less of the mRNA of a target gene (e.g., an anoikis-associated gene) as compared to unmodified placental stem cells (e.g., an equivalent amount of unmodified placental stem cells (i.e., placental stem cells that have not been contacted with a modulatory RNA molecule). In a specific embodiment, the anoikis resistant placental stem cells generated using the methods described herein produce 5%, 10%, 15%, 20%, 25%, 30%, 35%, 40%, 45%, 50%, 55%, 60%, 65%, 70%, 75%, 80%, 85%, 90%, 95%, or 100% less of the protein of a target gene (e.g., an anoikis-associated gene) as compared to unmodified placental stem cells (e.g., an equivalent amount of unmodified placental stem cells (i.e., placental stem cells that have not been contacted with a modulatory RNA molecule).

### 5.2 USES OF ANOIKIS RESISTANT PLACENTAL STEM CELLS

One advantage of the arPSCs described herein is that they maintain the functional characteristics of unmodified placental stem cells (e.g., the placental stem cells described in U.S. Patent Nos. 7,311,904; 7,311,905; 7,468,276 and 8,057,788, the disclosures of which are hereby incorporated by reference in their entireties), yet are resistant to anoikis and thus demonstrate increased survival in low-attachment conditions as compared to, e.g., unmodified placental stem cells, which are not anoikis-resistant. Accordingly, the arPSCs described herein can be advantageously used in methods that comprise the administration of placental stem cells to a subject, wherein the placental stem cells are administered in a low-attachment environment, e.g., the placental stem cells are administered systemically or the placental stem cells are administered locally and do not adhere to a substrate (e.g., extracellular matrix) in the local environment.

In one embodiment, the arPSCs described herein can be used in methods of treating an individual having or at risk of developing a disease, disorder or condition caused by, or relating to, an unwanted or harmful immune response, for instance, a disease, disorder or condition having an inflammatory component. In another embodiment, provided herein are methods for the modulation, *e*.*g*., suppression, of the activity, *e*.*g*., proliferation, of an immune cell, or plurality of immune cells, by contacting the immune cell(s) with a plurality of arPSCs (e.g., a composition comprising arPSCs). In accordance with such methods, a therapeutically effective amount of arPSCs can be administered to the individual, wherein the administered arPSCs can survive in low-attachment conditions in said individual for greater periods of time than, e.g., unmodified placental stem cells administered in the same fashion.

In a specific embodiment, provided herein is a method of suppressing an immune response comprising contacting a plurality of immune cells with a plurality of anoikis resistant placental stem cells for a time sufficient for said anoikis resistant placental stem cells to detectably suppress an immune response, wherein said anoikis resistant placental stem cells detectably suppress T cell proliferation in a mixed lymphocyte reaction (MLR) assay or a regression assay. An "immune cell" in the context of this method means any cell of the immune system, particularly T cells and NK (natural killer) cells. Thus, in various embodiments of the method, anoikis resistant placental stem cells are contacted with a plurality of immune cells, wherein the plurality of immune cells are, or comprises, a plurality of T cells (*e*.*g*., a plurality of CD3⁺ T cells, CD4⁺ T cells and/or CD8⁺ T cells) and/or natural killer cells. An "immune response" in the context of the method can be any response by an immune cell to a stimulus normally perceived by an immune cell, *e*.*g*., a response to the presence of an antigen. In various embodiments, an immune response can be the proliferation of T cells (*e*.*g*., CD3⁺ T cells, CD4⁺ T cells and/or CD8⁺ T cells) in response to a foreign antigen, such as an antigen present in a transfusion or graft, or to a self-antigen, as in an autoimmune disease. The immune response can also be a proliferation of T cells contained within a graft. The immune response can also be any activity of a natural killer (NK) cell, the maturation of a dendritic cell, or the like. The immune response can also be a local, tissue- or organ-specific, or systemic effect of an activity of one or more classes of immune cells, *e*.*g*., the immune response can be graft versus host disease, inflammation, formation of inflammation-related scar tissue, an autoimmune condition (*e*.*g*., rheumatoid arthritis, Type I diabetes, lupus erythematosus, *etc*.). and the like.

"Contacting," as used herein in such a context, encompasses bringing the placental stem cells and immune cells together in a single container (*e*.*g*., culture dish, flask, vial, *etc*.) or *in vivo,* for example, in the same individual (*e*.*g*., mammal, for example, human). In one embodiment, the contacting is for a time sufficient, and with a sufficient number of arPSCs and immune cells, that a change in an immune function of the immune cells is detectable. In certain embodiments, said contacting is sufficient to suppress immune function (*e*.*g*., T cell proliferation in response to an antigen) by at least 50%, 60%, 70%, 80%, 90% or 95%, compared to the immune function in the absence of the arPSCs. Such suppression in an *in vivo* context can be determined in an *in vitro* assay (*see* below); that is, the degree of suppression in the *in vitro* assay can be extrapolated, for a particular number of anoikis resistant placental stem cells and a number of immune cells in a recipient individual, to a degree of suppression in the individual.

The ability of anoikis resistant placental stem cells to suppress an immune response can be, e.g., assessed in vitro. In certain embodiments, an anoikis resistant placental stem cell provided herein suppresses an immune response at least 50%, 60%, 70%, 75%, 80%, 85%, 90%, 95%, or 99% as well as an unmodified placental stem cell (e.g., placental stem cells that are not resistant to anoikis). In certain embodiments, an anoikis resistant placental stem cell provided herein suppresses an immune response to the same extent as an unmodified placental stem cell (e.g., placental stem cells that are not resistant to anoikis). For example, a plurality of anoikis resistant placental stem cells can be tested in an MLR comprising combining CD4⁺ or CD8⁺ T cells, dendritic cells (DC) and anoikis resistant placental stem cells in a ratio of about 10:1:2, wherein the T cells are stained with a dye such as, *e*.*g*., CFSE that partitions into daughter cells, and wherein the T cells are allowed to proliferate for about 6 days. The plurality of anoikis resistant placental stem cells is immunosuppressive if the T cell proliferation at 6 days in the presence of anoikis resistant placental stem cells is detectably reduced compared to T cell proliferation in the presence of DC and absence of placental stem cells. Additionally, a control using unmodified placental stem cells can be run in parallel to demonstrate that the anoikis resistant placental stem cells are more immunosuppressive than unmodified or untreated placental stem cells. In such an MLR, for example, anoikis resistant placental stem cells can be either thawed or harvested from culture. About 20,000 anoikis resistant placental stem cells are resuspended in 100 µl of medium (RPMI 1640, 1 mM HEPES buffer, antibiotics, and 5% pooled human serum), and allowed to attach to the bottom of a well for 2 hours. CD4⁺ and/or CD8⁺ T cells are isolated from whole peripheral blood mononuclear cells Miltenyi magnetic beads. The cells are CFSE stained, and a total of 100,000 T cells (CD4⁺ T cells alone, CD8⁺ T cells alone, or equal amounts of CD4⁺ and CD8⁺ T cells) are added per well. The volume in the well is brought to 200µl, and the MLR is allowed to proceed. A regression assay or BTR assay can be used in similar fashion.

In another aspect, provided herein is a method for promoting angiogenesis. In a specific embodiment, provided herein is a method for promoting angiogenesis in a subject, e.g., a human subject, comprising administering to said subject the arPSCs described herein, or a composition thereof. In certain embodiments, an anoikis resistant placental stem cell provided herein promotes angiogenesis at least 50%, 60%, 70%, 75%, 80%, 85%, 90%, 95%, or 99% as well as an unmodified placental stem cell (e.g., placental stem cells that are not resistant to anoikis). In certain embodiments, an anoikis resistant placental stem cell provided herein promotes angiogenesis to the same extent as an unmodified placental stem cell (e.g., placental stem cells that are not resistant to anoikis). Assays for measuring the ability of cells (e.g., placental stem cells, including arPSCs) to promote angiogenesis are known in the art (see, e.g., U.S. Patent Application Publication No. 2011/0250182, the disclosure of which is herein incorporated by reference in its entirety), e.g., assaying the ability of cells to promote tube formation by endothelial cells, assaying the ability of cells to promote endothelial cell migration and/or proliferation, and assaying the ability of cells to secrete factors that promote angiogenesis.

The anoikis resistant placental stem cells described herein can be administered with one or more second types of stem cells, *e*.*g*., mesenchymal stem cells from bone marrow. Such second stem cells can be administered to an individual with said anoikis resistant placental stem cells in a ratio of, *e*.*g*., about 1:10 to about 10:1.

The anoikis resistant placental stem cells described herein can be administered to an individual in any manner known in the art, *e*.*g*., systemically, locally, intravenously, intramuscularly, intraperitoneally, intraocularly, parenterally, intrathecally, or directly into an organ, *e*.*g*., pancreas. For *in vivo* administration, the anoikis resistant placental stem cells can be formulated as a pharmaceutical composition, as described below.

### 5.3 ANOIKIS RESISTANT PLACENTAL STEM CELLS AND ANOIKIS RESISTANT PLACENTAL STEM CELL POPULATIONS

The anoikis resistant placental stem cells (arPSCs) provided herein are produced from placental stem cells using the methods described herein. In accordance with the methods described herein for producing arPSCs, the arPSCs described herein express one or more anoikis-associated genes (as identified herein, e.g., one or more anoikis associated genes identified in Table 1, above) at a decreased level as compared to the expression of the same anoikis associated gene in an unmodified placental stem cell (i.e., the expression of the one or more anoikis-associated genes is downregulated).

Placental stem cells from which anoikis resistant placental stem cells are produced are not derived from blood, *e*.*g*., placental blood or umbilical cord blood. The placental stem cells used to produce the anoikis resistant placental stem cells used in the methods and compositions provided herein have the capacity, and can be selected for their capacity, to suppress the immune system of an individual.

Placental stem cells can be either fetal or maternal in origin (that is, can have the genotype of either the mother or fetus). Populations of placental stem cells, or populations of cells comprising placental stem cells, can comprise placental stem cells that are solely fetal or maternal in origin, or can comprise a mixed population of placental stem cells of both fetal and maternal origin. The placental stem cells, and populations of cells comprising the placental stem cells, can be identified and selected by, e.g., the morphological, marker, and culture characteristics discussed below.

### 5.3.1 Physical and Morphological Characteristics

The placental stem cells used in the methods described herein for generating arPSCs, when cultured in primary cultures or in cell culture, adhere to the tissue culture substrate, *e*.*g*., tissue culture container surface (*e*.*g*., tissue culture plastic). Placental stem cells in culture assume a generally fibroblastoid, stellate appearance, with a number of cytoplasmic processes extending from the central cell body. The placental stem cells used in the methods for generating arPSCs described herein are, however, morphologically differentiable from fibroblasts cultured under the same conditions, as the placental stem cells exhibit a greater number of such processes than do fibroblasts. Morphologically, placental stem cells are also differentiable from hematopoietic stem cells, which generally assume a more rounded, or cobblestone, morphology in culture.

The arPSCs described herein are thus distinct from, e.g., fibroblasts and hematopoietic stem cells. Further, the arPSCs described herein are distinct from the placental stem cells used to generate the arPSCS, particularly with respect to the ability of the cells to survive in low-attachment conditions; the arPSCs described herein exhibit an increased ability to survive in low-attachment conditions relative to unmodified placental stem cells because they are resistant to anoikis, whereas the unmodified placental stem cells are not anoikis resistant.

### 5.3.2 Cell Surface, Molecular and Genetic Markers

As with unmodified placental stem cells, the arPSCs described herein express a plurality of markers that can be used to identify and/or isolate the arPSCs, or populations of cells that comprise the arPSCs. Generally, the identifying markers associated with the arPSCs described herein are the same as those that can be used to identify the placental stem cells from which the arPSCs are derived (i.e., the placental stem cells used in the methods described herein for generating arPSCs). Thus, the arPSCs described herein are comparable to unmodified to placental stem cells in terms of cell surface, molecular, and genetic markers, with the difference between the cells being that the arPSCs described herein express at least one of anoikis associated gene (e.g., at least one of the genes identified in Table 1, above) at a lower level relative to the expression of said gene in an equivalent amount of unmodified placental stem cells, i.e., at least one anoikis associated gene is downregulated/inhibited in the arPSCs described herein, wherein said anoikis associated gene is not downregulated/inhibited in unmodified placental stem cells.

The arPSCs described herein, like the placental stem cells from which the arPSCs are derived, are not bone marrow-derived mesenchymal cells, adipose-derived mesenchymal stem cells, or mesenchymal cells obtained from umbilical cord blood, placental blood, or peripheral blood.

In certain embodiments, the arPSCs described herein (and/or the placental stem cells used in the methods described herein for producing arPSCs) are CD34⁻, CD10⁺ and CD105⁺ as detected by flow cytometry. In a specific embodiment, the isolated CD34⁻, CD10⁺, CD105⁺ arPSCs described herein (and/or the placental stem cells used in the methods described herein for producing arPSCs) have the potential to differentiate into cells of a neural phenotype, cells of an osteogenic phenotype, and/or cells of a chondrogenic phenotype. In another specific embodiment, the isolated CD34⁻, CD10⁺, CD105⁺ arPSCs described herein (and/or the placental stem cells used in the methods described herein for producing arPSCs) are additionally CD200⁺. In another specific embodiment, the isolated CD34⁻, CD10⁺, CD105⁺ arPSCs described herein (and/or the placental stem cells used in the methods described herein for producing arPSCs) are additionally CD45⁻ or CD90⁺. In another specific embodiment, the isolated CD34⁻, CD10⁺, CD105⁺ arPSCs described herein (and/or the placental stem cells used in the methods described herein for producing arPSCs) are additionally CD45⁻ and CD90⁺, as detected by flow cytometry. In another specific embodiment, the isolated CD34⁻, CD10⁺, CD105⁺, CD200⁺ arPSCs described herein (and/or the placental stem cells used in the methods described herein for producing arPSCs) are additionally CD90⁺ or CD45⁻, as detected by flow cytometry. In another specific embodiment, the isolated CD34⁻, CD10⁺, CD105⁺, CD200⁺ arPSCs described herein (and/or the placental stem cells used in the methods described herein for producing arPSCs) are additionally CD90⁺ and CD45⁻, as detected by flow cytometry, *i*.*e*., the cells are CD34⁻, CD10⁺, CD45⁻, CD90⁺, CD105⁺ and CD200⁺. In another specific embodiment, said CD34⁻, CD10⁺, CD45⁻, CD90⁺, CD105⁺, CD200⁺ arPSCs described herein (and/or the placental stem cells used in the methods described herein for producing arPSCs) are additionally CD80⁻ and CD86⁻.

In certain embodiments, the arPSCs described herein (and/or the placental stem cells used in the methods described herein for producing arPSCs) are CD34⁻, CD10⁺, CD105⁺ and CD200⁺, and one or more of CD38⁻, CD45⁻, CD80⁻, CD86⁻, CD133⁻, HLA-DR,DP,DQ⁻, SSEA3⁻, SSEA4⁻, CD29⁺, CD44⁺, CD73⁺, CD90⁺, CD105⁺, HLA-A,B,C⁺, PDL1⁺, ABC-p⁺, and/or OCT-4⁺, as detected by flow cytometry. In other embodiments, any of the CD34⁻, CD10⁺, CD105⁺ arPSCs described herein (and/or the placental stem cells used in the methods described herein for producing arPSCs) are additionally one or more of CD29⁺, CD38⁻, CD44⁺, CD54⁺, SH3⁺ or SH4⁺. In another specific embodiment, the arPSCs described herein (and/or the placental stem cells used in the methods described herein for producing arPSCs) are additionally CD44⁺. In another specific embodiment of any of the isolated CD34⁻, CD10⁺, CD105⁺ arPSCs described herein (and/or the placental stem cells used in the methods described herein for producing arPSCs) are additionally one or more of CD117⁻, CD133⁻, KDR (VEGFR2⁻), HLA-A,B,C⁺, HLA-DP,DQ,DR⁻, or Programmed Death-1 Ligand (PDL1)⁺, or any combination thereof.

In another embodiment, the CD34⁻, CD10⁺, CD105⁺ arPSCs described herein (and/or the placental stem cells used in the methods described herein for producing arPSCs) are additionally one or more of CD13⁺, CD29⁺, CD33⁺, CD38⁻, CD44⁺, CD45⁻, CD54⁺, CD62E⁻, CD62L⁻, CD62P⁻, SH3⁺ (CD73⁺), SH4⁺ (CD73⁺), CD80⁻, CD86⁻, CD90⁺, SH2⁺ (CD105⁺), CD106/VCAM⁺, CD117⁻, CD144/VE-cadherin^{low}, CD184/CXCR4⁻, CD200⁺, CD133⁻, OCT-4⁺, SSEA3⁻, SSEA4⁻, ABC-p⁺, KDR⁻ (VEGFR2⁻), HLA-A,B,C⁺, HLA-DP,DQ,DR⁻, HLA-G⁻, or Programmed Death-1 Ligand (PDL1)⁺, or any combination thereof. In another embodiment, the CD34⁻, CD10⁺, CD105⁺ arPSCs described herein (and/or the placental stem cells used in the methods described herein for producing arPSCs) are additionally CD13⁺, CD29⁺, CD33⁺, CD38⁻, CD44⁺, CD45⁻, CD54/ICAM⁺, CD62E⁻, CD62L⁻, CD62P⁻, SH3⁺ (CD73⁺), SH4⁺ (CD73⁺), CD80⁻, CD86⁻, CD90⁺, SH2⁺ (CD105⁺), CD106/VCAM⁺, CD117⁻, CD144/VE-cadherin^{low}, CD184/CXCR4⁻, CD200⁺, CD133⁻, OCT-4⁺, SSEA3⁻, SSEA4⁻, ABC-p⁺, KDR⁻ (VEGFR2⁻), HLA-A,B,C⁺, HLA-DP,DQ,DR⁻, HLA-G⁻, and Programmed Death-1 Ligand (PDL1)⁺.

In another specific embodiment, any of the arPSCs described herein (and/or the placental stem cells used in the methods described herein for producing arPSCs) are additionally ABC-p⁺, as detected by flow cytometry, or OCT-4⁺ (POU5F1⁺), as determined by reverse-transcriptase polymerase chain reaction (RT-PCR), wherein ABC-p is a placenta-specific ABC transporter protein (also known as breast cancer resistance protein (BCRP) or as mitoxantrone resistance protein (MXR)), and OCT-4 is the Octamer-4 protein (POU5F1). In another specific embodiment, any of the arPSCs described herein (and/or the placental stem cells used in the methods described herein for producing arPSCs) are additionally SSEA3⁻ or SSEA4⁻, as determined by flow cytometry, wherein SSEA3 is Stage Specific Embryonic Antigen 3, and SSEA4 is Stage Specific Embryonic Antigen 4. In another specific embodiment, any of the arPSCs described herein (and/or the placental stem cells used in the methods described herein for producing arPSCs) are additionally SSEA3⁻ and SSEA4⁻.

In another specific embodiment, any of the arPSCs described herein (and/or the placental stem cells used in the methods described herein for producing arPSCs) are, or are additionally, one or more of MHC-I⁺ (*e*.*g*., HLA-A,B,C⁺), MHC-II⁻ (*e*.*g*., HLA-DP,DQ,DR⁻) or HLA-G⁻. In another specific embodiment, any of the arPSCs described herein (and/or the placental stem cells used in the methods described herein for producing arPSCs) are additionally MHC-I⁺ (*e*.*g*., HLA-A,B,C⁺), MHC-II⁻ (*e*.*g*., HLA-DP,DQ,DR⁻) and HLA-G⁻.

Also provided herein are populations of the arPSCs described herein. In certain embodiments, described herein are populations of arPSCs comprising the isolated arPSCs described herein, wherein the populations of cells comprise, *e*.*g*., at least 10%, 15%, 20%, 25%, 30%, 35%, 40%, 45%, 50%, 55%, 60%, 65%, 70%, 75%, 80%, 85%, 90%, 95% or 98% isolated CD10⁺, CD105⁺ and CD34⁻ arPSCs; that is, at least 10%, 15%, 20%, 25%, 30%, 35%, 40%, 45%, 50%, 55%, 60%, 65%, 70%, 75%, 80%, 85%, 90%, 95% or 98% of cells in said population are isolated CD10⁺, CD105⁺ and CD34⁻ arPSCs. In a specific embodiment, the isolated CD34⁻, CD10⁺, CD105⁺ arPSCs are additionally CD200⁺. In another specific embodiment, the isolated CD34⁻, CD10⁺, CD105⁺, CD200⁺ arPSCs are additionally CD90⁺ or CD45⁻, as detected by flow cytometry. In another specific embodiment, the isolated CD34⁻, CD10⁺, CD105⁺, CD200⁺ arPSCs are additionally CD90⁺ and CD45⁻, as detected by flow cytometry. In another specific embodiment, any of the isolated CD34⁻, CD10⁺, CD105⁺ arPSCs described above are additionally one or more of CD29⁺, CD38⁻, CD44⁺, CD54⁺, SH3⁺ or SH4⁺. In another specific embodiment, the isolated CD34⁻, CD10⁺, CD105⁺ arPSCs, or isolated CD34⁻, CD10⁺, CD105⁺, CD200⁺ placental stem cells, are additionally CD44⁺. In a specific embodiment of any of the populations of cells comprising isolated CD34⁻, CD10⁺, CD105⁺ arPSCs above, the isolated arPSCs are additionally one or more of CD13⁺, CD29⁺, CD33⁺, CD38⁻, CD44⁺, CD45⁻, CD54⁺, CD62E⁻, CD62L⁻, CD62P⁻, SH3⁺ (CD73⁺), SH4⁺ (CD73⁺), CD80⁻, CD86⁻, CD90⁺, SH2⁺ (CD105⁻), CD106/VCAM⁺, CD117⁻, CD144/VE-cadherin^{low}, CD184/CXCR4⁻, CD200⁺, CD133⁻, OCT-4⁺, SSEA3⁻, SSEA4⁻, ABC-p⁺, KDR⁻ (VEGFR2⁻), HLA-A,B,C⁺, HLA-DP,DQ,DR⁻, HLA-G⁻, or Programmed Death-1 Ligand (PDL1)⁺, or any combination thereof. In another specific embodiment, the CD34⁻, CD10⁺, CD105⁺ arPSCs are additionally CD13⁺, CD29⁺, CD33⁺, CD38⁻, CD44⁺, CD45⁻, CD54/ICAM⁺, CD62E⁻, CD62L⁻, CD62P⁻, SH3⁺ (CD73⁺), SH4⁺ (CD73⁺), CD80⁻, CD86⁻, CD90⁺, SH2⁺ (CD105⁺), CD106/VCAM⁺, CD117⁻, CD144/VE-cadherin^{low}, CD184/CXCR4⁻, CD200⁺, CD133⁻, OCT-4⁺, SSEA3⁻, SSEA4⁻, ABC-p⁺, KDR⁻ (VEGFR2⁻), HLA-A,B,C⁺, HLA-DP,DQ,DR⁻, HLA-G⁻, and Programmed Death-1 Ligand (PDL1)⁺.

In certain embodiments, the isolated arPSCs in said population of cells are one or more, or all, of CD10⁺, CD29⁺, CD34⁻, CD38⁻, CD44⁺, CD45⁻, CD54⁺, CD90⁺, SH2⁺, SH3⁺, SH4⁺, SSEA3⁻, SSEA4⁻, OCT-4⁺, and ABC-p⁺, wherein said the placental stem cells used in the method of generating said isolated arPSCs were obtained by physical and/or enzymatic disruption of placental tissue. In a specific embodiment, the isolated arPSCs are OCT-4⁺ and ABC-p⁺. In another specific embodiment, the isolated arPSCs are OCT-4⁺ and CD34⁻, wherein said isolated arPSCs have at least one of the following characteristics: CD10⁺, CD29⁺, CD44⁺, CD45⁻, CD54⁺, CD90⁺, SH3⁺, SH4⁺, SSEA3⁻, and SSEA4⁻. In another specific embodiment, the isolated arPSCs are OCT-4⁺, CD34⁻, CD10⁺, CD29⁺, CD44⁺, CD45⁻, CD54⁺, CD90⁺, SH3⁺, SH4⁺, SSEA3⁻, and SSEA4⁻. In another embodiment, the isolated arPSCs are OCT-4⁺, CD34⁻, SSEA3⁻, and SSEA4⁻. In another specific embodiment, the isolated arPSCs are OCT-4⁺ and CD34⁻, and is either SH2⁺ or SH3⁺. In another specific embodiment, the isolated arPSCs are OCT-4⁺, CD34⁻, SH2⁺, and SH3⁺. In another specific embodiment, the isolated arPSCs are OCT-4⁺, CD34⁻, SSEA3⁻, and SSEA4⁻, and are either SH2⁺ or SH3⁺. In another specific embodiment, the isolated arPSCs are OCT-4⁺ and CD34⁻, and either SH2⁺ or SH3⁺, and at least one of CD10⁺, CD29⁺, CD44⁺, CD45⁻, CD54⁺, CD90⁺, SSEA3⁻, or SSEA4⁻. In another specific embodiment, the isolated arPSCs are OCT-4⁺, CD34⁻, CD10⁺, CD29⁺, CD44⁺, CD45⁻, CD54⁺, CD90⁺, SSEA3⁻, and SSEA4⁻, and either SH2⁺ or SH3⁺.

In another embodiment, the isolated arPSCs are SH2⁺, SH3⁺, SH4⁺ and OCT-4⁺. In another specific embodiment, the isolated arPSCs are CD10⁺, CD29⁺, CD44⁺, CD54⁺, CD90⁺, CD34⁻, CD45⁻, SSEA3⁻, or SSEA4⁻. In another embodiment, the isolated arPSCs are SH2⁺, SH3⁺, SH4⁺, SSEA3⁻ and SSEA4⁻. In another specific embodiment, the isolated arPSCs are SH2⁺, SH3⁺, SH4⁺, SSEA3⁻ and SSEA4⁻, CD10⁺, CD29⁺, CD44⁺, CD54⁺, CD90⁺, OCT-4⁺, CD34⁻ or CD45⁻.

In another embodiment, the isolated arPSCs described herein are CD10⁺, CD29⁺, CD34⁻, CD44⁺, CD45⁻, CD54⁺, CD90⁺, SH2⁺, SH3⁺, and SH4⁺; wherein said isolated arPSCs are additionally one or more of OCT-4⁺, SSEA3⁻ or SSEA4⁻.

In certain embodiments, isolated arPSCs are CD200⁺ or HLA-G⁻. In a specific embodiment, the isolated arPSCs are CD200⁺ and HLA-G⁻. In another specific embodiment, the isolated arPSCs are additionally CD73⁺ and CD105⁺. In another specific embodiment, the isolated arPSCs are additionally CD34⁻, CD38⁻ or CD45⁻. In another specific embodiment, the isolated arPSCs are additionally CD34⁻, CD38⁻ and CD45⁻. In another specific embodiment, said arPSCs are CD34⁻, CD38⁻, CD45⁻, CD73⁺ and CD105⁺. In another specific embodiment, said isolated CD200⁺ or HLA-G⁻ arPSCs facilitate the formation of embryoid-like bodies in a population of placental cells comprising the isolated placental stem cells, under conditions that allow the formation of embryoid-like bodies. In another specific embodiment, the isolated arPSCs are isolated away from placental cells that are not said arPSCs. In another specific embodiment, said isolated arPSCs are isolated away from placental cells that do not display this combination of markers.

In another embodiment, a cell population useful in the methods and compositions described herein is a population of cells comprising, *e*.*g*., that is enriched for, CD200⁺, HLA-G⁻ arPSCs. In a specific embodiment, said population is a population of placental cells. In various embodiments, at least about 10%, at least about 20%, at least about 30%, at least about 40%, at least about 50%, or at least about 60% of cells in said cell population are isolated CD200⁺, HLA-G⁻ arPSCs. Preferably, at least about 70% of cells in said cell population are isolated CD200⁺, HLA-G⁻ arPSCs. More preferably, at least about 90%, 95%, or 99% of said cells are isolated CD200⁺, HLA-G⁻ arPSCs. In a specific embodiment of the cell populations, said isolated CD200⁺, HLA-G⁻ arPSCs are also CD73⁺ and CD105⁺. In another specific embodiment, said isolated CD200⁺, HLA-G⁻ arPSCs are also CD34⁻, CD38⁻ or CD45⁻. In another specific embodiment, said isolated CD200⁺, HLA-G⁻ arPSCs are also CD34⁻, CD38⁻, CD45⁻, CD73⁺ and CD105⁺. In another embodiment, said cell population produces one or more embryoid-like bodies when cultured under conditions that allow the formation of embryoid-like bodies. In another specific embodiment, said cell population is isolated away from placental cells that are not arPSCs. In another specific embodiment, said isolated CD200⁺, HLA-G⁻ arPSCs are isolated away from placental cells that do not display these markers.

In another embodiment, the isolated arPSCs described herein are CD73⁺, CD105⁺, and CD200⁺. In another specific embodiment, the isolated arPSCs are HLA-G⁻. In another specific embodiment, the isolated arPSCs are CD34⁻, CD38⁻ or CD45⁻. In another specific embodiment, the isolated arPSCs are CD34⁻, CD38⁻ and CD45⁻. In another specific embodiment, the isolated arPSCs are CD34⁻, CD38⁻, CD45⁻, and HLA-G⁻. In another specific embodiment, the isolated CD73⁺, CD105⁺, and CD200⁺ arPSCs facilitate the formation of one or more embryoid-like bodies in a population of placental cells comprising the isolated arPSCs, when the population is cultured under conditions that allow the formation of embryoid-like bodies. In another specific embodiment, the isolated arPSCs are isolated away from placental cells that are not the isolated arPSCs. In another specific embodiment, the isolated arPSCs are isolated away from placental cells that do not display these markers.

In another embodiment, a cell population useful in the methods and compositions described herein is a population of cells comprising, e.g., that is enriched for, isolated CD73⁺, CD105⁺, CD200⁺ arPSCs. In various embodiments, at least about 10%, at least about 20%, at least about 30%, at least about 40%, at least about 50%, or at least about 60% of cells in said cell population are isolated CD73⁺, CD105⁺, CD200⁺ arPSCs. In another embodiment, at least about 70% of said cells in said population of cells are isolated CD73⁺, CD105⁺, CD200⁺ arPSCs. In another embodiment, at least about 90%, 95% or 99% of cells in said population of cells are isolated CD73⁺, CD105⁺, CD200⁺ arPSCs. In a specific embodiment of said populations, the isolated arPSCs are HLA-G⁻. In another specific embodiment, the isolated arPSCs are additionally CD34⁻, CD38⁻ or CD45⁻. In another specific embodiment, the isolated arPSCs are additionally CD34⁻, CD38⁻ and CD45⁻. In another specific embodiment, the isolated arPSCs are additionally CD34⁻, CD38⁻, CD45⁻, and HLA-G⁻. In another specific embodiment, said population of cells produces one or more embryoid-like bodies when cultured under conditions that allow the formation of embryoid-like bodies. In another specific embodiment, said population of arPSCs is isolated away from placental cells that are not arPSCs. In another specific embodiment, said population of arPSCs is isolated away from placental cells that do not display these characteristics.

In certain other embodiments, the isolated arPSCs are one or more of CD10⁺, CD29⁺, CD34⁻, CD38⁻, CD44⁺, CD45⁻, CD54⁺, CD90⁺, SH2⁺, SH3⁺, SH4⁺, SSEA3-, SSEA4⁻, OCT-4⁺, HLA-G⁻ or ABC-p⁺. In a specific embodiment, the isolated arPSCs are CD10⁺, CD29⁺, CD34⁻, CD38⁻, CD44⁺, CD45⁻, CD54⁺, CD90⁺, SH2⁺, SH3⁺, SH4⁺, SSEA3-, SSEA4⁻, and OCT-4⁺. In another specific embodiment, the isolated arPSCs are CD10⁺, CD29⁺, CD34⁻, CD38⁻, CD45⁻, CD54⁺, SH2⁺, SH3⁺, and SH4⁺. In another specific embodiment, the isolated arPSCs CD10⁺, CD29⁺, CD34⁻, CD38⁻, CD45⁻, CD54⁺, SH2⁺, SH3⁺, SH4⁺ and OCT-4⁺. In another specific embodiment, the isolated arPSCs are CD10⁺, CD29⁺, CD34⁻, CD38⁻, CD44⁺, CD45⁻, CD54⁺, CD90⁺, HLA-G⁻, SH2⁺, SH3⁺, SH4⁺. In another specific embodiment, the isolated arPSCs are OCT-4⁺ and ABC-p⁺. In another specific embodiment, the isolated arPSCs are SH2⁺, SH3⁺, SH4⁺ and OCT-4⁺. In another embodiment, the isolated arPSCs are OCT-4⁺, CD34⁻, SSEA3⁻, and SSEA4⁻. In a specific embodiment, said isolated OCT-4⁺, CD34⁻, SSEA3⁻ , and SSEA4⁻ arPSCs are additionally CD10⁺, CD29⁺, CD34⁻, CD44⁺, CD45⁻, CD54⁺, CD90⁺, SH2⁺, SH3⁺, and SH4⁺. In another embodiment, the isolated arPSCs are OCT-4⁺ and CD34⁻, and either SH3⁺ or SH4⁺. In another embodiment, the isolated arPSCs are CD34⁻ and either CD10⁺, CD29⁺, CD44⁺, CD54⁺, CD90⁺, or OCT-4⁺.

In another embodiment, isolated arPSCs are CD200⁺ and OCT-4⁺. In a specific embodiment, the isolated arPSCs are CD73⁺ and CD105⁺. In another specific embodiment, said isolated arPSCs are HLA-G⁻. In another specific embodiment, said isolated CD200⁺, OCT-4⁺ arPSCs are CD34⁻, CD38⁻ or CD45⁻. In another specific embodiment, said isolated CD200⁺, OCT-4⁺ arPSCs are CD34⁻, CD38⁻ and CD45⁻. In another specific embodiment, said isolated CD200⁺, OCT-4⁺ arPSCs are CD34⁻, CD38⁻, CD45⁻, CD73⁺, CD105⁺ and HLA-G⁻. In another specific embodiment, the isolated CD200⁺, OCT-4⁺ arPSCs facilitate the production of one or more embryoid-like bodies by a population of placental cells that comprises the arPSCs, when the population is cultured under conditions that allow the formation of embryoid-like bodies. In another specific embodiment, said isolated CD200⁺, OCT-4⁺ arPSCs are isolated away from placental cells that are not said arPSCs. In another specific embodiment, said isolated CD200⁺, OCT-4⁺ arPSCs are isolated away from placental cells that do not display these characteristics.

In another embodiment, a cell population useful in the methods and compositions described herein is a population of cells comprising, *e*.*g*., that is enriched for, CD200⁺, OCT-4⁺ arPSCs. In various embodiments, at least about 10%, at least about 20%, at least about 30%, at least about 40%, at least about 50%, or at least about 60% of cells in said cell population are isolated CD200⁺, OCT-4⁺ arPSCs. In another embodiment, at least about 70% of said cells are said isolated CD200⁺, OCT-4⁺ arPSCs. In another embodiment, at least about 80%, 90%, 95%, or 99% of cells in said cell population are said isolated CD200⁺, OCT-4⁺ arPSCs. In a specific embodiment of the isolated populations, said isolated CD200⁺, OCT-4⁺ arPSCs are additionally CD73⁺ and CD105⁺. In another specific embodiment, said isolated CD200⁺, OCT-4⁺ arPSCs are additionally HLA-G⁻. In another specific embodiment, said isolated CD200⁺, OCT-4⁺ arPSCs are additionally CD34⁻, CD38⁻ and CD45⁻. In another specific embodiment, said isolated CD200⁺, OCT-4⁺ arPSCs are additionally CD34⁻, CD38⁻, CD45⁻, CD73⁺, CD105⁺ and HLA-G⁻. In another specific embodiment, the cell population produces one or more embryoid-like bodies when cultured under conditions that allow the formation of embryoid-like bodies. In another specific embodiment, said cell population is isolated away from placental cells that are not isolated CD200⁺, OCT-4⁺ arPSCs. In another specific embodiment, said cell population is isolated away from placental cells that do not display these markers.

In another embodiment, the isolated arPSCs useful in the methods and compositions described herein are CD73⁺, CD105⁺ and HLA-G⁻. In another specific embodiment, the isolated CD73⁺, CD105⁺ and HLA-G⁻ arPSCs are additionally CD34⁻, CD38⁻ or CD45⁻. In another specific embodiment, the isolated CD73⁺, CD105⁺, HLA-G⁻ arPSCs are additionally CD34⁻, CD38⁻ and CD45⁻. In another specific embodiment, the isolated CD73⁺, CD105+, HLA-G⁻ arPSCs are additionally OCT-4⁺. In another specific embodiment, the isolated CD73⁺, CD105⁺, HLA-G⁻ arPSCs are additionally CD200⁺. In another specific embodiment, the isolated CD73⁺, CD105⁺, HLA-G⁻ arPSCs are additionally CD34⁻, CD38⁻, CD45⁻, OCT-4⁺ and CD200⁺. In another specific embodiment, the isolated CD73⁺, CD105⁺, HLA-G⁻ arPSCs facilitate the formation of embryoid-like bodies in a population of placental cells comprising said arPSCs, when the population is cultured under conditions that allow the formation of embryoid-like bodies. In another specific embodiment, the isolated CD73⁺, CD105⁺, HLA-G⁻ arPSCs are isolated away from placental cells that are not the isolated CD73⁺, CD105⁺, HLA-G⁻ arPSCs. In another specific embodiment, said the isolated CD73⁺, CD105⁺, HLA-G⁻ arPSCs are isolated away from placental cells that do not display these markers.

In another embodiment, a cell population useful in the methods and compositions described herein is a population of cells comprising, *e*.*g*., that is enriched for, isolated CD73⁺, CD105⁺ and HLA-G⁻ arPSCs. In various embodiments, at least about 10%, at least about 20%, at least about 30%, at least about 40%, at least about 50%, or at least about 60% of cells in said population of cells are isolated CD73⁺, CD105⁺, HLA-G⁻ arPSCs. In another embodiment, at least about 70% of cells in said population of cells are isolated CD73⁺, CD105⁺, HLA-G⁻ arPSCs. In another embodiment, at least about 90%, 95% or 99% of cells in said population of cells are isolated CD73⁺, CD105⁺, HLA-G⁻ arPSCs. In a specific embodiment of the above populations, said isolated CD73⁺, CD105⁺, HLA-G⁻ arPSCs are additionally CD34⁻, CD38⁻ or CD45⁻. In another specific embodiment, said isolated CD73⁺, CD105⁺, HLA-G⁻ arPSCs are additionally CD34⁻, CD38⁻ and CD45⁻. In another specific embodiment, said isolated CD73⁺, CD105⁺, HLA-G⁻ arPSCs are additionally OCT-4⁺. In another specific embodiment, said isolated CD73⁺, CD105⁺, HLA-G⁻ arPSCs are additionally CD200⁺. In another specific embodiment, said isolated CD73⁺, CD105⁺, HLA-G⁻ arPSCs are additionally CD34⁻, CD38⁻, CD45⁻, OCT-4⁺ and CD200⁺. In another specific embodiment, said cell population is isolated away from placental cells that are not CD73⁺, CD105⁺, HLA-G⁻ arPSCs. In another specific embodiment, said cell population is isolated away from placental cells that do not display these markers.

In another embodiment, the isolated arPSCs are CD73⁺ and CD105⁺ and facilitate the formation of one or more embryoid-like bodies in a population of isolated placental cells comprising said CD73⁺, CD105⁺ cells when said population is cultured under conditions that allow formation of embryoid-like bodies. In another specific embodiment, said isolated CD73⁺, CD105⁺ arPSCs are additionally CD34⁻, CD38⁻ or CD45⁻. In another specific embodiment, said isolated CD73⁺, CD105⁺ arPSCs are additionally CD34⁻, CD38⁻ and CD45⁻. In another specific embodiment, said isolated CD73⁺, CD105⁺ arPSCs are additionally OCT-4⁺. In another specific embodiment, said isolated CD73⁺, CD105⁺ arPSCs are additionally OCT-4⁺, CD34⁻, CD38⁻ and CD45⁻. In another specific embodiment, said isolated CD73⁺, CD105⁺ arPSCs are isolated away from placental cells that are not said cells. In another specific embodiment, said isolated CD73⁺, CD105⁺ arPSCs are isolated away from placental cells that do not display these characteristics.

In another embodiment, a cell population useful in the methods and compositions described herein is a population of cells comprising, *e*.*g*., that is enriched for, isolated arPSCs that are CD73⁺, CD105⁺ and facilitate the formation of one or more embryoid-like bodies in a population of isolated placental cells comprising said cells when said population is cultured under conditions that allow formation of embryoid-like bodies. In various embodiments, at least about 10%, at least about 20%, at least about 30%, at least about 40%, at least about 50%, or at least about 60% of cells in said population of cells are said isolated CD73⁺, CD105⁺ arPSCs. In another embodiment, at least about 70% of cells in said population of cells are said isolated CD73⁺, CD105⁺ arPSCs. In another embodiment, at least about 90%, 95% or 99% of cells in said population of cells are said isolated CD73⁺, CD105⁺ arPSCs. In a specific embodiment of the above populations, said isolated CD73⁺, CD105⁺ arPSCs are additionally CD34⁻, CD38⁻ or CD45⁻. In another specific embodiment, said isolated CD73⁺, CD105⁺ arPSCs are additionally CD34⁻, CD38⁻ and CD45⁻. In another specific embodiment, said isolated CD73⁺, CD105⁺ arPSCs are additionally OCT-4⁺. In another specific embodiment, said isolated CD73⁺, CD105⁺ arPSCs are additionally CD200⁺. In another specific embodiment, said isolated CD73⁺, CD105⁺ arPSCs are additionally CD34⁻, CD38⁻, CD45⁻, OCT-4⁺ and CD200⁺. In another specific embodiment, said cell population is isolated away from placental cells that are not said isolated CD73⁺, CD105⁺ arPSCs. In another specific embodiment, said cell population is isolated away from placental cells that do not display these markers.

In another embodiment, the isolated arPSCs are OCT-4⁺ and facilitate formation of one or more embryoid-like bodies in a population of isolated placental cells comprising said arPSCs when said population of cells is cultured under conditions that allow formation of embryoid-like bodies. In a specific embodiment, said isolated OCT-4⁺ arPSCs are additionally CD73⁺ and CD105⁺. In another specific embodiment, said isolated OCT-4⁺ arPSCs are additionally CD34⁻, CD38⁻, or CD45⁻. In another specific embodiment, said isolated OCT-4⁺ arPSCs are additionally CD200⁺. In another specific embodiment, said isolated OCT-4⁺ arPSCs are additionally CD73⁺, CD105⁺, CD200⁺, CD34⁻, CD38⁻, and CD45⁻. In another specific embodiment, said isolated OCT-4⁺ arPSCs are isolated away from placental cells that are not OCT-4⁺ arPSCs. In another specific embodiment, said isolated OCT-4⁺ arPSCs are isolated away from placental cells that do not display these characteristics.

In another embodiment, a cell population useful in the methods and compositions described herein is a population of cells comprising, *e*.*g*., that is enriched for, isolated arPSCs that are OCT-4⁺ and facilitate the formation of one or more embryoid-like bodies in a population of isolated placental cells comprising said cells when said population is cultured under conditions that allow formation of embryoid-like bodies. In various embodiments, at least about 10%, at least about 20%, at least about 30%, at least about 40%, at least about 50%, or at least about 60% of cells in said population of cells are said isolated OCT-4⁺ arPSCs. In another embodiment, at least about 70% of cells in said population of cells are said isolated OCT-4⁺ arPSCs. In another embodiment, at least about 80%, 90%, 95% or 99% of cells in said population of cells are said isolated OCT-4⁺ arPSCs. In a specific embodiment of the above populations, said isolated OCT-4⁺ arPSCs are additionally CD34⁻, CD38⁻ or CD45⁻. In another specific embodiment, said isolated OCT-4⁺ arPSCs are additionally CD34⁻, CD38⁻ and CD45⁻. In another specific embodiment, said isolated OCT-4⁺ arPSCs are additionally CD73⁺ and CD105⁺. In another specific embodiment, said isolated OCT-4⁺ arPSCs are additionally CD200⁺. In another specific embodiment, said isolated OCT-4⁺ arPSCs are additionally CD73⁺, CD105⁺, CD200⁺, CD34⁻, CD38⁻, and CD45⁻. In another specific embodiment, said cell population is isolated away from placental cells that are not said arPSCs. In another specific embodiment, said cell population is isolated away from placental cells that do not display these markers.

In another embodiment, the isolated placental stem cells useful in the methods and compositions described herein are isolated HLA-A,B,C⁺, CD45⁻, CD133⁻ and CD34⁻ arPSCs. In another embodiment, a cell population useful in the methods and compositions described herein is a population of cells comprising isolated arPSCs, wherein at least about 70%, at least about 80%, at least about 90%, at least about 95% or at least about 99% of cells in said population of cells are isolated HLA-A,B,C⁺, CD45⁻, CD133⁻ and CD34⁻ arPSCs. In a specific embodiment, said isolated arPSCs or population of isolated arPSCs is isolated away from placental cells that are not HLA-A,B,C⁺, CD45⁻, CD133⁻ and CD34⁻ arPSCs. In another specific embodiment, said isolated arPSCs are non-maternal in origin. In another specific embodiment, said population of isolated arPSCs are substantially free of maternal components; *e*.*g*., at least about 40%, 45%, 5-0%, 55%, 60%, 65%, 70%, 75%, 90%, 85%, 90%, 95%, 98% or 99% of said cells in said population of isolated arPSCs are non-maternal in origin.

In another embodiment, the isolated arPSCs useful in the methods and compositions described herein are isolated CD10⁺, CD13⁺, CD33⁺, CD45⁻, CD117⁻ and CD133⁻ arPSCs. In another embodiment, a cell population useful in the methods and compositions described herein is a population of cells comprising isolated arPSCs, wherein at least about 70%, at least about 80%, at least about 90%, at least about 95% or at least about 99% of cells in said population of cells are isolated CD10⁺, CD13⁺, CD33⁺, CD45⁻, CD117⁻ and CD133⁻ arPSCs. In a specific embodiment, said isolated arPSCs or population of isolated arPSCs is isolated away from placental cells that are not said isolated arPSCs. In another specific embodiment, said isolated CD10⁺, CD13⁺, CD33⁺, CD45⁻, CD117⁻ and CD133⁻ arPSCs are non-maternal in origin, *i*.*e*., have the fetal genotype. In another specific embodiment, at least about 40%, 45%, 50%, 55%, 60%, 65%, 70%, 75%, 90%, 85%, 90%, 95%, 98% or 99% of said cells in said population of isolated arPSCs, are non-maternal in origin. In another specific embodiment, said isolated arPSCs or population of isolated arPSCs are isolated away from placental cells that do not display these characteristics.

In another embodiment, the isolated arPSCs are isolated CD10⁺ CD33⁻, CD44⁺, CD45⁻, and CD117⁻ arPSCs. In another embodiment, a cell population useful for the in the methods and compositions described herein is a population of cells comprising, *e*.*g*., enriched for, isolated arPSCs, wherein at least about 70%, at least about 80%, at least about 90%, at least about 95% or at least about 99% of cells in said population of cells are isolated CD10⁺ CD33⁻, CD44⁺, CD45⁻, and CD117⁻ arPSCs. In a specific embodiment, said isolated arPSCs or population of isolated arPSCs is isolated away from placental cells that are not said cells. In another specific embodiment, said isolated arPSCs are non-maternal in origin. In another specific embodiment, at least about 40%, 45%, 50%, 55%, 60%, 65%, 70%, 75%, 90%, 85%, 90%, 95%, 98% or 99% of said arPSCs in said cell population are non-maternal in origin. In another specific embodiment, said isolated arPSCs or population of isolated arPSCs is isolated away from placental cells that do not display these markers.

In another embodiment, the isolated arPSCs useful in the methods and compositions described herein are isolated CD10⁺ CD13⁻, CD33⁻, CD45⁻, and CD117⁻ arPSCs. In another embodiment, a cell population useful in the methods and compositions described herein is a population of cells comprising, *e*.*g*., enriched for, isolated CD10⁺, CD13⁻, CD33⁻, CD45⁻, and CD117⁻ arPSCs, wherein at least about 70%, at least about 80%, at least about 90%, at least about 95% or at least about 99% of cells in said population are CD10+ CD13⁻, CD33⁻, CD45⁻, and CD117⁻ arPSCs. In a specific embodiment, said isolated arPSCs or population of isolated arPSCs are isolated away from placental cells that are not said arPSCs. In another specific embodiment, said isolated placental cells are non-maternal in origin. In another specific embodiment, at least about 40%, 45%, 50%, 55%, 60%, 65%, 70%, 75%, 90%, 85%, 90%, 95%, 98% or 99% of said cells in said cell population are non-maternal in origin. In another specific embodiment, said isolated arPSCs or population of isolated arPSCs is isolated away from placental cells that do not display these characteristics.

In another embodiment, the isolated arPSCs useful in the methods and compositions described herein are HLA A,B,C⁺, CD45⁻, CD34⁻, and CD133⁻, and are additionally CD10⁺, CD13⁺, CD38⁺, CD44⁺, CD90⁺, CD105⁺, CD200⁺ and/or HLA-G⁻, and/or negative for CD117. In another embodiment, a cell population useful in the methods described herein is a population of cells comprising isolated arPSCs, wherein at least about 20%, 25%, 30%, 35%, 40%, 45%, 50%, 55%, 60%, 65%, 70%, 75%, 80%, 85%, 90%, 95%, 98% or about 99% of the cells in said population are isolated arPSCs that are HLA A,B,C⁻, CD45⁻, CD34⁻, CD133⁻, and that are additionally positive for CD10, CD13, CD38, CD44, CD90, CD105, CD200, and/or negative for CD117 and/or HLA-G. In a specific embodiment, said isolated arPSCs or population of isolated arPSCs are isolated away from placental cells that are not said arPSCs. In another specific embodiment, said isolated arPSCs are non-maternal in origin. In another specific embodiment, at least about 40%, 45%, 50%, 55%, 60%, 65%, 70%, 75%, 90%, 85%, 90%, 95%, 98% or 99% of said arPSCs in said cell population are non-maternal in origin. In another specific embodiment, said isolated arPSCs or population of isolated arPSCs are isolated away from placental cells that do not display these characteristics.

In another embodiment, the isolated arPSCs are isolated arPSCs that are CD200⁺ and CD10⁺, as determined by antibody binding, and CD117⁻, as determined by both antibody binding and RT-PCR. In another embodiment, the isolated arPSCs are isolated placental stem cells that are CD10⁺, CD29⁻, CD54⁺, CD200⁺, HLA-G⁻, MHC class I⁺ and β-2-microglobulin⁺. In another embodiment, isolated arPSCs useful in the methods and compositions described herein are arPSCs wherein the expression of at least one cellular marker is at least two-fold higher than in an equivalent number of mesenchymal stem cells, *e*.*g*., bone marrow-derived mesenchymal stem cells. In another specific embodiment, said isolated arPSCs are non-maternal in origin. In another specific embodiment, at least about 40%, 45%, 50%, 55%, 60%, 65%, 70%, 75%, 90%, 85%, 90%, 95%, 98% or 99% of said cells in said cell population are non-maternal in origin.

In another embodiment, the isolated arPSCs are isolated arPSCs that are one or more of CD10⁺, CD29⁺, CD44⁺, CD45⁻, CD54/ICAM⁺, CD62E⁻, CD62L⁻, CD62P⁻, CD80⁻, CD86⁻, CD103⁻, CD104⁻, CD105⁺, CD106/VCAM⁺, CD144/VE-cadherin^{low}, CD184/CXCR4⁻, β2-microglobulin^{low}, MHC-I^{low}, MHC-II⁻, HLA-G^{low}, and/or PDL1^{low}. In a specific embodiment, the isolated arPSCs are at least CD29⁺ and CD54⁺. In another specific embodiment, the isolated arPSCs are at least CD44⁺ and CD106⁺. In another specific embodiment, the isolated arPSCs are at least CD29⁺.

In another embodiment, a cell population useful in the methods and compositions described herein comprises isolated arPSCs, and at least 50%, 60%, 70%, 80%, 90%, 95%, 98% or 99% of the cells in said cell population are isolated arPSCs that are one or more of CD10⁺, CD29⁺, CD44⁺, CD45⁻, CD54/ICAM⁺, CD62-E⁻, CD62-L⁻, CD62-P⁻, CD80⁻, CD86⁻, CD103⁻, CD104⁻, CD105⁺, CD106/VCAM⁺, CD144/VE-cadherin^{dim}, CD184/CXCR4⁻, β2-microglobulin^{dim}, HLA-I^{dim}, HLA-II⁻, HLA-G^{dim}, and/or PDL1^{dim} arPSCs. In another specific embodiment, at least 50%, 60%, 70%, 80%, 90%, 95%, 98% or 99% of cells in said cell population are CD10⁺, CD29⁺, CD44⁺, CD45⁻, CD54/ICAM⁺, CD62-E⁻, CD62-L⁻, CD62-P⁻, CD80⁻, CD86⁻, CD103⁻, CD104⁻, CD105⁺, CD106/VCAM⁺, CD144/VE-cadherin^{dim}, CD184/CXCR4⁻, β2-microglobulin^{dim}, MHC-I^{dim}, MHC-II⁻, HLA-G^{dim}, and PDL1^{dim} arPSCs. In certain embodiments, the arPSCs express HLA-II markers when induced by interferon gamma (IFN-γ).

In another embodiment, the isolated arPSCs useful in the methods and compositions described herein are isolated arPSCs that are one or more, or all, of CD10⁺, CD29⁺, CD34⁻, CD38⁻, CD44⁺, CD45⁻, CD54⁺, CD90⁺, SH2⁺, SH3⁺, SH4⁺, SSEA3⁻, SSEA4⁻, OCT-4⁺, and ABC-p⁺, where ABC-p is a placenta-specific ABC transporter protein (also known as breast cancer resistance protein (BCRP) or as mitoxantrone resistance protein (MXR)), wherein said isolated arPSCs are derived from placental stem cells obtained by perfusion of a mammalian, *e*.*g*., human, placenta that has been drained of cord blood and perfused to remove residual blood.

In another specific embodiment of any of the above embodiments, expression of the recited cellular marker(s) (*e*.*g*., cluster of differentiation or immunogenic marker(s)) is determined by flow cytometry. In another specific embodiment, expression of the marker(s) is determined by RT-PCR.

Gene profiling confirms that isolated arPSCs, and populations of isolated arPSCs, are distinguishable from other cells, *e*.*g*., mesenchymal stem cells, *e*.*g*., bone marrow-derived mesenchymal stem cells. The isolated arPSCs described herein can be distinguished from, *e*.*g*., bone marrow-derived mesenchymal stem cells on the basis of the expression of one or more genes, the expression of which is significantly higher in the isolated arPSCs in comparison to bone marrow-derived mesenchymal stem cells. In particular, the isolated arPSCs, useful in the methods of treatment provided herein, can be distinguished from bone marrow-derived mesenchymal stem cells on the basis of the expression of one or more genes, the expression of which is significantly higher (that is, at least twofold higher) in the isolated arPSCs than in an equivalent number of bone marrow-derived mesenchymal stem cells, wherein the one or more gene comprise ACTG2, ADARB1, AMIGO2, ARTS-1, B4GALT6, BCHE, C11orf9, CD200, COL4A1, COL4A2, CPA4, DMD, DSC3, DSG2, ELOVL2, F2RL1, FLJ10781, GATA6, GPR126, GPRC5B, ICAM1, IER3, IGFBP7, ILIA, IL6, IL18, KRT18, KRT8, LIPG, LRAP, MATN2, MEST, NFE2L3, NUAK1, PCDH7, PDLIM3, PKP2, RTN1, SERPINB9, ST3GAL6, ST6GALNAC5, SLC12A8, TCF21, TGFB2, VTN, ZC3H12A, or a combination of any of the foregoing, when the cells are grown under equivalent conditions. *See*, *e*.*g*., U.S. Patent Application Publication No. 2007/0275362, the disclosure of which is incorporated herein by reference in its entirety. In certain specific embodiments, said expression of said one or more genes is determined, *e*.*g*., by RT-PCR or microarray analysis, *e*.*g*., using a U133-A microarray (Affymetrix).

In another specific embodiment, said isolated arPSCs express said one or more genes when cultured for a number of population doublings, *e*.*g*., anywhere from about 3 to about 35 population doublings, in a medium comprising DMEM-LG (*e*.*g*., from Gibco); 2% fetal calf serum (*e*.*g*., from Hyclone Labs.); 1x insulin-transferrin-selenium (ITS); 1x linoleic acid-bovine serum albumin (LA-BSA); 10⁻⁹ M dexamethasone (*e*.*g*., from Sigma); 10⁻⁴ M ascorbic acid 2-phosphate (*e*.*g*., from Sigma); epidermal growth factor 10 ng/mL (*e*.*g*., from R&D Systems); and platelet-derived growth factor (PDGF-BB) 10 ng/mL (*e*.*g*., from R&D Systems). In another specific embodiment, the placental cell-specific gene is CD200.

Specific sequences for these genes can be found in GenBank at accession nos. NM_001615 (ACTG2), BC065545 (ADARB1), (NM_181847 (AMIGO2), AY358590 (ARTS-1), BC074884 (B4GALT6), BC008396 (BCHE), BC020196 (C11orf9), BC031103 (CD200), NM_001845 (COL4A1), NM_001846 (COL4A2), BC052289 (CPA4), BC094758 (DMD), AF293359 (DSC3), NM_001943 (DSG2), AF338241 (ELOVL2), AY336105 (F2RL1), NM_018215 (FLJ10781), AY416799 (GATA6), BC075798 (GPR126), NM_016235 (GPRC5B), AF340038 (ICAM1), BC000844 (IER3), BC066339 (IGFBP7), BC013142 (ILIA), BT019749 (IL6), BC007461 (IL18), (BC072017) KRT18, BC075839 (KRT8), BC060825 (LIPG), BC065240 (LRAP), BC010444 (MATN2), BC011908 (MEST), BC068455 (NFE2L3), NM_014840 (NUAK1), AB006755 (PCDH7), NM_014476 (PDLIM3), BC126199 (PKP-2), BC090862 (RTN1), BC002538 (SERPINB9), BC023312 (ST3GAL6), BC001201 (ST6GALNAC5), BC126160 or BC065328 (SLC12A8), BC025697 (TCF21), BC096235 (TGFB2), BC005046 (VTN), and BC005001 (ZC3H12A) as of March 2008.

In certain specific embodiments, said isolated arPSCs express each of ACTG2, ADARB1, AMIGO2, ARTS-1, B4GALT6, BCHE, C11orf9, CD200, COL4A1, COL4A2, CPA4, DMD, DSC3, DSG2, ELOVL2, F2RL1, FLJ10781, GATA6, GPR126, GPRC5B, ICAM1, IER3, IGFBP7, ILIA, IL6, IL18, KRT18, KRT8, LIPG, LRAP, MATN2, MEST, NFE2L3, NUAK1, PCDH7, PDLIM3, PKP2, RTN1, SERPINB9, ST3GAL6, ST6GALNAC5, SLC12A8, TCF21, TGFB2, VTN, and ZC3H12A at a detectably higher level than an equivalent number of bone marrow-derived mesenchymal stem cells, when the cells are grown under equivalent conditions.

In specific embodiments, the arPSCs express CD200 and ARTS1 (aminopeptidase regulator of type 1 tumor necrosis factor); ARTS-1 and LRAP (leukocyte-derived arginine aminopeptidase); IL6 (interleukin-6) and TGFB2 (transforming growth factor, beta 2); IL6 and KRT18 (keratin 18); IER3 (immediate early response 3), MEST (mesoderm specific transcript homolog) and TGFB2; CD200 and IER3; CD200 and IL6; CD200 and KRT18; CD200 and LRAP; CD200 and MEST; CD200 and NFE2L3 (nuclear factor (erythroid-derived 2)-like 3); or CD200 and TGFB2 at a detectably higher level than an equivalent number of bone marrow-derived mesenchymal stem cells wherein said bone marrow-derived mesenchymal stem cells have undergone a number of passages in culture equivalent to the number of passages said isolated placental stem cells have undergone. In other specific embodiments, the arPSCs express ARTS-1, CD200, IL6 and LRAP; ARTS-1, IL6, TGFB2, IER3, KRT18 and MEST; CD200, IER3, IL6, KRT18, LRAP, MEST, NFE2L3, and TGFB2; ARTS-1, CD200, IER3, IL6, KRT18, LRAP, MEST, NFE2L3, and TGFB2; or IER3, MEST and TGFB2 at a detectably higher level than an equivalent number of bone marrow-derived mesenchymal stem cells, wherein said bone marrow-derived mesenchymal stem cells have undergone a number of passages in culture equivalent to the number of passages said isolated arPSCs have undergone.

Expression of the above-referenced genes can be assessed by standard techniques. For example, probes based on the sequence of the gene(s) can be individually selected and constructed by conventional techniques. Expression of the genes can be assessed, *e*.*g*., on a microarray comprising probes to one or more of the genes, *e*.*g*., an Affymetrix GENECHIP® Human Genome U133A 2.0 array, or an Affymetrix GENECHIP® Human Genome U133 Plus 2.0 (Santa Clara, California). Expression of these genes can be assessed even if the sequence for a particular GenBank accession number is amended because probes specific for the amended sequence can readily be generated using well-known standard techniques.

The level of expression of these genes can be used to confirm the identity of a population of isolated arPSCs, to identify a population of cells as comprising at least a plurality of isolated arPSCs, or the like. Populations of isolated arPSCs, the identity of which is confirmed, can be clonal, *e*.*g*., populations of isolated arPSCs expanded from a single isolated arPSC, or a mixed population of arPSCs, *e*.*g*., a population of cells comprising isolated arPSCs that are expanded from multiple isolated arPSCs, or a population of cells comprising isolated arPSCs, as described herein, and at least one other type of cell.

The level of expression of these genes can be used to select populations of isolated arPSCs. For example, a population of cells, *e*.*g*., clonally-expanded arPSCs, may be selected if the expression of one or more of the genes listed above is significantly higher in a sample from the population of cells than in an equivalent population of bone marrow-derived mesenchymal stem cells. Such selecting can be of a population from a plurality of isolated arPSC populations, from a plurality of cell populations, the identity of which is not known, *etc.*

Isolated arPSCs can be selected on the basis of the level of expression of one or more such genes as compared to the level of expression in said one or more genes in, *e.g.,* a bone marrow-derived mesenchymal stem cell control. In one embodiment, the level of expression of said one or more genes in a sample comprising an equivalent number of bone marrow-derived mesenchymal stem cells is used as a control. In another embodiment, the control, for isolated arPSCs tested under certain conditions, is a numeric value representing the level of expression of said one or more genes in bone marrow-derived mesenchymal stem cells under said conditions.

Similarly, the expression of anoikis associated genes can be used to select populations of isolated arPSCs. For example, a population of cells, *e*.*g*., clonally-expanded arPSCs, may be selected if the expression of one or more anoikis associated genes (e.g., one or more of the anoikis associated genes described herein) is decreased in a sample from the population of cells relative an equivalent population of unmodified placental stem cells.

The isolated arPSCs described herein display the above characteristics (*e*.*g*., combinations of cell surface markers and/or gene expression profiles) in primary culture, or during proliferation in medium comprising, *e*.*g*., DMEM-LG (Gibco), 2% fetal calf serum (FCS) (Hyclone Laboratories), 1x insulin-transferrin-selenium (ITS), 1x linoleic-acid-bovine-serum-albumin (LA-BSA), 10⁻⁹M dexamethasone (Sigma), 10⁻⁴M ascorbic acid 2-phosphate (Sigma), epidermal growth factor (EGF) 10ng/ml (R&D Systems), platelet derived-growth factor (PDGF-BB) 10ng/ml (R&D Systems), and 100U penicillin/1000U streptomycin.

In certain embodiments of any of the arPSCs disclosed herein, the cells are human. In certain embodiments of any of the arPSCs disclosed herein, the cellular marker characteristics or gene expression characteristics are human markers or human genes.

In another specific embodiment of the isolated arPSCs or populations of cells comprising the isolated arPSCs, said cells or population have been expanded, for example, passaged at least, about, or no more than, 1, 2, 3, 4, 5, 6, 7, 8, 9, 10, 11, 12, 13, 14, 15, 16, 17, 18, 19, or 20 times, or proliferated for at least, about, or no more than, 1, 2, 3, 4, 5, 6, 7, 8, 9, 10, 12, 14, 16, 18, 20, 22, 24, 26, 28, 30, 32, 34, 36, 38 or 40 population doublings. In another specific embodiment of said isolated arPSCs or populations of cells comprising the isolated arPSCs, said cells or population are primary isolates. In another specific embodiment of the isolated arPSCs, or populations of cells comprising isolated arPSCs, that are disclosed herein, said isolated arPSCs are fetal in origin (that is, have the fetal genotype).

In certain embodiments, said isolated arPSCs do not differentiate during culturing in growth medium, *i*.*e*., medium formulated to promote proliferation, *e*.*g*., during proliferation in growth medium. In another specific embodiment, said isolated arPSCs do not require a feeder layer in order to proliferate. In another specific embodiment, said isolated arPSCs do not differentiate in culture in the absence of a feeder layer, solely because of the lack of a feeder cell layer.

In another embodiment, the isolated arPSCs are positive for aldehyde dehydrogenase (ALDH), as assessed by an aldehyde dehydrogenase activity assay. Such assays are known in the art (*see*, *e*.*g*., Bostian and Betts, Biochem. J., 173, 787, (1978)). In a specific embodiment, said ALDH assay uses ALDEFLUOR® (Aldagen, Inc., Ashland, Oregon) as a marker of aldehyde dehydrogenase activity. In a specific embodiment, between about 3% and about 25% of arPSCs are positive for ALDH. In another embodiment, said isolated arPSCs show at least three-fold, or at least five-fold, higher ALDH activity than a population of bone marrow-derived mesenchymal stem cells having about the same number of cells and cultured under the same conditions.

In certain embodiments of any of the populations of cells comprising the isolated arPSCs described herein, the arPSCs in said populations of cells are substantially free of cells having a maternal genotype; *e*.*g*., at least 40%, 45%, 50%, 55%, 60%, 65%, 70%, 75%, 80%, 85%, 90%, 95%, 98% or 99% of the arPSCs in said population have a fetal genotype. In certain other embodiments of any of the populations of cells comprising the isolated arPSCs described herein, the populations of cells comprising said arPSCs are substantially free of cells having a maternal genotype; *e*.*g*., at least 40%, 45%, 50%, 55%, 60%, 65%, 70%, 75%, 80%, 85%, 90%, 95%, 98% or 99% of the cells in said population have a fetal genotype.

In a specific embodiment of any of the above isolated arPSCs or cell populations comprising isolated arPSCs, the karyotype of the cells, *e*.*g*., all of the cells, or at least about 95% or about 99% of the cells in said population, is normal. In another specific embodiment of any of the above arPSCs or populations or arPSCs, the arPSCs are non-maternal in origin.

In a specific embodiment of any of the embodiments of placental cells disclosed herein, the placental cells are genetically stable, displaying a normal diploid chromosome count and a normal karyotype.

Isolated arPSCs, or populations of isolated arPSCs, bearing any of the above combinations of markers, can be combined in any ratio. Any two or more of the above isolated arPSCs populations can be combined to form an isolated arPSC population. For example, a population of isolated arPSCs can comprise a first population of isolated arPSCs defined by one of the marker combinations described above, and a second population of isolated arPSCs defined by another of the marker combinations described above, wherein said first and second populations are combined in a ratio of about 1:99, 2:98, 3:97, 4:96, 5:95, 10:90, 20:80, 30:70, 40:60, 50:50, 60:40, 70:30, 80:20, 90:10, 95:5, 96:4, 97:3, 98:2, or about 99:1. In like fashion, any three, four, five or more of the above-described isolated arPSCs or isolated placental stem cell populations can be combined.

Isolated placental stem cells useful in methods for generating the arPSCs described herein can be obtained, *e*.*g*., by disruption of placental tissue, with or without enzymatic digestion or perfusion. For example, populations of isolated placental stem cells can be produced according to a method comprising perfusing a mammalian placenta that has been drained of cord blood and perfused to remove residual blood; perfusing said placenta with a perfusion solution; and collecting said perfusion solution, wherein said perfusion solution after perfusion comprises a population of placental cells that comprises isolated placental stem cells; and isolating said placental stem cells from said population of cells. In a specific embodiment, the perfusion solution is passed through both the umbilical vein and umbilical arteries and collected after it exudes from the placenta. In another specific embodiment, the perfusion solution is passed through the umbilical vein and collected from the umbilical arteries, or passed through the umbilical arteries and collected from the umbilical vein.

In various embodiments, the isolated placental stem cells, useful in methods for generating the arPSCs described herein contained within a population of cells obtained from perfusion of a placenta, are at least 50%, 60%, 70%, 80%, 90%, 95%, 99% or at least 99.5% of said population of placental stem cells. In another specific embodiment, the isolated placental stem cells collected by perfusion comprise fetal and maternal cells. In another specific embodiment, the isolated placental stem cells collected by perfusion are at least 50%, 60%, 70%, 80%, 90%, 95%, 99% or at least 99.5% fetal cells.

In another specific embodiment, provided herein is a composition comprising a population of the isolated placental stem cells useful in methods for generating the arPSCs described herein, collected (isolated) by perfusion, wherein said composition comprises at least a portion of the perfusion solution used to isolate the placental stem cells.

Populations of the isolated placental stem cells useful in methods for generating the arPSCs described herein can be produced by digesting placental tissue with a tissue-disrupting enzyme to obtain a population of placental cells comprising the placental stem cells, and isolating, or substantially isolating, a plurality of the placental stem cells from the remainder of said placental cells. The whole, or any part of, the placenta can be digested to obtain the isolated placental stem cells described herein. In specific embodiments, for example, said placental tissue can be a whole placenta (*e*.*g*., including an umbilical cord), an amniotic membrane, chorion, a combination of amnion and chorion, or a combination of any of the foregoing. In other specific embodiments, the tissue-disrupting enzyme is trypsin or collagenase. In various embodiments, the isolated placental stem cells, contained within a population of cells obtained from digesting a placenta, are at least 50%, 60%, 70%, 80%, 90%, 95%, 99% or at least 99.5% of said population of placental cells.

The populations of isolated arPSCs described above, and populations of isolated arPSCs generally, can comprise about, at least, or no more than, 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, 1 x 10¹¹ or more of the isolated arPSCs. Populations of isolated arPSCs useful in the methods and compositions described herein comprise at least 50%, 55%, 60%, 65%, 70%, 75%, 80%, 85%, 90%, 95%, 98%, or 99% viable isolated placental stem cells, *e*.*g*., as determined by, *e*.*g*., trypan blue exclusion.

For any of the above placental stem cells, or populations of placental stem cells, (e.g., unmodified placental stem cells useful in methods of producing the arPSCs described herein, or the arPSCs described herein, or compositions thereof) the cells or population of placental stem cells are, or can comprise, cells that have been passaged at least 1, 2, 3, 4, 5, 6, 7, 8, 9, 10, 12, 14, 16, 18, or 20 times, or more, or expanded for 1, 2, 3, 4, 5, 6, 7, 8, 9, 10, 12, 14, 16, 18, 20, 22, 24, 26, 28, 30, 32, 34, 36, 38 or 40 population doublings, or more.

In a specific embodiment of any of the above placental stem cells or placental stem cells populations (e.g., unmodified placental stem cells useful in methods of producing the arPSCs described herein, or the arPSCs described herein, or compositions thereof), the karyotype of the cells, or at least about 95% or about 99% of the cells in said population, is normal. In another specific embodiment of any of the above placental stem cells or placental stem cells populations (e.g., unmodified placental stem cells useful in methods of producing the arPSCs described herein, or the arPSCs described herein, or compositions thereof), the cells, or cells in the population of cells, are non-maternal in origin.

Isolated placental stem cells, or populations of isolated placental stem cells, (e.g., unmodified placental stem cells useful in methods of producing the arPSCs described herein, or the arPSCs described herein, or compositions thereof) bearing any of the above combinations of markers, can be combined in any ratio. Any two or more of the above placental stem cells populations can be isolated, or enriched, to form a placental stem cells population. For example, an population of isolated placental stem cells comprising a first population of placental stem cells defined by one of the marker combinations described above can be combined with a second population of placental stem cells defined by another of the marker combinations described above, wherein said first and second populations are combined in a ratio of about 1:99, 2:98, 3:97, 4:96, 5:95, 10:90, 20:80, 30:70, 40:60, 50:50, 60:40, 70:30, 80:20, 90:10, 95:5, 96:4, 97:3, 98:2, or about 99:1. In like fashion, any three, four, five or more of the above-described placental stem cells or placental stem cells populations can be combined.

In a specific embodiment of the above-mentioned placental stem cells (e.g., unmodified placental stem cells useful in methods of producing the arPSCs described herein, or the arPSCs described herein, or compositions thereof), the placental stem cells constitutively secrete IL-6, IL-8 and monocyte chemoattractant protein (MCP-1).

The immunosuppressive pluralities of arPSCs described above can comprise about, at least, or no more than, 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, 1 x 10¹¹ or more arPSCs.

In certain embodiments, the arPSCs useful in the methods provided herein, do not express CD34, as detected by immunolocalization, after exposure to 1 to 100 ng/mL VEGF for 4 to 21 days. In another specific embodiment, said arPSCs induce endothelial cells to form sprouts or tube-like structures, *e*.*g*., when cultured in the presence of an angiogenic factor such as vascular endothelial growth factor (VEGF), epithelial growth factor (EGF), platelet derived growth factor (PDGF) or basic fibroblast growth factor (bFGF), *e*.*g*., on a substrate such as MATRIGEL™.

In another aspect, the arPSCs provided herein, or a population of cells, *e*.*g*., a population of arPSCs, or a population of cells wherein at least about 50%, 60%, 70%, 80%, 90%, 95% or 98% of cells in said population of cells are arPSCs, secrete one or more, or all, of VEGF, HGF, IL-8, MCP-3, FGF2, follistatin, G-CSF, EGF, ENA-78, GRO, IL-6, MCP-1, PDGF-BB, TIMP-2, uPAR, or galectin-1, *e*.*g*., into culture medium in which the cell, or cells, are grown. In another embodiment, the arPSCs express increased levels of CD202b, IL-8 and/or VEGF under hypoxic conditions (*e*.*g*., less than about 5% O₂) compared to normoxic conditions (*e*.*g*., about 20% or about 21%> O₂).

In another embodiment, any of the arPSCs or populations of cells comprising arPSCs described herein can cause the formation of sprouts or tube-like structures in a population of endothelial cells in contact with said arPSCs. In a specific embodiment, the arPSCs are co-cultured with human endothelial cells, which form sprouts or tube-like structures, or support the formation of endothelial cell sprouts, *e*.*g*., when cultured in the presence of extracellular matrix proteins such as collagen type I and IV, and/or angiogenic factors such as vascular endothelial growth factor (VEGF), epithelial growth factor (EGF), platelet derived growth factor (PDGF) or basic fibroblast growth factor (bFGF), *e*.*g*., in or on a substrate such as placental collagen or MATRIGEL™ for at least 4 days. In another embodiment, any of the populations of cells comprising arPSCs described herein secrete angiogenic factors such as vascular endothelial growth factor (VEGF), hepatocyte growth factor (HGF), platelet derived growth factor (PDGF), basic fibroblast growth factor (bFGF), or Interleukin-8 (IL-8) and thereby can induce human endothelial cells to form sprouts or tube-like structures when cultured in the presence of extracellular matrix proteins such as collagen type I and IV *e*.*g*., in or on a substrate such as placental collagen or MATRIGEL™.

In another embodiment, any of the above populations of cells comprising arPSCs secretes angiogenic factors. In specific embodiments, the population of cells secretes vascular endothelial growth factor (VEGF), hepatocyte growth factor (HGF), platelet derived growth factor (PDGF), basic fibroblast growth factor (bFGF), and/or interleukin-8 (IL-8). In other specific embodiments, the population of cells comprising arPSCs secretes one or more angiogenic factors and thereby induces human endothelial cells to migrate in an *in vitro* wound healing assay. In other specific embodiments, the population of cells comprising arPSCs induces maturation, differentiation or proliferation of human endothelial cells, endothelial progenitors, myocytes or myoblasts.

In another embodiment, provided herein are arPSCs, and populations of arPSCs, wherein said arPSCs comprise any of the foregoing characteristics (e.g., are CD34⁻, CD10⁺, CD105⁺ and CD200⁺), and wherein at least one anoikis associated gene is downregulated/inhibited in said arPSCs relative to the level of expression of said anoikis associated gene in an equivalent number of unmodified placental stem cells (e.g., CD34⁻, CD10⁺, CD105⁺ and CD200⁺ unmodified placental stem cells). In a specific embodiment, the at least one anoikis associated gene is AMIGO1 (NCBI GENE ID NO:57463); ARHGAP20 (NCBI GENE ID NO:57569); CD38 (NCBI GENE ID NO:952); CLCC1 (NCBI GENE ID NO:23155); CNTF (NCBI GENE ID NO:1270); ZFP91-CNTF (NCBI GENE ID NO:386607); COX8A (NCBI GENE ID NO:1351); DHX34 (NCBI GENE ID NO:9704); FAM175A (NCBI GENE ID NO:NO 51023); MRPS18C (NCBI GENE ID NO:84142); FAM44C (NCBI GENE ID NO:284257); FBP2 (NCBI GENE ID NO:8789); FLI1 (NCBI GENE ID NO:2313); FREM3 (NCBI GENE ID NO:166752); IFIT5 (NCBI GENE ID NO:24138); LOC399851 (NCBI GENE ID NO:399851); LOC400713 (NCBI GENE ID NO:400713); LOC651610 (NCBI GENE ID NO:651610); PIGP (NCBI GENE ID NO:51227); SH3TC2 (NCBI GENE ID NO:79628); SLC2A3 (NCBI GENE ID NO:6515); STAU2 (NCBI GENE ID NO:27067) TMEFF1 (NCBI GENE ID NO:8577); TMEM217 (NCBI GENE ID NO:221468); TMEM79 (NCBI GENE ID NO:84283); USHBP1 (NCBI GENE ID NO:83878); APH1B (NCBI GENE ID NO:83464); ATP2B2 (NCBI GENE ID NO:491); C13orf39 (NCBI GENE ID NO:196541); C4orf17 (NCBI GENE ID NO:84103); C4orf46 (NCBI GENE ID NO:201725); DDX41 (NCBI GENE ID NO:51428); DKFZp547J222 (NCBI GENE ID NO:84237); FGFR1 (NCBI GENE ID NO:2260); FHDC1 (NCBI GENE ID NO:85462); GNAI2 (NCBI GENE ID NO:2771); GP5 (NCBI GENE ID NO:2814); IL1RN (NCBI GENE ID NO:3557); KIF24 (NCBI GENE ID NO:347240); KNDC1 (NCBI GENE ID NO:85442); LOC100132598 (NCBI GENE ID NO:100132598); LOC151760 (NCBI GENE ID NO:151760); LOC152024 (NCBI GENE ID NO:152024); LOC339833 (NCBI GENE ID NO:339833); LPAR4 (NCBI GENE ID NO:2846); LSG1 (NCBI GENE ID NO:55341); MAP3K5 (NCBI GENE ID NO:4217); PDK3 (NCBI GENE ID NO:5165); PELI2 (NCBI GENE ID NO:57161); RNF103 (NCBI GENE ID NO:7844); SNX31 (NCBI GENE ID NO:169166); TXN2 (NCBI GENE ID NO:25828); or XKR7 (NCBI GENE ID NO:343702). In a specific embodiment, 2, 3, 4, 5, 6, 7, 8, 9, 10, or more than 10 of said anoikis associated genes are downregulated/inhibited in said arPSCs relative to the level of expression of said anoikis associated gene(s) in an equivalent number of unmodified placental stem cells (e.g., CD34⁻, CD10⁺, CD105⁺ and CD200⁺ unmodified placental stem cells).

In another specific embodiment, provided herein is an isolated CD34⁻, CD10⁺, CD105⁺ and CD200⁺ arPSC, wherein said arPSC expresses the anoikis associated gene FHDC1 (NCBI GENE ID NO:85462) at a decreased level as compared to the expression of the anoikis associated gene FHDC1 (NCBI GENE ID NO:85462) in an unmodified placental stem cell. In another specific embodiment, provided herein is an isolated CD34⁻, CD10⁺, CD105⁺ and CD200⁺ arPSC, wherein said arPSC expresses the anoikis associated gene GNAI2 (NCBI GENE ID NO:2771) at a decreased level as compared to the expression of the anoikis associated gene GNAI2 (NCBI GENE ID NO:2771) in an unmodified placental stem cell. In another specific embodiment, provided herein is an isolated CD34⁻, CD10⁺, CD105⁺ and CD200⁺ arPSC, wherein said arPSC expresses the anoikis associated gene KNDC1 (NCBI GENE ID NO:85442) at a decreased level as compared to the expression of the anoikis associated gene KNDC1 (NCBI GENE ID NO:85442) in an unmodified placental stem cell. In another specific embodiment, provided herein is an isolated CD34⁻, CD10⁺, CD105⁺ and CD200⁺ arPSC, wherein said arPSC expresses the anoikis associated gene LPAR4 (NCBI GENE ID NO:2846) at a decreased level as compared to the expression of the anoikis associated gene LPAR4 (NCBI GENE ID NO:2846) in an unmodified placental stem cell. In another specific embodiment, provided herein is an isolated CD34⁻, CD10⁺, CD105⁺ and CD200⁺ arPSC, wherein said arPSC expresses the anoikis associated gene MAP3K5 (NCBI GENE ID NO:4217) at a decreased level as compared to the expression of the anoikis associated gene MAP3K5 (NCBI GENE ID NO:4217) in an unmodified placental stem cell. In another specific embodiment, provided herein is an isolated CD34⁻, CD10⁺, CD105⁺ and CD200⁺ arPSC, wherein said arPSC expresses the anoikis associated gene SLC2A3 (NCBI GENE ID NO:6515) at a decreased level as compared to the expression of the anoikis associated gene SLC2A3 (NCBI GENE ID NO:6515) in an unmodified placental stem cell. In another specific embodiment, provided herein is an isolated CD34⁻, CD10⁺, CD105⁺ and CD200⁺ arPSC, wherein said arPSC expresses the anoikis associated gene STAU2 (NCBI GENE ID NO:27067) at a decreased level as compared to the expression of the anoikis associated gene STAU2 (NCBI GENE ID NO:27067) in an unmodified placental stem cell. Further provided herein are populations of cells comprising such arPSCs and compositions comprising such arPSCs.

In another specific embodiment, provided herein is an isolated CD34⁻, CD10⁺, CD105⁺ and CD200⁺ arPSC, wherein said arPSC expresses one, two, three, or more of the following placental stem cell anoikis-associated genes at a decreased level as compared to the expression of the same anoikis associated gene(s) in an unmodified placental stem cell: FHDC1 (NCBI GENE ID NO:85462), GNAI2 (NCBI GENE ID NO:2771), KNDC1 (NCBI GENE ID NO:85442), LPAR4 (NCBI GENE ID NO:2846), MAP3K5 (NCBI GENE ID NO:4217), SLC2A3 (NCBI GENE ID NO:6515), and STAU2 (NCBI GENE ID NO:27067). In another specific embodiment, provided herein is an isolated CD34⁻, CD10⁺, CD105⁺ and CD200⁺ arPSC, wherein said arPSC (i) expresses one, two, three, or more of the following placental stem cell anoikis-associated genes at a decreased level as compared to the expression of the same anoikis associated gene(s) in an unmodified placental stem cell: FHDC1 (NCBI GENE ID NO:85462), GNAI2 (NCBI GENE ID NO:2771), KNDC1 (NCBI GENE ID NO:85442), LPAR4 (NCBI GENE ID NO:2846), MAP3K5 (NCBI GENE ID NO:4217), SLC2A3 (NCBI GENE ID NO:6515), and STAU2 (NCBI GENE ID NO:27067); and (ii) expresses at least one additional anoikis associated gene recited in Table 1 at a decreased level as compared to the expression of the same anoikis associated gene(s) in an unmodified placental stem cell. Further provided herein are populations of cells comprising such arPSCs and compositions comprising such arPSCs.

### 5.3.3 Growth in Culture

The growth of the placental cells, including the arPSCs described herein, as for any mammalian cell, depends in part upon the particular medium selected for growth. During culture, the placental stem cells used in the methods of production of the arPSCs provided herein adhere to a substrate in culture, *e.g.* the surface of a tissue culture container (*e*.*g*., tissue culture dish plastic, fibronectin-coated plastic, and the like) and form a monolayer. In the absence of a substrate for the placental stem cells to adhere to (e.g., under low-attachment conditions), the placental stem cells undergo anoikis, and demonstrate diminished survival. In contrast, the arPSCs described herein do not undergo anoikis in the absence of a substrate for the arPSCs to adhere to (e.g., under low-attachment conditions), and thus demonstrate increased survival in such conditions relative to unmodified placental stem cells.

In a specific embodiment, the arPSCs described herein demonstrate increased survival relative to unmodified placental stem cells when cultured under low attachment conditions in vitro, e.g., when cultured in low-attachment tissue culture plates. In another specific embodiment, the arPSCs described herein demonstrate increased survival relative to unmodified placental stem cells when cultured under low attachment conditions in vivo, e.g., when administered to a subject systemically or locally, or by another administration method wherein the cells are administered in a low attachment environment.

In certain embodiments, when cultured under low-attachment conditions (either in vitro or in vivo), the arPSCs described herein demonstrate at least a 1.5-fold, 2-fold, 2.5-fold, 3-fold, 3.5-fold, 4-fold, 4.5-fold, 5-fold, 6-fold, 7-fold, 8-fold, 9-fold, or 10-fold increase in survival relative to an equivalent amount of unmodified placental stem cells cultured under the same conditions. In certain embodiments, when cultured under low-attachment conditions (either in vitro or in vivo), the arPSCs described herein demonstrate a 1.5-fold to 2.5-fold, a 2-fold to 3-fold, a 2.5-fold to 3.5-fold, a 3-fold to 4-fold, a 3.5-fold to 4.5-fold, a 4-fold to 5-fold, a 5-fold to 6-fold, a 6-fold to 7-fold, a 7-fold to 8-fold, an 8-fold to 9-fold, or a 9-fold to 10-fold increase in survival relative to an equivalent amount of unmodified placental stem cells cultured under the same conditions. In another specific embodiment, when cultured under low-attachment conditions (either in vitro or in vivo), the arPSCs described herein demonstrate a greater than 10-fold increase in survival relative to an equivalent amount of unmodified placental stem cells cultured under the same conditions. Survival of the arPSCs and unmodified placental stem cells can be assessed using methods known in the art, e.g., trypan blue exclusion assay, fluorescein diacetate uptake assay, propidium iodide uptake assay; thymidine uptake assay, and MTT (3-(4,5-Dimethylthiazol-2-yl)-2,5-diphenyltetrazolium bromide) assay.

### 5.4 METHODS OF OBTAINING PLACENTAL STEM CELLS FOR USE IN METHODS OF GENERATING ANOIKIS-RESISTANT PLACENTAL STEM CELLS

### 5.4.1 Stem Cell Collection Composition

Placental stem cells for use in the methods of generating arPSCs described herein can be collected and isolated according to the methods provided herein. Generally, placental stem cells are obtained from a mammalian placenta using a physiologically-acceptable solution, *e*.*g*., a stem cell collection composition. A stem cell collection composition is described in detail in related U.S. Patent Application Publication No. 20070190042.

The stem cell collection composition can comprise any physiologically-acceptable solution suitable for the collection and/or culture of stem cells, for example, a saline solution (*e*.*g*., phosphate-buffered saline, Kreb's solution, modified Kreb's solution, Eagle's solution, 0.9% NaCl. *etc*.), a culture medium (*e*.*g*., DMEM, HDMEM, *etc*.), and the like.

The stem cell collection composition can comprise one or more components that tend to preserve placental stem cells, that is, prevent the placental stem cells from dying, or delay the death of the placental stem cells, reduce the number of placental stem cells in a population of cells that die, or the like, from the time of collection to the time of culturing. Such components can be, *e*.*g*., an apoptosis inhibitor (*e*.*g*., a caspase inhibitor or JNK inhibitor); a vasodilator (*e*.*g*., magnesium sulfate, an antihypertensive drug, atrial natriuretic peptide (ANP), adrenocorticotropin, corticotropin-releasing hormone, sodium nitroprusside, hydralazine, adenosine triphosphate, adenosine, indomethacin or magnesium sulfate, a phosphodiesterase inhibitor, *etc*.); a necrosis inhibitor (*e*.*g*., 2-(1H-Indol-3-yl)-3-pentylamino-maleimide, pyrrolidine dithiocarbamate, or clonazepam); a TNF-α inhibitor; and/or an oxygen-carrying perfluorocarbon (*e*.*g*., perfluorooctyl bromide, perfluorodecyl bromide, *etc*.).

The stem cell collection composition can comprise one or more tissue-degrading enzymes, *e*.*g*., a metalloprotease, a serine protease, a neutral protease, an RNase, or a DNase, or the like. Such enzymes include, but are not limited to, collagenases (*e*.*g*., collagenase I, II, III or IV, a collagenase from *Clostridium histolyticum*, *etc.*); dispase, thermolysin, elastase, trypsin, LIBERASE, hyaluronidase, and the like.

The stem cell collection composition can comprise a bacteriocidally or bacteriostatically effective amount of an antibiotic. In certain non-limiting embodiments, the antibiotic is a macrolide (*e*.*g*., tobramycin), a cephalosporin (*e*.*g*., cephalexin, cephradine, cefuroxime, cefprozil, cefaclor, cefixime or cefadroxil), a clarithromycin, an erythromycin, a penicillin (*e*.*g*., penicillin V) or a quinolone (*e*.*g*., ofloxacin, ciprofloxacin or norfloxacin), a tetracycline, a streptomycin, *etc.* In a particular embodiment, the antibiotic is active against Gram(+) and/or Gram(-) bacteria, *e*.*g*., *Pseudomonas aeruginosa*, *Staphylococcus aureus*, and the like.

The stem cell collection composition can also comprise one or more of the following compounds: adenosine (about 1 mM to about 50 mM); D-glucose (about 20 mM to about 100 mM); magnesium ions (about 1 mM to about 50 mM); a macromolecule of molecular weight greater than 20,000 daltons, in one embodiment, present in an amount sufficient to maintain endothelial integrity and cellular viability (*e*.*g*., a synthetic or naturally occurring colloid, a polysaccharide such as dextran or a polyethylene glycol present at about 25 g/l to about 100 g/l, or about 40 g/l to about 60 g/l); an antioxidant (*e*.*g*., butylated hydroxyanisole, butylated hydroxytoluene, glutathione, vitamin C or vitamin E present at about 25 µM to about 100 µM); a reducing agent (*e*.*g*., N-acetylcysteine present at about 0.1 mM to about 5 mM); an agent that prevents calcium entry into cells (*e*.*g*., verapamil present at about 2 µM to about 25 µM); nitroglycerin (*e*.*g*., about 0.05 g/L to about 0.2 g/L); an anticoagulant, in one embodiment, present in an amount sufficient to help prevent clotting of residual blood (*e*.*g*., heparin or hirudin present at a concentration of about 1000 units/l to about 100,000 units/l); or an amiloride containing compound (*e*.*g*., amiloride, ethyl isopropyl amiloride, hexamethylene amiloride, dimethyl amiloride or isobutyl amiloride present at about 1.0 µM to about 5 µM).

### 5.4.2 Collection and Handling of Placenta

Generally, a human placenta is recovered shortly after its expulsion after birth. In a preferred embodiment, the placenta is recovered from a patient after informed consent and after a complete medical history of the patient is taken and is associated with the placenta. Preferably, the medical history continues after delivery. Such a medical history can be used to coordinate subsequent use of the placenta or the stem cells harvested therefrom. For example, human placental cells can be used, in light of the medical history, for personalized medicine for the infant associated with the placenta, or for parents, siblings or other relatives of the infant.

Prior to recovery of placental stem cells, the umbilical cord blood and placental blood are removed. In certain embodiments, after delivery, the cord blood in the placenta is recovered. The placenta can be subjected to a conventional cord blood recovery process. Typically a needle or cannula is used, with the aid of gravity, to exsanguinate the placenta (*see*, *e*.*g*., Anderson, U.S. Patent No. 5,372,581; Hessel et al., U.S. Patent No. 5,415,665). The needle or cannula is usually placed in the umbilical vein and the placenta can be gently massaged to aid in draining cord blood from the placenta. Such cord blood recovery may be performed commercially, *e*.*g*., LifeBank Inc., Cedar Knolls, N.J., ViaCord, Cord Blood Registry and Cryocell. Preferably, the placenta is gravity drained without further manipulation so as to minimize tissue disruption during cord blood recovery.

Typically, a placenta is transported from the delivery or birthing room to another location, *e*.*g*., a laboratory, for recovery of cord blood and collection of stem cells by, *e*.*g*., perfusion or tissue dissociation. The placenta is preferably transported in a sterile, thermally insulated transport device (maintaining the temperature of the placenta between 20-28°C), for example, by placing the placenta, with clamped proximal umbilical cord, in a sterile zip-lock plastic bag, which is then placed in an insulated container. In another embodiment, the placenta is transported in a cord blood collection kit substantially as described in pending United States patent application no. 11/230,760, filed September 19, 2005. Preferably, the placenta is delivered to the laboratory four to twenty-four hours following delivery. In certain embodiments, the proximal umbilical cord is clamped, preferably within 4-5 cm (centimeter) of the insertion into the placental disc prior to cord blood recovery. In other embodiments, the proximal umbilical cord is clamped after cord blood recovery but prior to further processing of the placenta.

The placenta, prior to placental stem cell collection, can be stored under sterile conditions and at either room temperature or at a temperature of 5 to 25°C (centigrade). The placenta may be stored for a period of longer than forty eight hours, and preferably for a period of four to twenty-four hours prior to perfusing the placenta to remove any residual cord blood. The placenta is preferably stored in an anticoagulant solution at a temperature of 5 to 25°C (centigrade). Suitable anticoagulant solutions are well known in the art. For example, a solution of heparin or warfarin sodium can be used. In a preferred embodiment, the anticoagulant solution comprises a solution of heparin (*e*.*g*., 1% w/w in 1:1000 solution). The exsanguinated placenta is preferably stored for no more than 36 hours before placental cells are collected.

The mammalian placenta or a part thereof, once collected and prepared generally as above, can be treated in any art-known manner, *e*.*g*., can be perfused or disrupted, *e*.*g*., digested with one or more tissue-disrupting enzymes, to obtain stem cells.

### 5.4.3 Physical Disruption and Enzymatic Digestion of Placental Tissue

In one embodiment, placental stem cells are collected from a mammalian placenta by physical disruption, *e*.*g*., enzymatic digestion, of the organ, *e*.*g*., using the stem cell collection composition described above. For example, the placenta, or a portion thereof, may be, *e*.*g*., crushed, sheared, minced, diced, chopped, macerated or the like, while in contact with, *e*.*g*., a buffer, medium or a stem cell collection composition, and the tissue subsequently digested with one or more enzymes. The placenta, or a portion thereof, may also be physically disrupted and digested with one or more enzymes, and the resulting material then immersed in, or mixed into, a buffer, medium or a stem cell collection composition. Any method of physical disruption can be used, provided that the method of disruption leaves a plurality, more preferably a majority, and more preferably at least 60%, 70%, 80%, 90%, 95%, 98%, or 99% of the cells in said organ viable, as determined by, *e*.*g*., trypan blue exclusion.

Typically, placental cells can be obtained by disruption of a small block of placental tissue, *e*.*g*., a block of placental tissue that is about 1, 2, 3, 4, 5, 6, 7, 8, 9, 10, 20, 30, 40, 50, 60, 70, 80, 90, 100, 200, 300, 400, 500, 600, 700, 800, 900 or about 1000 cubic millimeters in volume.

Enzymatic digestion can be performed using single enzymes or combinations of enzymes. In one embodiment, enzymatic digestion of placental tissue uses a combination of a matrix metalloprotease, a neutral protease, and a mucolytic enzyme for digestion of hyaluronic acid, such as a combination of collagenase, dispase, and hyaluronidase or a combination of LIBERASE (Boehringer Mannheim Corp., Indianapolis, Ind.) and hyaluronidase. Other enzymes that can be used to disrupt placenta tissue include papain, deoxyribonucleases, serine proteases, such as trypsin, chymotrypsin, or elastase. Serine proteases may be inhibited by alpha 2 microglobulin in serum and therefore the medium used for digestion is usually serum-free. EDTA and DNase are commonly used in enzyme digestion procedures to increase the efficiency of cell recovery. The digestate is preferably diluted so as to avoid trapping stem cells within the viscous digest.

Typical concentrations for tissue digestion enzymes include, *e*.*g*., 50-200 U/mL for collagenase I and collagenase IV, 1-10 U/mL for dispase, and 10-100 U/mL for elastase. Proteases can be used in combination, that is, two or more proteases in the same digestion reaction, or can be used sequentially in order to liberate placental cells. For example, in one embodiment, a placenta, or part thereof, is digested first with an appropriate amount of collagenase I at 2 mg/ml for 30 minutes, followed by digestion with trypsin, 0.25%, for 10 minutes, at 37°C. Serine proteases are preferably used consecutively following use of other enzymes.

In another embodiment, the tissue can further be disrupted by the addition of a chelator, *e*.*g*., ethylene glycol bis(2-aminoethyl ether)-N,N,N'N'-tetraacetic acid (EGTA) or ethylenediaminetetraacetic acid (EDTA) to the stem cell collection composition comprising the stem cells, or to a solution in which the tissue is disrupted and/or digested prior to isolation of the placental stem cells with the stem cell collection composition.

It will be appreciated that where an entire placenta, or portion of a placenta comprising both fetal and maternal cells (for example, where the portion of the placenta comprises the chorion or cotyledons) is digested to obtain placental stem cells, the placental cells collected will comprise a mix of placental cells derived from both fetal and maternal sources. Where a portion of the placenta that comprises no, or a negligible number of, maternal cells (for example, amnion) is used to obtain placental stem cells, the placental stem cells collected will comprise almost exclusively fetal placental stem cells.

### 5.4.4 Placental Perfusion

Placental stem cells can also be obtained by perfusion of the mammalian placenta. Methods of perfusing mammalian placenta to obtain stem cells are disclosed, *e*.*g*., in Hariri, U.S. Application Publication No. 2002/0123141, and in related U.S. Provisional Application No. 60/754,969, entitled "Improved Composition for Collecting and Preserving Placental Cells and Methods of Using the Composition" filed on December 29, 2005.

Placental stem cells can be collected by perfusion, *e*.*g*., through the placental vasculature, using, *e*.*g*., a stem cell collection composition as a perfusion solution. In one embodiment, a mammalian placenta is perfused by passage of perfusion solution through either or both of the umbilical artery and umbilical vein. The flow of perfusion solution through the placenta may be accomplished using, *e*.*g*., gravity flow into the placenta. Preferably, the perfusion solution is forced through the placenta using a pump, *e*.*g*., a peristaltic pump. The umbilical vein can be, *e*.*g*., cannulated with a cannula, *e*.*g*., a TEFLON® or plastic cannula, that is connected to a sterile connection apparatus, such as sterile tubing. The sterile connection apparatus is connected to a perfusion manifold.

In preparation for perfusion, the placenta is preferably oriented (*e*.*g*., suspended) in such a manner that the umbilical artery and umbilical vein are located at the highest point of the placenta. The placenta can be perfused by passage of a perfusion fluid, *e*.*g*., the stem cell collection composition provided herein, through the placental vasculature, or through the placental vasculature and surrounding tissue. In one embodiment, the umbilical artery and the umbilical vein are connected simultaneously to a pipette that is connected via a flexible connector to a reservoir of the perfusion solution. The perfusion solution is passed into the umbilical vein and artery. The perfusion solution exudes from and/or passes through the walls of the blood vessels into the surrounding tissues of the placenta, and is collected in a suitable open vessel from the surface of the placenta that was attached to the uterus of the mother during gestation. The perfusion solution may also be introduced through the umbilical cord opening and allowed to flow or percolate out of openings in the wall of the placenta which interfaced with the maternal uterine wall. In another embodiment, the perfusion solution is passed through the umbilical veins and collected from the umbilical artery, or is passed through the umbilical artery and collected from the umbilical veins.

In one embodiment, the proximal umbilical cord is clamped during perfusion, and more preferably, is clamped within 4-5 cm (centimeter) of the cord's insertion into the placental disc.

The first collection of perfusion fluid from a mammalian placenta during the exsanguination process is generally colored with residual red blood cells of the cord blood and/or placental blood; this portion of the perfusion can be discarded. The perfusion fluid becomes more colorless as perfusion proceeds and the residual cord blood cells are washed out of the placenta.

The volume of perfusion liquid used to collect placental stem cells may vary depending upon the number of placental stem cells to be collected, the size of the placenta, the number of collections to be made from a single placenta, *etc.* In various embodiments, the volume of perfusion liquid may be from 50 mL to 5000 mL, 50 mL to 4000 mL, 50 mL to 3000 mL, 100 mL to 2000 mL, 250 mL to 2000 mL, 500 mL to 2000 mL, or 750 mL to 2000 mL. Typically, the placenta is perfused with 700-800 mL of perfusion liquid following exsanguination.

The placenta can be perfused a plurality of times over the course of several hours or several days. Where the placenta is to be perfused a plurality of times, it may be maintained or cultured under aseptic conditions in a container or other suitable vessel, and perfused with the stem cell collection composition, or a standard perfusion solution (*e*.*g*., a normal saline solution such as phosphate buffered saline ("PBS")) with or without an anticoagulant (*e*.*g*., heparin, warfarin sodium, coumarin, bishydroxycoumarin), and/or with or without an antimicrobial agent (*e*.*g*., β-mercaptoethanol (0.1 mM); antibiotics such as streptomycin (*e*.*g*., at 40-100 µg/ml), penicillin (*e*.*g*., at 40U/ml), amphotericin B (*e*.*g*., at 0.5 µg/ml). In one embodiment, an isolated placenta is maintained or cultured for a period of time without collecting the perfusate, such that the placenta is maintained or cultured for 1, 2, 3, 4, 5, 6, 7, 8, 9, 10, 11, 12, 13, 14, 15, 16, 17, 18, 19, 20, 21, 22, 23, or 24 hours, or 2 or 3 or more days before perfusion and collection of perfusate. The perfused placenta can be maintained for one or more additional time(s), *e*.*g*., 1, 2, 3, 4, 5, 6, 7, 8, 9, 10, 11, 12, 13, 14, 15, 16, 17, 18, 19, 20, 21, 22, 23, 24 or more hours, and perfused a second time with, *e*.*g*., 700-800 mL perfusion fluid. The placenta can be perfused 1, 2, 3, 4, 5 or more times, for example, once every 1, 2, 3, 4, 5 or 6 hours. In a preferred embodiment, perfusion of the placenta and collection of perfusion solution, *e*.*g*., stem cell collection composition, is repeated until the number of recovered nucleated cells falls below 100 cells/ml. The perfusates at different time points can be further processed individually to recover time-dependent populations of placental stem cells. Perfusates from different time points can also be pooled.

Without wishing to be bound by any theory, after exsanguination and a sufficient time of perfusion of the placenta, placental stem cells are believed to migrate into the exsanguinated and perfused microcirculation of the placenta where they are collectable, preferably by washing into a collecting vessel by perfusion. Perfusing the isolated placenta not only serves to remove residual cord blood but also provide the placenta with the appropriate nutrients, including oxygen. The placenta may be cultivated and perfused with a similar solution which was used to remove the residual cord blood cells, preferably, without the addition of anticoagulant agents.

Stem cells can be isolated from placenta by perfusion with a solution comprising one or more proteases or other tissue-disruptive enzymes. In a specific embodiment, a placenta or portion thereof is brought to 25-37°C, and is incubated with one or more tissue-disruptive enzymes in 200 mL of a culture medium for 30 minutes. Cells from the perfusate are collected, brought to 4°C, and washed with a cold inhibitor mix comprising 5 mM EDTA, 2 mM dithiothreitol and 2 mM beta-mercaptoethanol. The placental stem cells are washed after several minutes with a cold (*e*.*g*., 4°C) stem cell collection composition described elsewhere herein.

Perfusion using the pan method, that is, whereby perfusate is collected after it has exuded from the maternal side of the placenta, results in a mix of fetal and maternal cells. As a result, the cells collected by this method comprise a mixed population of placental stem cells of both fetal and maternal origin. In contrast, perfusion solely through the placental vasculature, whereby perfusion fluid is passed through one or two placental vessels and is collected solely through the remaining vessel(s), results in the collection of a population of placental stem cells almost exclusively of fetal origin.

### 5.4.5 Isolation, Sorting, and Characterization of Placental cells

Stem cells from mammalian placenta, whether obtained by perfusion or enyzmatic digestion, can initially be purified from (*i*.*e*., be isolated from) other cells by Ficoll gradient centrifugation. Such centrifugation can follow any standard protocol for centrifugation speed, *etc.* In one embodiment, for example, cells collected from the placenta are recovered from perfusate by centrifugation at 5000 x g for 15 minutes at room temperature, which separates cells from, *e*.*g*., contaminating debris and platelets. In another embodiment, placental perfusate is concentrated to about 200 ml, gently layered over Ficoll, and centrifuged at about 1100 x g for 20 minutes at 22°C, and the low-density interface layer of cells is collected for further processing.

Cell pellets can be resuspended in fresh stem cell collection composition, or a medium suitable for stem cell maintenance, *e*.*g*., IMDM serum-free medium containing 2U/ml heparin and 2mM EDTA (GibcoBRL, NY). The total mononuclear cell fraction can be isolated, *e*.*g*., using Lymphoprep (Nycomed Pharma, Oslo, Norway) according to the manufacturer's recommended procedure.

As used herein, "isolating" placental stem cells means removing at least 20%, 30%, 40%, 50%, 60%, 70%, 80%, 90%, 95% or 99% of the cells with which the placental stem cells are normally associated in the intact mammalian placenta.

Placental stem cells obtained by perfusion or digestion can, for example, be further, or initially, isolated by differential trypsinization using, *e*.*g*., a solution of 0.05% trypsin with 0.2% EDTA (Sigma, St. Louis MO). Differential trypsinization is possible because placental stem cells typically detach from plastic surfaces within about five minutes whereas other adherent populations typically require more than 20-30 minutes incubation. The detached placental stem cells can be harvested following trypsinization and trypsin neutralization, using, *e*.*g*., Trypsin Neutralizing Solution (TNS, Cambrex).

In one embodiment of isolation of placental stem cells, aliquots of, for example, about 5-10 x 10⁶ placental cells are placed in each of several T-75 flasks, preferably fibronectin-coated T75 flasks. In such an embodiment, the cells can be cultured with commercially available Mesenchymal Stem Cell Growth Medium (MSCGM) (Cambrex), and placed in a tissue culture incubator (37°C, 5% CO₂). After 10 to 15 days, non-adherent cells are removed from the flasks by washing with PBS. The PBS is then replaced by MSCGM. Flasks are preferably examined daily for the presence of various adherent cell types and in particular, for identification and expansion of clusters of fibroblastoid cells.

The number and type of cells collected from a mammalian placenta can be monitored, for example, by measuring changes in morphology and cell surface markers using standard cell detection techniques such as flow cytometry, cell sorting, immunocytochemistry (*e*.*g*., staining with tissue specific or cell-marker specific antibodies) fluorescence activated cell sorting (FACS), magnetic activated cell sorting (MACS), by examination of the morphology of cells using light or confocal microscopy, and/or by measuring changes in gene expression using techniques well known in the art, such as PCR and gene expression profiling. These techniques can be used, too, to identify cells that are positive for one or more particular markers. For example, using antibodies to CD34, one can determine, using the techniques above, whether a cell comprises a detectable amount of CD34 as compared to, for example, an isotype control; if so, the cell is CD34⁺. Likewise, if a cell produces enough OCT-4 RNA to be detectable by RT-PCR, or significantly more OCT-4 RNA than a terminally-differentiated cell, the cell is OCT-4⁺. Antibodies to cell surface markers (*e*.*g*., CD markers such as CD34) and the sequence of stem cell-specific genes, such as OCT-4, are well-known in the art.

Placental cells, particularly cells that have been isolated by Ficoll separation, differential adherence, or a combination of both, may be sorted, *e*.*g*., further isolated, using a fluorescence activated cell sorter (FACS). Fluorescence activated cell sorting (FACS) is a well-known method for separating particles, including cells, based on the fluorescent properties of the particles (Kamarch, 1987, Methods Enzymol, 151:150-165). Laser excitation of fluorescent moieties in the individual particles results in a small electrical charge allowing electromagnetic separation of positive and negative particles from a mixture. In one embodiment, cell surface marker-specific antibodies or ligands are labeled with distinct fluorescent labels. Cells are processed through the cell sorter, allowing separation of cells based on their ability to bind to the antibodies used. FACS sorted particles may be directly deposited into individual wells of 96-well or 384-well plates to facilitate separation and cloning.

In one sorting scheme, placental stem cells can be sorted on the basis of expression of the markers CD34, CD38, CD44, CD45, CD73, CD105, OCT-4 and/or HLA-G, or any of the other markers listed elsewhere herein. This can be accomplished in connection with procedures to select stem cells on the basis of their adherence properties in culture. For example, adherence selection of placental stem cells can be accomplished before or after sorting on the basis of marker expression. In one embodiment, for example, placental stem cells can be sorted first on the basis of their expression of CD34; CD34⁻ cells are retained, and cells that are CD200⁺ or HLA-G⁺, are separated from all other CD34⁻ cells. In another embodiment, placental stem cells can be sorted based on their expression of CD200 and/or HLA-G, or lack thereof; for example, cells displaying either of these markers can be isolated for further use. Cells that express, *e*.*g*., CD200 and/or HLA-G can, in a specific embodiment, be further sorted based on their expression of CD73 and/or CD105, or epitopes recognized by antibodies SH2, SH3 or SH4, or lack of expression of CD34, CD38 or CD45. For example, in one embodiment, placental stem cells are sorted by expression, or lack thereof, of CD200, HLA-G, CD73, CD105, CD34, CD38 and CD45, and placental stem cells that are CD200⁺, HLA-G⁻, CD73⁺, CD105⁺, CD34⁻, CD38⁻ and CD45⁻ are isolated from other placental cells for further use.

In another embodiment, magnetic beads can be used to separate cells, *e*.*g*., separate placental stem cells from other placental cells. The cells may be sorted using a magnetic activated cell sorting (MACS) technique, a method for separating particles based on their ability to bind magnetic beads (0.5-100 µm diameter). A variety of useful modifications can be performed on the magnetic microspheres, including covalent addition of antibody that specifically recognizes a particular cell surface molecule or hapten. The beads are then mixed with the cells to allow binding. Cells are then passed through a magnetic field to separate out cells having the specific cell surface marker. In one embodiment, these cells can then isolated and re-mixed with magnetic beads coupled to an antibody against additional cell surface markers. The cells are again passed through a magnetic field, isolating cells that bound both the antibodies. Such cells can then be diluted into separate dishes, such as microtiter dishes for clonal isolation.

Placental stem cells can also be characterized and/or sorted based on cell morphology and growth characteristics. For example, placental stem cells can be characterized as having, and/or selected on the basis of, *e*.*g*., a fibroblastoid appearance in culture. Placental stem cells can also be characterized as having, and/or be selected, on the basis of their ability to form embryoid-like bodies. In one embodiment, for example, placental cells that are fibroblastoid in shape, express CD73 and CD105, and produce one or more embryoid-like bodies in culture can be isolated from other placental cells. In another embodiment, OCT-4⁺ placental cells that produce one or more embryoid-like bodies in culture are isolated from other placental cells.

In another embodiment, placental stem cells can be identified and characterized by a colony forming unit assay. Colony forming unit assays are commonly known in the art, such as Mesen Cult™ medium (Stem Cell Technologies, Inc., Vancouver British Columbia).

Placental stem cells can be assessed for viability, proliferation potential, and longevity using standard techniques known in the art, such as trypan blue exclusion assay, fluorescein diacetate uptake assay, propidium iodide uptake assay (to assess viability); and thymidine uptake assay, MTT cell proliferation assay (to assess proliferation). Longevity may be determined by methods well known in the art, such as by determining the maximum number of population doubling in an extended culture.

Placental stem cells can also be separated from other placental cells using other techniques known in the art, *e*.*g*., selective growth of desired cells (positive selection), selective destruction of unwanted cells (negative selection); separation based upon differential cell agglutinability in the mixed population as, for example, with soybean agglutinin; freeze-thaw procedures; filtration; conventional and zonal centrifugation; centrifugal elutriation (counter-streaming centrifugation); unit gravity separation; countercurrent distribution; electrophoresis; and the like.

### 5.5 CULTURE OF PLACENTAL STEM CELLS

### 5.5.1 Culture Media

Placental stem cells, including the arPSCs described herein, can be cultured in any medium, and under any conditions, recognized in the art as acceptable for the culture of stem cells. In certain embodiments, the culture medium comprises serum. In certain embodiments, placental stem cells, including the asPSCs described herein, can be cultured in, for example, DMEM-LG (Dulbecco's Modified Essential Medium, low glucose)/MCDB 201 (chick fibroblast basal medium) containing ITS (insulin-transferrin-selenium), LA+BSA (linoleic acid-bovine serum albumin), dextrose, L-ascorbic acid, PDGF, EGF, IGF-1, and penicillin/streptomycin; DMEM-HG (high glucose) comprising 10% fetal bovine serum (FBS); DMEM-HG comprising 15% FBS; IMDM (Iscove's modified Dulbecco's medium) comprising 10% FBS, 10% horse serum, and hydrocortisone; M199 comprising 10% FBS, EGF, and heparin; α-MEM (minimal essential medium) comprising 10% FBS, GlutaMAX™ and gentamicin; DMEM comprising 10% FBS, GlutaMAX™ and gentamicin, *etc.* A preferred medium is DMEM-LG/MCDB-201 comprising 2% FBS, ITS, LA+BSA, dextrose, L-ascorbic acid, PDGF, EGF, and penicillin/streptomycin.

Other media in that can be used to culture placental stem cells, including the asPSCs described herein, include DMEM (high or low glucose), Eagle's basal medium, Ham's F10 medium (F10), Ham's F-12 medium (F12), Iscove's modified Dulbecco's medium, Mesenchymal Stem Cell Growth Medium (MSCGM), Liebovitz's L-15 medium, MCDB, DMIEM/F12, RPMI 1640, advanced DMEM (Gibco), DMEM/MCDB201 (Sigma), and CELL-GRO FREE.

The culture medium can be supplemented with one or more components including, for example, serum (*e*.*g*., fetal bovine serum (FBS), preferably about 2-15% (v/v); equine (horse) serum (ES); human serum (HS)); beta-mercaptoethanol (BME), preferably about 0.001% (v/v); one or more growth factors, for example, platelet-derived growth factor (PDGF), epidermal growth factor (EGF), basic fibroblast growth factor (bFGF), insulin-like growth factor-1 (IGF-1), leukemia inhibitory factor (LIF), vascular endothelial growth factor (VEGF), and erythropoietin (EPO); amino acids, including L-valine; and one or more antibiotic and/or antimycotic agents to control microbial contamination, such as, for example, penicillin G, streptomycin sulfate, amphotericin B, gentamicin, and nystatin, either alone or in combination.

### 5.5.2 Expansion and Proliferation of Placental Stem Cells

Once placental stem cells, including the asPSCs described herein, are isolated (e.g., separated from at least 50% of the placental cells with which the stem cell or population of stem cells is normally associated *in vivo*), the stem cells or population of stem cells can be proliferated and expanded *in vitro.* For example, once anoikis resistant placental stem cells are produced, such cells can also be proliferated and expanded *in vitro.* Placental stem cells, including the asPSCs described herein, can be cultured in tissue culture containers, *e*.*g*., dishes, flasks, multiwell plates, or the like, for a sufficient time for the placental stem cells to proliferate to 70-90% confluence, that is, until the placental stem cells and their progeny occupy 70-90% of the culturing surface area of the tissue culture container.

Placental stem cells, including the asPSCs described herein, can be seeded in culture vessels at a density that allows cell growth. For example, the placental stem cells may be seeded at low density (*e*.*g*., about 1,000 to about 5,000 cells/cm²) to high density (*e*.*g*., about 50,000 or more cells/cm²). In a preferred embodiment, the placental stem cells are cultured at about 0 to about 5 percent by volume CO₂ in air. In some preferred embodiments, the placental stem cells are cultured at about 2 to about 25 percent O₂ in air, preferably about 5 to about 20 percent O₂ in air. The placental stem cells preferably are cultured at about 25°C to about 40°C, preferably 37°C. The placental stem cells are preferably cultured in an incubator. The culture medium can be static or agitated, for example, using a bioreactor. Placental stem cells can be grown under low oxidative stress (*e*.*g*., with addition of glutathione, ascorbic acid, catalase, tocopherol, N-acetylcysteine, or the like).

Once 70%-90% confluence is obtained, the placental stem cells, including the asPSCs described herein, may be passaged. For example, the cells can be enzymatically treated, *e*.*g*., trypsinized, using techniques well-known in the art, to separate them from the tissue culture surface. After removing the placental stem cells by pipetting and counting the cells, about 20,000-100,000 stem cells, preferably about 50,000 placental stem cells, are passaged to a new culture container containing fresh culture medium. Typically, the new medium is the same type of medium from which the stem cells were removed. Provided herein are populations of placental stem cells, including the asPSCs described herein, that have been passaged at least 1, 2, 3, 4, 5, 6, 7, 8, 9, 10, 12, 14, 16, 18, or 20 times, or more, and combinations of the same.

### 5.6 PRESERVATION OF ANOIKIS RESISTANT PLACENTAL CELLS

Anoikis resistant placental stem cells can be preserved, that is, placed under conditions that allow for long-term storage, or conditions that inhibit cell death by, *e*.*g*., apoptosis or necrosis.

Anoikis resistant placental stem cells can be preserved using, *e*.*g*., a composition comprising an apoptosis inhibitor, necrosis inhibitor and/or an oxygen-carrying perfluorocarbon, as described in related U.S. Provisional Application No. 60/754,969, entitled "Improved Composition for Collecting and Preserving Placental Cells and Methods of Using the Composition" filed on December 25, 2005.

In one embodiment, provided herein is a method of preserving anoikis resistant placental stem cells comprising contacting said anoikis resistant placental stem cells with a stem cell collection composition comprising an inhibitor of apoptosis and an oxygen-carrying perfluorocarbon, wherein said inhibitor of apoptosis is present in an amount and for a time sufficient to reduce or prevent apoptosis in the population of anoikis resistant placental stem cells, as compared to a population of anoikis resistant placental stem cells not contacted with the inhibitor of apoptosis. In a specific embodiment, said inhibitor of apoptosis is a caspase inhibitor. In another specific embodiment, said inhibitor of apoptosis is a JNK inhibitor. In a more specific embodiment, said JNK inhibitor does not modulate differentiation or proliferation of said anoikis resistant placental stem cells. In another embodiment, said stem cell collection composition comprises said inhibitor of apoptosis and said oxygen-carrying perfluorocarbon in separate phases. In another embodiment, said stem cell collection composition comprises said inhibitor of apoptosis and said oxygen-carrying perfluorocarbon in an emulsion. In another embodiment, the stem cell collection composition additionally comprises an emulsifier, *e*.*g*., lecithin. In another embodiment, said apoptosis inhibitor and said perfluorocarbon are between about 0°C and about 25°C at the time of contacting the stem cells. In another more specific embodiment, said apoptosis inhibitor and said perfluorocarbon are between about 2°C and 10°C, or between about 2°C and about 5°C, at the time of contacting the stem cells. In another more specific embodiment, said contacting is performed during transport of said anoikis resistant placental stem cells. In another more specific embodiment, said contacting is performed during freezing and thawing of said population of anoikis resistant placental stem cells.

In another embodiment, anoikis resistant placental stem cells can be preserved by a method comprising contacting said anoikis resistant placental stem cells with an inhibitor of apoptosis and an organ-preserving compound, wherein said inhibitor of apoptosis is present in an amount and for a time sufficient to reduce or prevent apoptosis of the anoikis resistant placental stem cells, as compared to anoikis resistant placental stem cells not contacted with the inhibitor of apoptosis. In a specific embodiment, the organ-preserving compound is UW solution (described in U.S. Patent No. 4,798,824; also known as ViaSpan; *see also* Southard et al., Transplantation 49(2):251-257 (1990)) or a solution described in Stern et al., U.S. Patent No. 5,552,267. In another embodiment, said organ-preserving compound is hydroxyethyl starch, lactobionic acid, raffinose, or a combination thereof.

In another embodiment, placental stem cells, to be used to produce anoikis resistant placental stem cells, are contacted with a stem cell collection composition comprising an apoptosis inhibitor and oxygen-carrying perfluorocarbon, organ-preserving compound, or combination thereof, during perfusion. In another embodiment, said placental stem cells, to be used to produce anoikis resistant placental stem cells, are contacted during a process of tissue disruption, *e*.*g*., enzymatic digestion. In another embodiment, placental cells, to be used to produce anoikis resistant placental stem cells, are contacted with said stem cell collection compound after collection by perfusion, or after collection by tissue disruption, *e*.*g*., enzymatic digestion.

Typically, during placental stem cell collection, enrichment and isolation, it is preferable to minimize or eliminate cell stress due to hypoxia and mechanical stress. In another embodiment of the method, therefore, placental stem cells, to be used to produce anoikis resistant placental stem cells, are exposed to a hypoxic condition during collection, enrichment or isolation for less than six hours during said preservation, wherein a hypoxic condition is a concentration of oxygen that is less than normal blood oxygen concentration. In a more specific embodiment, said placental stem cells are exposed to said hypoxic condition for less than two hours during said preservation. In another more specific embodiment, said placental stem cells are exposed to said hypoxic condition for less than one hour, or less than thirty minutes, or is not exposed to a hypoxic condition, during collection, enrichment or isolation. In another specific embodiment, said placental stem cells are not exposed to shear stress during collection, enrichment or isolation.

The anoikis resistant placental stem cells, as well as the placental stem cells to be used to produce anoikis resistant placental stem cells, described herein can be cryopreserved, *e*.*g*., in cryopreservation medium in small containers, *e*.*g*., ampoules. Suitable cryopreservation medium includes, but is not limited to, culture medium including, *e*.*g*., growth medium, or cell freezing medium, for example commercially available cell freezing medium, *e*.*g*., C2695, C2639 or C6039 (Sigma). Cryopreservation medium preferably comprises DMSO (dimethylsulfoxide), at a concentration of, *e*.*g*., about 10% (v/v). Cryopreservation medium may comprise additional agents, for example, Plasmalyte, methylcellulose with or without glycerol. The stem cells are preferably cooled at about 1°C/min during cryopreservation. A preferred cryopreservation temperature is about -80°C to about -180°C, preferably about -125°C to about -140°C. Cryopreserved cells can be transferred to liquid nitrogen prior to thawing for use. In some embodiments, for example, once the ampoules have reached about -90°C, they are transferred to a liquid nitrogen storage area. Cryopreserved cells preferably are thawed at a temperature of about 25°C to about 40°C, preferably to a temperature of about 37°C. In certain embodiments, anoikis resistant placental stem cells provided herein are cryopreserved about 12, 24, 36, 48, 60 or 72 hours after being contacted with modulatory RNA molecules (*e*.*g*., transfection). In one embodiment, anoikis resistant placental stem cells provided herein are cryopreserved about 24 hours after being contacted with modulatory RNA molecules (*e*.*g*., transfection).

### 5.7 COMPOSITIONS

### 5.7.1 Compositions Comprising Anoikis Resistant Placental Stem Cells

Provided herein are compositions comprising the anoikis resistant placental stem cells described herein. Such compositions may comprise populations of anoikis resistant placental stem cells provided herein combined with any physiologically-acceptable or medically-acceptable compound, composition or device for use in, *e*.*g*., research or therapeutics.

### 5.7.1.1 Cryopreserved Anoikis Resistant Placental Stem Cells

The anoikis resistant placental stem cells described herein can be preserved, for example, cryopreserved for later use. Methods for cryopreservation of cells, such as stem cells, are well known in the art. Anoikis resistant placental stem cells can be prepared in a form that is easily administrable to an individual. For example, anoikis resistant placental stem cells described herein can be contained within a container that is suitable for medical use. Such a container can be, for example, a sterile plastic bag, flask, jar, vial, or other container from which the placental cell population can be easily dispensed. For example, the container can be a blood bag or other plastic, medically-acceptable bag suitable for the intravenous administration of a liquid to a recipient. The container is preferably one that allows for cryopreservation of the anoikis resistant placental stem cells.

Cryopreserved anoikis resistant placental stem cell populations can comprise anoikis resistant placental stem cells derived from a single donor, or from multiple donors. The anoikis resistant placental stem cells can be completely HLA-matched to an intended recipient, or partially or completely HLA-mismatched.

Thus, in one embodiment, provided herein is a composition comprising anoikis resistant placental stem cells in a container. In a specific embodiment, the anoikis resistant placental stem cells cryopreserved. In another specific embodiment, the container is a bag, flask, vial or jar. In more specific embodiment, said bag is a sterile plastic bag. In a more specific embodiment, said bag is suitable for, allows or facilitates intravenous administration of said anoikis resistant placental stem cells. The bag can comprise multiple lumens or compartments that are interconnected to allow mixing of the anoikis resistant placental stem cells and one or more other solutions, *e*.*g*., a drug, prior to, or during, administration. In another specific embodiment, the composition comprises one or more compounds that facilitate cryopreservation of the combined stem cell population. In another specific embodiment, said anoikis resistant placental stem cells are contained within a physiologically-acceptable aqueous solution. In a more specific embodiment, said physiologically-acceptable aqueous solution is a 0.9% NaCl solution. In another specific embodiment, said anoikis resistant placental stem cells are HLA-matched to a recipient of said anoikis resistant placental stem cells. In another specific embodiment, said anoikis resistant placental stem cells are at least partially HLA-mismatched to a recipient of said anoikis resistant placental stem cells. In another specific embodiment, said anoikis resistant placental stem cells are derived from placental stem cells from a plurality of donors.

### 5.7.1.2 Pharmaceutical Compositions

In another aspect, provided herein is a pharmaceutical composition for treating an individual having or at risk of developing a disease, disorder or condition having an inflammatory component, said pharmaceutical composition comprising a therapeutically effective amount of anoikis resistant placental stem cells.

The anoikis resistant placental stem cells provided herein can be formulated into pharmaceutical compositions for use *in vivo.* Such pharmaceutical compositions can comprise anoikis resistant placental stem cells in a pharmaceutically-acceptable carrier, *e*.*g*., a saline solution or other accepted physiologically-acceptable solution for *in vivo* administration. Pharmaceutical compositions provided herein can comprise any of the anoikis resistant placental stem cells described herein. The pharmaceutical compositions can comprise fetal, maternal, or both fetal and maternal anoikis resistant placental stem cells. The pharmaceutical compositions provided herein can further comprise anoikis resistant placental stem cells produced from placental stem cells obtained from a single individual or placenta, or from a plurality of individuals or placentae.

The pharmaceutical compositions provided herein can comprise any number of anoikis resistant placental stem cells. For example, a single unit dose of anoikis resistant placental stem cells can comprise, in various embodiments, about, at least, or no more than 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, 1 x 10¹¹ or more anoikis resistant placental stem cells.

The pharmaceutical compositions provided herein can comprise populations of anoikis resistant placental stem cells that comprise 50% viable anoikis resistant placental stem cells or more (that is, at least 50% of the cells in the population are functional or living). Preferably, at least 60% of the cells in the population are viable. More preferably, at least 70%, 80%, 90%, 95%, or 99% of the anoikis resistant placental stem cells in the population in the pharmaceutical composition are viable.

### 5.7.1.3 Matrices Comprising Anoikis Resistant Placental Stem Cells

Further provided herein are matrices, hydrogels, scaffolds, and the like that comprise anoikis resistant placental stem cells. The anoikis resistant placental stem cells provided herein can be seeded onto a natural matrix, *e*.*g*., a placental biomaterial such as an amniotic membrane material. Such an amniotic membrane material can be, *e*.*g*., amniotic membrane dissected directly from a mammalian placenta; fixed or heat-treated amniotic membrane, substantially dry (*i*.*e*., <20% H₂O) amniotic membrane, chorionic membrane, substantially dry chorionic membrane, substantially dry amniotic and chorionic membrane, and the like. Preferred placental biomaterials on which anoikis resistant placental stem cells can be seeded are described in Hariri, U.S. Application Publication No. 2004/0048796.

The anoikis resistant placental stem cells provided herein can be suspended in a hydrogel solution suitable for, *e*.*g*., injection. Suitable hydrogels for such compositions include self-assembling peptides, such as RAD16. Anoikis resistant placental stem cells can also be combined with, *e*.*g*., alginate or platelet-rich plasma, or other fibrin-containing matrices, for local injection. In one embodiment, a hydrogel solution comprising anoikis resistant placental stem cells can be allowed to harden, for instance in a mold, to form a matrix having the cells dispersed therein for implantation. Anoikis resistant placental stem cells in such a matrix can also be cultured so that the cells are mitotically expanded prior to implantation. The hydrogel can be, *e*.*g*., an organic polymer (natural or synthetic) that is cross-linked via covalent, ionic, or hydrogen bonds to create a three-dimensional open-lattice structure that entraps water molecules to form a gel. Hydrogel-forming materials include polysaccharides such as alginate and salts thereof, peptides, polyphosphazines, and polyacrylates, which are crosslinked ionically, or block polymers such as polyethylene oxide-polypropylene glycol block copolymers which are crosslinked by temperature or pH, respectively. In some embodiments, the hydrogel or matrix is biodegradable.

In some embodiments, the matrix comprises an *in situ* polymerizable gel (*see*., *e*.*g*., U.S. Patent Application Publication 2002/0022676; Anseth et al., J. Control Release, 78(1-3):199-209 (2002); Wang et al., Biomaterials, 24(22):3969-80 (2003).

In some embodiments, the polymers are at least partially soluble in aqueous solutions, such as water, buffered salt solutions, or aqueous alcohol solutions, that have charged side groups, or a monovalent ionic salt thereof. Examples of polymers having acidic side groups that can be reacted with cations are poly(phosphazenes), poly(acrylic acids), poly(methacrylic acids), copolymers of acrylic acid and methacrylic acid, poly(vinyl acetate), and sulfonated polymers, such as sulfonated polystyrene. Copolymers having acidic side groups formed by reaction of acrylic or methacrylic acid and vinyl ether monomers or polymers can also be used. Examples of acidic groups are carboxylic acid groups, sulfonic acid groups, halogenated (preferably fluorinated) alcohol groups, phenolic OH groups, and acidic OH groups.

The anoikis resistant placental stem cells can be seeded onto a three-dimensional framework or scaffold and implanted *in vivo.* Such a framework can be implanted in combination with any one or more growth factors, cells, drugs or other components that stimulate tissue formation or otherwise enhance or improve the practice of the methods of treatment described elsewhere herein.

Examples of scaffolds that can be used herein include nonwoven mats, porous foams, or self assembling peptides. Nonwoven mats can be formed using fibers comprised of a synthetic absorbable copolymer of glycolic and lactic acids (*e*.*g*., PGA/PLA) (VICRYL, Ethicon, Inc., Somerville, N.J.). Foams, composed of, *e*.*g*., poly(ε-caprolactone)/poly(glycolic acid) (PCL/PGA) copolymer, formed by processes such as freeze-drying, or lyophilization (*see*, *e*.*g*., U.S. Pat. No. 6,355,699), can also be used as scaffolds.

In another embodiment, the scaffold is, or comprises, a nanofibrous scaffold, *e*.*g*., an electrospun nanofibrous scaffold. In a more specific embodiment, said nanofibrous scaffold comprises poly(L-lactic acid) (PLLA), type I collagen, a copolymer of vinylidene fluoride and trifluoroethylnee (PVDF-TrFE), poly(-caprolactone), poly(L-lactide-co-ε-caprolactone) [P(LLA-CL)] (*e*.*g*., 75:25), and/or a copolymer of poly(3-hydroxybutyrate-*co*-3-hydroxyvalerate) (PHBV) and type I collagen. Methods of producing nanofibrous scaffolds, *e*.*g*., electrospun nanofibrous scaffolds, are known in the art. *See*, *e*.*g*., Xu et al., Tissue Engineering 10(7):1160-1168 (2004); Xu et al., Biomaterials 25:877-886 (20040; Meng et al., J. Biomaterials Sci., Polymer Edition 18(1):81-94 (2007).

The anoikis resistant placental stem cells described herein can also be seeded onto, or contacted with, a physiologically-acceptable ceramic material including, but not limited to, mono-, di-, tri-, alpha-tri-, beta-tri-, and tetra-calcium phosphate, hydroxyapatite, fluoroapatites, calcium sulfates, calcium fluorides, calcium oxides, calcium carbonates, magnesium calcium phosphates, biologically active glasses such as BIOGLASS®, and mixtures thereof. Porous biocompatible ceramic materials currently commercially available include SURGIBONE® (CanMedica Corp., Canada), ENDOBON® (Merck Biomaterial France, France), CEROS® (Mathys, AG, Bettlach, Switzerland), and mineralized collagen bone grafting products such as HEALOS™ (DePuy, Inc., Raynham, MA) and VITOSS®, RHAKOSS™, and CORTOSS® (Orthovita, Malvern, Pa.). The framework can be a mixture, blend or composite of natural and/or synthetic materials.

In another embodiment, anoikis resistant placental stem cells can be seeded onto, or contacted with, a felt, which can be, *e*.*g*., composed of a multifilament yarn made from a bioabsorbable material such as PGA, PLA, PCL copolymers or blends, or hyaluronic acid.

The anoikis resistant placental stem cells described herein can, in another embodiment, be seeded onto foam scaffolds that may be composite structures. Such foam scaffolds can be molded into a useful shape. In some embodiments, the framework is treated, *e*.*g*., with 0.1M acetic acid followed by incubation in polylysine, PBS, and/or collagen, prior to inoculation of the anoikis resistant placental stem cells in order to enhance cell attachment. External surfaces of a matrix may be modified to improve the attachment or growth of cells and differentiation of tissue, such as by plasma-coating the matrix, or addition of one or more proteins (*e*.*g*., collagens, elastic fibers, reticular fibers), glycoproteins, glycosaminoglycans (*e*.*g*., heparin sulfate, chondroitin-4-sulfate, chondroitin-6-sulfate, dermatan sulfate, keratin sulfate, *etc*.), a cellular matrix, and/or other materials such as, but not limited to, gelatin, alginates, agar, agarose, and plant gums, and the like.

In some embodiments, the scaffold comprises, or is treated with, materials that render it non-thrombogenic. These treatments and materials may also promote and sustain endothelial growth, migration, and extracellular matrix deposition. Examples of these materials and treatments include but are not limited to natural materials such as basement membrane proteins such as laminin and Type IV collagen, synthetic materials such as EPTFE, and segmented polyurethaneurea silicones, such as PURSPAN™ (The Polymer Technology Group, Inc., Berkeley, Calif.). The scaffold can also comprise anti-thrombotic agents such as heparin; the scaffolds can also be treated to alter the surface charge (*e*.*g*., coating with plasma) prior to seeding with anoikis resistant placental stem cells.

### 6. EXAMPLES

### 6.1 EXAMPLE 1: IDENTIFICATION OF ANOIKIS ASSOCIATED GENES IN PLACENTAL STEM CELLS

The existence of anoikis associated genes in placental stem cells was determined using the Decode™ RNAi Viral Screening Library (Thermo Scientific) in accordance with manufacturer's instructions. Briefly, the assay utilizes RNAi-based lentiviral technology to incorporate shRNAmirs into the genes of the target host cell genome. Cells are transduced with the shRNAmirs, and can be selected for by cell sorting based on the expression of green fluorescent protein (GFP) by the shRNAmirs or using a puromycin assay (because the shRNAmirs contain a gene that confers puromycin resistance to transduced cells). Selective pressure is then applied to identify cells that survive the pressure, and thus express certain genes at increased or decreased levels as a survival phenotype. Such differentially expressed genes are identified by PCR amplification of the genomic DNA of the surviving cells, wherein the sequences of the shRNAmirs incorporated into specific genes (and that thus inhibit/downregulate the expression of those genes) are amplified. Accordingly, the specific genes implicated in conferring the survival phenotype can be identified.

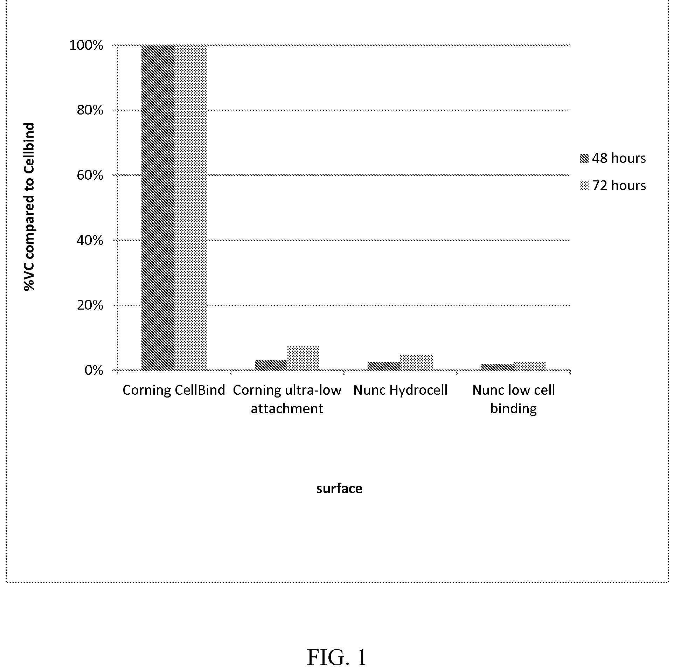

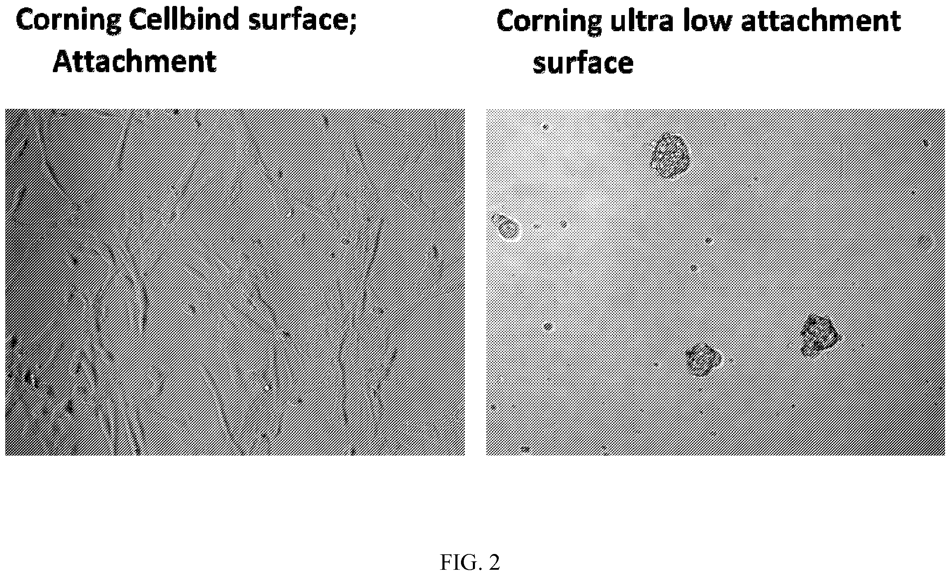

An anoikis assay for placental stem cells was first developed. It was determined that a suitable anoikis assay for placental stem cells that fulfilled the goal of having greater than 90% of unmodified placental stem cells dead or apoptotic as compared to the control unmodified placental stem cells (cultured under attachment conditions) consisted of the following: plating of placental stem cells at a concentration of 1 x 10⁵ cells/ml in DMEM supplemented with 0.1% FBS and culturing the cells at 37°C, 5% CO₂, for 48-72 hours on a control plate (which allows cell attachment) or on a low-attachment plates selected from Corning Ultra-Low Attachment, Nunc Hydrocell, or Nunc Low Cell Binding. Figure 1 demonstrates that unmodified placental stem cells exhibit very low survival under low attachment conditions after 48 hours of culture, whereas equivalent numbers of unmodified placental stem cells demonstrate near 100% survival under assay conditions that allow cell attachment. As shown in Figure 2, microscpopy confirmed that the placental stem cells cultured under attachment conditions (Corning CellBind plates) were viable and demonstrated morphology characteristic of placental stem cells after 72 hours of culture, whereas as the placental stem cells cultured under low-attachment conditions (Corning Ultra-Low Attachment plates) failed to survive after 72 hours of culture under comparable culture conditions (the exception being the attachment conditions).

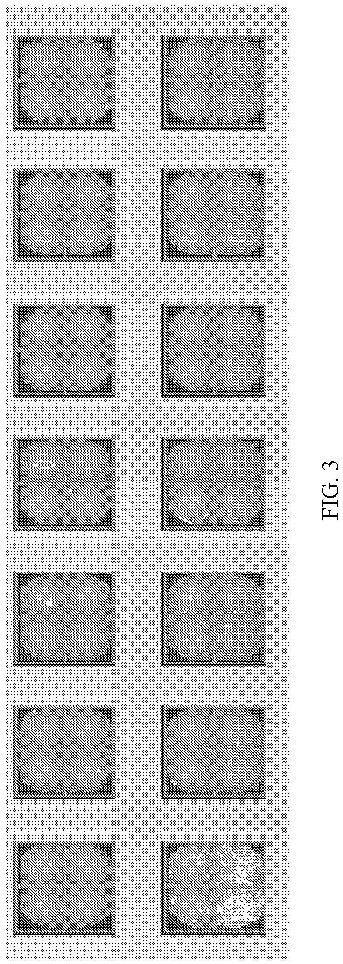

The established placental stem cell anoikis assay was used as the selective pressure in the Decode™ RNAi Viral Screening Library (Thermo Scientific). Briefly, placental stem cells were transduced with the Decode Viral Library at a MOI of 0.3 in serum-free DMEM with Polybrene according to the instructions of the manufacturer. Transduced cells were selected for using a FACS Aria (Becton Dickinson) cell sorter using GFP as the selectable marker. Next, the transduced placental stem cells were subjected to the optimized anoikis assay described above for selection of anoikis-resistant placental stem cells. Surviving cells (e.g., anoikis resistant placental stem cells) after 48-hours of culture in the anoikis assay were isolated by either single cell sorting (using FACS) or serial dilution of GFP+ cells. The isolated cells were expanded in 384-well plates to reach >500 cells per well. Figure 3 depicts wells comprising populations of expanded placental stem cells identified in the assay (the bright-colored markings in the well represent GFP positive cells). The gene expression profiles from 187 wells of cells (wells with strong GFP expression) were assessed to identify anoikis associated genes by isolating genomic DNA from the cells and subsequently PCR amplifying the barcode-containing fragments to facilitate sequence-based target gene identification performed. The anoikis associated genes comprise those that were inhibited/downregulated in the surviving cells and which thus were identified as being associated with the anoikis pathway in the placental stem cells.

Seventy-three genes were identified as having a role in placental stem cell anoikis, including the following genes: AMIGO1 (NCBI GENE ID NO:57463); ARHGAP20 (NCBI GENE ID NO:57569); CD38 (NCBI GENE ID NO:952); CLCC1 (NCBI GENE ID NO:23155); CNTF (NCBI GENE ID NO:1270); ZFP91-CNTF (NCBI GENE ID NO:386607); COX8A (NCBI GENE ID NO:1351); DHX34 (NCBI GENE ID NO:9704); FAM175A (NCBI GENE ID NO:NO 51023); MRPS18C (NCBI GENE ID NO:84142); FAM44C (NCBI GENE ID NO:284257); FBP2 (NCBI GENE ID NO:8789); FLI1 (NCBI GENE ID NO:2313); FREM3 (NCBI GENE ID NO:166752); IFIT5 (NCBI GENE ID NO:24138); LOC399851 (NCBI GENE ID NO:399851); LOC400713 (NCBI GENE ID NO:400713); LOC651610 (NCBI GENE ID NO:651610); PIGP (NCBI GENE ID NO:51227); SH3TC2 (NCBI GENE ID NO:79628); SLC2A3 (NCBI GENE ID NO:6515); STAU2 (NCBI GENE ID NO:27067) TMEFF1 (NCBI GENE ID NO:8577); TMEM217 (NCBI GENE ID NO:221468); TMEM79 (NCBI GENE ID NO:84283); USHBP1 (NCBI GENE ID NO:83878); APH1B (NCBI GENE ID NO:83464); ATP2B2 (NCBI GENE ID NO:491); C13orf39 (NCBI GENE ID NO:196541); C4orf17 (NCBI GENE ID NO:84103); C4orf46 (NCBI GENE ID NO:201725); DDX41 (NCBI GENE ID NO:51428); DKFZp547J222 (NCBI GENE ID NO:84237); FGFR1 (NCBI GENE ID NO:2260); FHDC1 (NCBI GENE ID NO:85462); GNAI2 (NCBI GENE ID NO:2771); GP5 (NCBI GENE ID NO:2814); IL1RN (NCBI GENE ID NO:3557); KIF24 (NCBI GENE ID NO:347240); KNDC1 (NCBI GENE ID NO:85442); LOC100132598 (NCBI GENE ID NO:100132598); LOC151760 (NCBI GENE ID NO: 151760); LOC152024 (NCBI GENE ID NO:152024); LOC339833 (NCBI GENE ID NO:339833); LPAR4 (NCBI GENE ID NO:2846); LSG1 (NCBI GENE ID NO:55341); MAP3K5 (NCBI GENE ID NO:4217); PDK3 (NCBI GENE ID NO:5165); PELI2 (NCBI GENE ID NO:57161); RNF103 (NCBI GENE ID NO:7844); SNX31 (NCBI GENE ID NO:169166); TXN2 (NCBI GENE ID NO:25828); and XKR7 (NCBI GENE ID NO:343702).

This Example demonstrates that placental stem cells undergo anoikis in low attachment conditions and that specific placental stem cell genes that cause anoikis in placental stem cells (anoikis associated genes) exist.

### 6.2 EXAMPLE 2: GENERATION OF ANOIKIS RESISTANT PLACENTAL STEM CELLS

Selected anoikis associated genes identified in Example 1 were targeted in placental stem cells using siRNA directed to the particular genes of interest. Placental stem cells were transfected using Dharmacon ON-TARGETplus SMARTpool siRNA specific to selected genes at a final siRNA concentration of 25nM, with Dharmafect 1 transfection reagent. Gene expression was analyzed using quantitative real-time PCR analysis was performed using 7900HT Fast Real-Time PCR System with TaqMan® Gene Expression kits to examine gene silencing efficiency.

Once it was confirmed that the siRNA specific to selected anoikis associated genes effectively inhibited/downregulated the expression of such genes, placental stem cells in which anoikis associated genes were targeted were cultured in the anoikis assay described in Example 1. The viability of these placental stem cells was assessed using the CellTiter AQueous One Solution Cell Proliferation Assay (MTS) and the CyQuant Direct assay, to determine whether anoikis resistant placental stem cells could be generated by specifically targeting anoikis associated genes in placental stem cells.

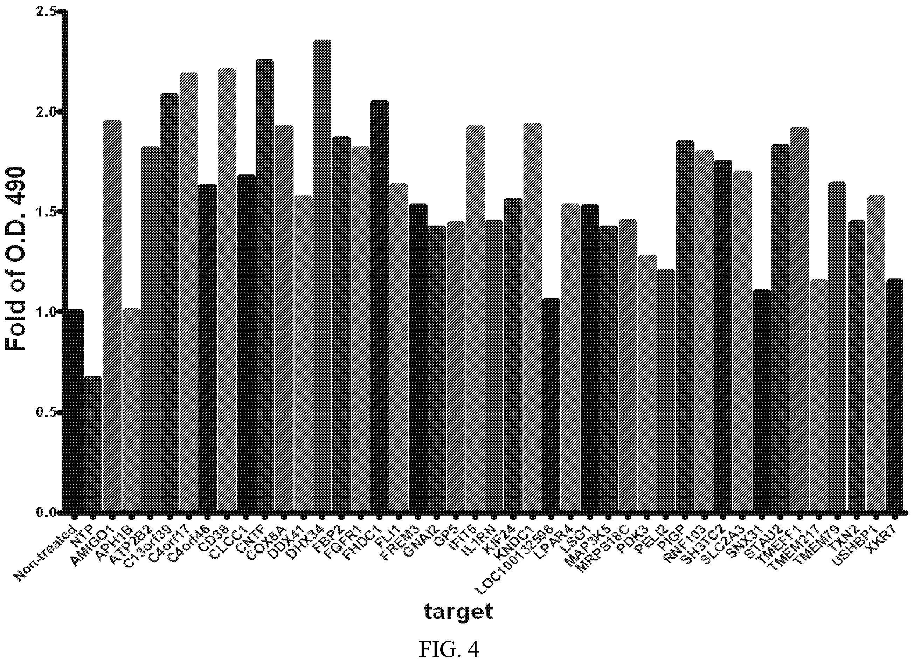

Figure 4 depicts the results of an MTS assay, wherein selected anoikis associated genes identified in Example 1 were inhibited/downregulated in placental stem cells using siRNA specific to the genes. The placental stem cells were subjected the anoikis assay described in Example 1 for 48 hours, and the viability of such cells was determined and compared to the viability of unmodified placental stem cells (placental stem cells not contacted with an siRNA specific to an anoikis associated gene; "Non-treated") and placental stem cells that were contacted with non-targeting pool siRNA ("NTP"), which is not specific to any of the anoikis associated genes identified herein.

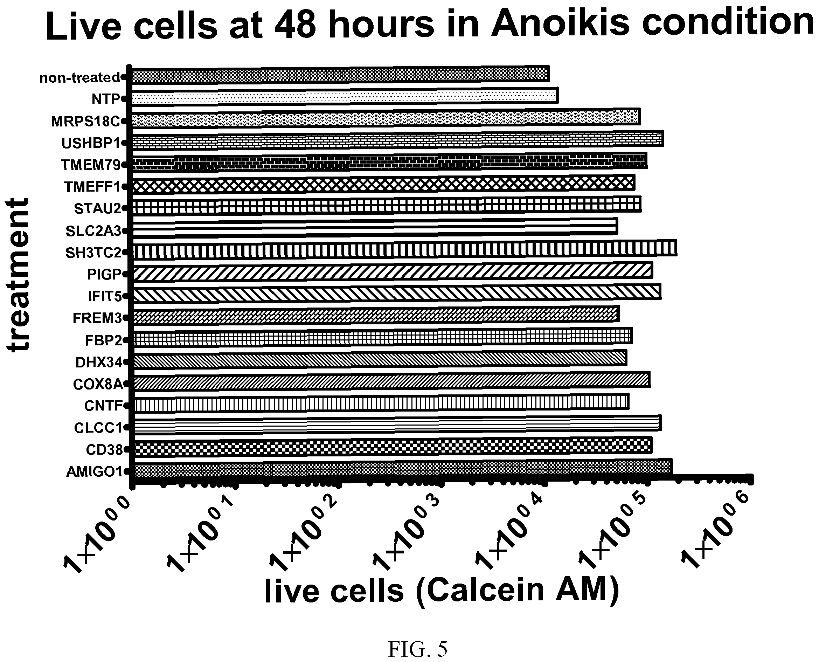

As shown in Figure 4, the targeting of numerous of the anoikis associated genes identified in Example 1 resulted in increased viability of placental stem cells as compared to the non-treated and NTP placental stem cell groups (in all cases, placental stem cells targeted with anoikis associated gene-specific siRNA demonstrated increased viability relative to the NTP placental stem cell group). The placental stem cells that exhibit increased viability following targeting of anoikis associated genes represent anoikis resistant placental stem cells (arPSCs), based on their increased ability to survive in low-attachment conditions as compared to unmodified placental stem cells. The CyQuant Direct viability assay verified that, under comparable conditions as the MTS assay, arPSCs could be generated by targeting anoikis associated genes in placental stem cells (Figure 5).

Further analyses were performed on selected anoikis associated genes, the inhibition of which in placental stem cells resulted in significant increases in placental stem cell viability in the anoikis assay (i.e., in low attachment conditions). In particular, the effects of inhibition of the following anoikis associated genes were further assessed: FH2 domain containing 1 (FHDC1: NCBI GENE ID NO:85462), guanine nucleotide binding protein alpha inhibiting 2 (GNAI2; NCBI GENE ID NO:2771), kinase non-catalytic C- lobe domain containing 1 (KNDC1; NCBI GENE ID NO:85442), lysophosphatidic acid receptor 4 (LPAR4; NCBI GENE ID NO:2846), mitogen-activated protein kinase kinase kinase 5 (MAP3K5; NCBI GENE ID NO:4217), solute carrier family 2, member 3 (SLC2A3; NCBI GENE ID NO:6515), and staufen homolog 2 (STAU2; NCBI GENE ID NO:27067).

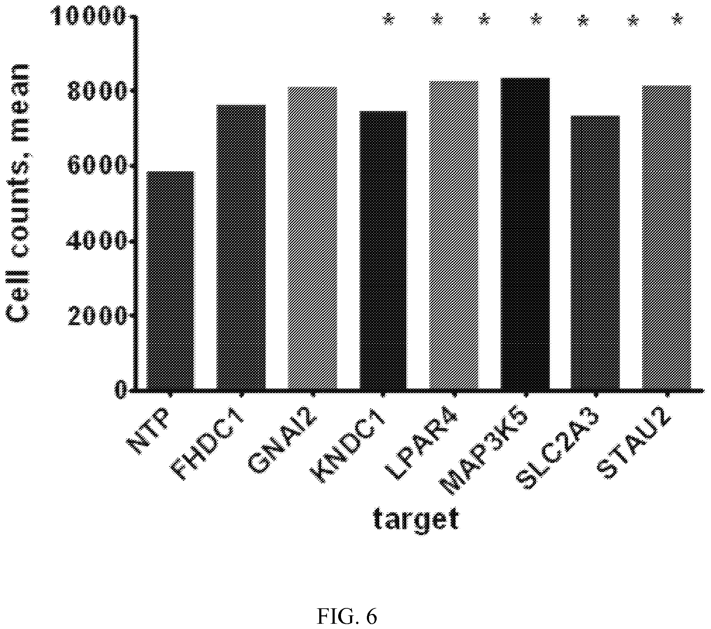

The CyQuant Direct viability assay confirmed that, after culturing for 48 hours in the anoikis assay described above, arPSCs could be generated by targeting anoikis associated genes in placental stem cells (Figure 6). The inhibition/downregulation of each anoikis associated gene assayed resulted in increased ability of the placental stem cells to survive in low-attachment conditions as compared to placental stem cells targeted with non-specific siRNA (NTP), with inhibition/downregulation of five of the seven genes tested resulting statistically significant increases in survival of the placental stem cells, confirming that the placental stem cells had become resistant to anoikis.

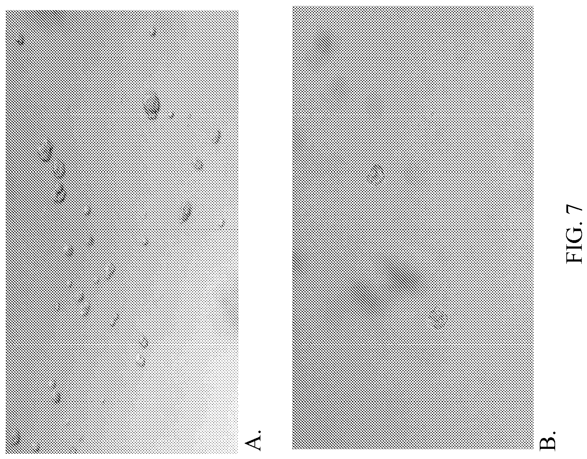

To further confirm viability of the anoikis resistant stem cells, an arPSC population wherein solute carrier family 2, member 3 (SLC2A3; NCBI GENE ID NO:6515) was inhibited/downregulated, and an equivalent amount of unmodified placental stem cells were separately cultured for 3 days under low attachment conditions. After the three day culture period, the two cell populations were visualized using microscopy. Figure 7 demonstrates that higher numbers of anoikis resistant placental stem cells remained viable after the culture period (Figure 7A) as compared to the number of viable unmodified placental stem cells (Figure 7B).

This Example demonstrates that placental stem cells can be made resistant to anoikis by targeting particular anoikis associated genes in the placental stem cells using approaches that modulate the expression of the anoikis associated genes, including targeting such genes with siRNA. The arPSCs generated in this Example can be advantageously used as therapeutics based on the fact that they do not require a substrate to adhere to in order to remain viable in vivo (for example, after systemic or local administration to a subject) and also may be advantageously used in the large-scale propagation of placental stem cells as suspension cultures.

### Equivalents:

The compositions and methods disclosed herein are not to be limited in scope by the specific embodiments described herein. Indeed, various modifications of the compositions and methods in addition to those described will become apparent to those skilled in the art from the foregoing description and accompanying figures. Such modifications are intended to fall within the scope of the appended claims.

Various publications, patents and patent applications are cited herein, the disclosures of which are incorporated by reference in their entireties.

The application discloses inter alia following items:
1. An isolated placental stem cell, wherein said placental stem cell is resistant to anoikis.
2. An isolated placental stem cell, wherein said placental stem cell expresses at least one anoikis associated gene at a decreased level as compared to the expression of the same anoikis associated gene in an unmodified placental stem cell.
3. The isolated placental stem cell of item 2, wherein said anoikis associated gene is AMIGO1 (NCBI GENE ID NO:57463); ARHGAP20 (NCBI GENE ID NO:57569); CD38 (NCBI GENE ID NO:952); CLCC1 (NCBI GENE ID NO:23155); CNTF (NCBI GENE ID NO:1270); ZFP91-CNTF (NCBI GENE ID NO:386607); COX8A (NCBI GENE ID NO:1351); DHX34 (NCBI GENE ID NO:9704); FAM175A (NCBI GENE ID NO:NO 51023); MRPS18C (NCBI GENE ID NO:84142); FAM44C (NCBI GENE ID NO:284257); FBP2 (NCBI GENE ID NO:8789); FLI1 (NCBI GENE ID NO:2313); FREM3 (NCBI GENE ID NO:166752); IFIT5 (NCBI GENE ID NO:24138); LOC399851 (NCBI GENE ID NO:399851); LOC400713 (NCBI GENE ID NO:400713); LOC651610 (NCBI GENE ID NO:651610); PIGP (NCBI GENE ID NO:51227); SH3TC2 (NCBI GENE ID NO:79628); SLC2A3 (NCBI GENE ID NO:6515); STAU2 (NCBI GENE ID NO:27067) TMEFF1 (NCBI GENE ID NO:8577); TMEM217 (NCBI GENE ID NO:221468); TMEM79 (NCBI GENE ID NO:84283); USHBP1 (NCBI GENE ID NO:83878); APH1B (NCBI GENE ID NO:83464); ATP2B2 (NCBI GENE ID NO:491); C13orf39 (NCBI GENE ID NO:196541); C4orf17 (NCBI GENE ID NO:84103); C4orf46 (NCBI GENE ID NO:201725); DDX41 (NCBI GENE ID NO:51428); DKFZp547J222 (NCBI GENE ID NO:84237); FGFR1 (NCBI GENE ID NO:2260); FHDC1 (NCBI GENE ID NO:85462); GNAI2 (NCBI GENE ID NO:2771); GP5 (NCBI GENE ID NO:2814); IL1RN (NCBI GENE ID NO:3557); KIF24 (NCBI GENE ID NO:347240); KNDC1 (NCBI GENE ID NO:85442); LOC100132598 (NCBI GENE ID NO:100132598); LOC151760 (NCBI GENE ID NO:151760); LOC152024 (NCBI GENE ID NO:152024); LOC339833 (NCBI GENE ID NO:339833); LPAR4 (NCBI GENE ID NO:2846); LSG1 (NCBI GENE ID NO:55341); MAP3K5 (NCBI GENE ID NO:4217); PDK3 (NCBI GENE ID NO:5165); PELI2 (NCBI GENE ID NO:57161); RNF103 (NCBI GENE ID NO:7844); SNX31 (NCBI GENE ID NO:169166); TXN2 (NCBI GENE ID NO:25828); or XKR7 (NCBI GENE ID NO:343702).
4. The isolated placental stem cell of item 3, wherein said anoikis associated gene is FHDC1 (NCBI GENE ID NO:85462),), GNAI2 (NCBI GENE ID NO:2771), KNDC1 (NCBI GENE ID NO:85442), LPAR4 (NCBI GENE ID NO:2846), MAP3K5 (NCBI GENE ID NO:4217), SLC2A3 (NCBI GENE ID NO:6515), or STAU2 (NCBI GENE ID NO:27067).
5. An isolated population of cells comprising anoikis resistant placental stem cells.
6. The isolated population of cells of item 5, wherein at least 50% of the cells in said population of cells are anoikis resistant placental stem cells.
7. The isolated population of cells of item 5, wherein at least 60%, at least 70%, at least 75%, at least 80%, and least 85%, at least 90%, at least 95%, or at least 99% of the cells in said population of cells are anoikis resistant placental stem cells.
8. The isolated population of cells of any one of items 5-7, wherein said anoikis resistant placental stem cells express at least one anoikis associated gene at a decreased level as compared to the expression of the same anoikis associated gene in an unmodified placental stem cell.
9. The isolated population of cells of item 8, wherein said anoikis associated gene is AMIGO1 (NCBI GENE ID NO:57463); ARHGAP20 (NCBI GENE ID NO:57569); CD38 (NCBI GENE ID NO:952); CLCC1 (NCBI GENE ID NO:23155); CNTF (NCBI GENE ID NO:1270); ZFP91-CNTF (NCBI GENE ID NO:386607); COX8A (NCBI GENE ID NO:1351); DHX34 (NCBI GENE ID NO:9704); FAM175A (NCBI GENE ID NO:NO 51023); MRPS18C (NCBI GENE ID NO:84142); FAM44C (NCBI GENE ID NO:284257); FBP2 (NCBI GENE ID NO:8789); FLI1 (NCBI GENE ID NO:2313); FREM3 (NCBI GENE ID NO:166752); IFIT5 (NCBI GENE ID NO:24138); LOC399851 (NCBI GENE ID NO:399851); LOC400713 (NCBI GENE ID NO:400713); LOC651610 (NCBI GENE ID NO:651610); PIGP (NCBI GENE ID NO:51227); SH3TC2 (NCBI GENE ID NO:79628); SLC2A3 (NCBI GENE ID NO:6515); STAU2 (NCBI GENE ID NO:27067) TMEFF1 (NCBI GENE ID NO:8577); TMEM217 (NCBI GENE ID NO:221468); TMEM79 (NCBI GENE ID NO:84283); USHBP1 (NCBI GENE ID NO:83878); APH1B (NCBI GENE ID NO:83464); ATP2B2 (NCBI GENE ID NO:491); C13orf39 (NCBI GENE ID NO:196541); C4orf17 (NCBI GENE ID NO:84103); C4orf46 (NCBI GENE ID NO:201725); DDX41 (NCBI GENE ID NO:51428); DKFZp547J222 (NCBI GENE ID NO:84237); FGFR1 (NCBI GENE ID NO:2260); FHDC1 (NCBI GENE ID NO:85462); GNAI2 (NCBI GENE ID NO:2771); GP5 (NCBI GENE ID NO:2814); IL1RN (NCBI GENE ID NO:3557); KIF24 (NCBI GENE ID NO:347240); KNDC1 (NCBI GENE ID NO:85442); LOC100132598 (NCBI GENE ID NO:100132598); LOC151760 (NCBI GENE ID NO:151760); LOC152024 (NCBI GENE ID NO:152024); LOC339833 (NCBI GENE ID NO:339833); LPAR4 (NCBI GENE ID NO:2846); LSG1 (NCBI GENE ID NO:55341); MAP3K5 (NCBI GENE ID NO:4217); PDK3 (NCBI GENE ID NO:5165); PELI2 (NCBI GENE ID NO:57161); RNF103 (NCBI GENE ID NO:7844); SNX31 (NCBI GENE ID NO:169166); TXN2 (NCBI GENE ID NO:25828); or XKR7 (NCBI GENE ID NO:343702).
10. The isolated population of cells of item 9, wherein said anoikis associated gene is FHDC1 (NCBI GENE ID NO:85462),), GNAI2 (NCBI GENE ID NO:2771), KNDC1 (NCBI GENE ID NO:85442), LPAR4 (NCBI GENE ID NO:2846), MAP3K5 (NCBI GENE ID NO:4217), SLC2A3 (NCBI GENE ID NO:6515), or STAU2 (NCBI GENE ID NO:27067).
11. A method of producing anoikis resistant placental stem cells comprising contacting the placental stem cells with an effective amount of modulatory RNA molecules, such that said placental stem cells, after having been contacted with said modulatory RNA molecules (i) express at least one anoikis associated gene at a decreased level as compared to the expression of the same anoikis associated gene in an equivalent amount of placental stem cells not contacted with said modulatory RNA molecules and/or (ii) survive in a low-attachment environment for a longer duration of time than an equivalent amount of placental stem cells not contacted with said modulatory RNA molecules.
12. The method of item 11, wherein said modulatory RNA molecules comprise small interfering RNAs (siRNAs), microRNA inhibitors (miR inhibitors), micro RNA mimics (miR mimics), antisense RNAs, short hairpin RNAs (shRNAs), or any combinations thereof.
13. The method of item 11 or 12, wherein said modulatory RNA molecules target at least one anoikis associated gene of said placental stem cells.
14. The method of item 13, wherein said anoikis associated gene is AMIGO1 (NCBI GENE ID NO:57463); ARHGAP20 (NCBI GENE ID NO:57569); CD38 (NCBI GENE ID NO:952); CLCC1 (NCBI GENE ID NO:23155); CNTF (NCBI GENE ID NO:1270); ZFP91-CNTF (NCBI GENE ID NO:386607); COX8A (NCBI GENE ID NO:1351); DHX34 (NCBI GENE ID NO:9704); FAM175A (NCBI GENE ID NO:NO 51023); MRPS18C (NCBI GENE ID NO:84142); FAM44C (NCBI GENE ID NO:284257); FBP2 (NCBI GENE ID NO:8789); FLI1 (NCBI GENE ID NO:2313); FREM3 (NCBI GENE ID NO:166752); IFIT5 (NCBI GENE ID NO:24138); LOC399851 (NCBI GENE ID NO:399851); LOC400713 (NCBI GENE ID NO:400713); LOC651610 (NCBI GENE ID NO:651610); PIGP (NCBI GENE ID NO:51227); SH3TC2 (NCBI GENE ID NO:79628); SLC2A3 (NCBI GENE ID NO:6515); STAU2 (NCBI GENE ID NO:27067) TMEFF1 (NCBI GENE ID NO:8577); TMEM217 (NCBI GENE ID NO:221468); TMEM79 (NCBI GENE ID NO:84283); USHBP1 (NCBI GENE ID NO:83878); APH1B (NCBI GENE ID NO:83464); ATP2B2 (NCBI GENE ID NO:491); C13orf39 (NCBI GENE ID NO:196541); C4orf17 (NCBI GENE ID NO:84103); C4orf46 (NCBI GENE ID NO:201725); DDX41 (NCBI GENE ID NO:51428); DKFZp547J222 (NCBI GENE ID NO:84237); FGFR1 (NCBI GENE ID NO:2260); FHDC1 (NCBI GENE ID NO:85462); GNAI2 (NCBI GENE ID NO:2771); GP5 (NCBI GENE ID NO:2814); IL1RN (NCBI GENE ID NO:3557); KIF24 (NCBI GENE ID NO:347240); KNDC1 (NCBI GENE ID NO:85442); LOC100132598 (NCBI GENE ID NO:100132598); LOC151760 (NCBI GENE ID NO:151760); LOC152024 (NCBI GENE ID NO:152024); LOC339833 (NCBI GENE ID NO:339833); LPAR4 (NCBI GENE ID NO:2846); LSG1 (NCBI GENE ID NO:55341); MAP3K5 (NCBI GENE ID NO:4217); PDK3 (NCBI GENE ID NO:5165); PELI2 (NCBI GENE ID NO:57161); RNF103 (NCBI GENE ID NO:7844); SNX31 (NCBI GENE ID NO:169166); TXN2 (NCBI GENE ID NO:25828); or XKR7 (NCBI GENE ID NO:343702).
15. The method of item 14, wherein said anoikis associated gene is FHDC1 (NCBI GENE ID NO:85462),), GNAI2 (NCBI GENE ID NO:2771), KNDC1 (NCBI GENE ID NO:85442), LPAR4 (NCBI GENE ID NO:2846), MAP3K5 (NCBI GENE ID NO:4217), SLC2A3 (NCBI GENE ID NO:6515), or STAU2 (NCBI GENE ID NO:27067).
16. An isolated anoikis resistant placental stem cell or population thereof produced by the method of any one of items 11-15.
17. A composition comprising the isolated anoikis resistant placental stem cell of item 1.
18. A composition comprising an isolated anoikis resistant placental stem cell produced by the method of any one of items 11-15.
19. The composition of item 18, wherein said anoikis resistant placental stem cell (i) expresses at least one anoikis associated gene at a decreased level as compared to the expression of the same anoikis associated gene in a placental stem cell not contacted with said modulatory RNA molecules and/or (ii) survives in a low-attachment environment for a longer duration of time than a placental stem cell not contacted with said modulatory RNA molecules.
20. A composition comprising enhanced placental stem cells, wherein said enhanced placental stem cells have been modified by the method of item 19.
21. The placental stem cell of any one of items 1-4 or 16, wherein said placental stem cell is a CD10+, CD34-, CD105+, CD200+ placental stem cell.
22. The population of cells of any one of items 5-10, wherein said anoikis resistant placental stem cells in said population are CD10+, CD34-, CD105+, CD200+ placental stem cells.
23. The method of any one of items 11-15, wherein said anoikis resistant placental stem cells are CD10+, CD34-, CD105+, CD200+ placental stem cells.
24. The composition of any one of items 17-20, wherein said anoikis resistant placental stem cells are CD10+, CD34-, CD105+, CD200+ placental stem cells.

## Claims

1. An isolated placental stem cell, wherein said placental stem cell is resistant to anoikis, wherein said anokis resistant placental stem cell expresses at least one anoikis associated gene at a decreased level as compared to the expression of the same anoikis associated gene in an unmodified placental stem cell, wherein said anoikis associated gene is AMIGO1 (NCBI GENE ID NO:57463); ARHGAP20 (NCBI GENE ID NO:57569); CD38 (NCBI GENE ID NO:952); CLCC1 (NCBI GENE ID NO:23155); CNTF (NCBI GENE ID NO:1270); ZFP91-CNTF (NCBI GENE ID NO:386607); COX8A (NCBI GENE ID NO:1351); DHX34 (NCBI GENE ID NO:9704); FAM175A (NCBI GENE ID NO:NO 51023); MRPS18C (NCBI GENE ID NO:84142); FAM44C (NCBI GENE ID NO:284257); FBP2 (NCBI GENE ID NO:8789); FLI1 (NCBI GENE ID NO:2313); FREM3 (NCBI GENE ID NO:166752); IFIT5 (NCBI GENE ID NO:24138); LOC399851 (NCBI GENE ID NO:399851); LOC400713 (NCBI GENE ID NO:400713); LOC651610 (NCBI GENE ID NO:651610); PIGP (NCBI GENE ID NO:51227); SH3TC2 (NCBI GENE ID NO:79628); SLC2A3 (NCBI GENE ID NO:6515); STAU2 (NCBI GENE ID NO:27067) TMEFF1 (NCBI GENE ID NO:8577); TMEM217 (NCBI GENE ID NO:221468); TMEM79 (NCBI GENE ID NO:84283); USHBP1 (NCBI GENE ID NO:83878); APH1B (NCBI GENE ID NO:83464); ATP2B2 (NCBI GENE ID NO:491); C13orf39 (NCBI GENE ID NO:196541); C4orf17 (NCBI GENE ID NO:84103); C4orf46 (NCBI GENE ID NO:201725); DDX41 (NCBI GENE ID NO:51428); DKFZp547J222 (NCBI GENE ID NO:84237); FGFR1 (NCBI GENE ID NO:2260); FHDC1 (NCBI GENE ID NO:85462); GNAI2 (NCBI GENE ID NO:2771); GP5 (NCBI GENE ID NO:2814); IL1RN (NCBI GENE ID NO:3557); KIF24 (NCBI GENE ID NO:347240); KNDC1 (NCBI GENE ID NO:85442); LOC100132598 (NCBI GENE ID NO:100132598); LOC151760 (NCBI GENE ID NO:151760); LOC152024 (NCBI GENE ID NO:152024); LOC339833 (NCBI GENE ID NO:339833); LPAR4 (NCBI GENE ID NO:2846); LSG1 (NCBI GENE ID NO:55341); MAP3K5 (NCBI GENE ID NO:4217); PDK3 (NCBI GENE ID NO:5165); PELI2 (NCBI GENE ID NO:57161); RNF103 (NCBI GENE ID NO:7844); SNX31 (NCBI GENE ID NO:169166); TXN2 (NCBI GENE ID NO:25828); or XKR7 (NCBI GENE ID NO:343702).

2. The placental stem cell of claim 1, wherein said placental stem cell is a CD10+, CD34-, CD105+, CD200+ placental stem cell.

3. An isolated population of cells comprising anoikis resistant placental stem cells, wherein said anoikis resistant placental stem cell expresses at least one anoikis associated gene at a decreased level as compared to the expression of the same anoikis associated gene in an unmodified placental stem cell, wherein said anoikis associated gene is AMIGO1 (NCBI GENE ID NO:57463); ARHGAP20 (NCBI GENE ID NO:57569); CD38 (NCBI GENE ID NO:952); CLCC1 (NCBI GENE ID NO:23155); CNTF (NCBI GENE ID NO:1270); ZFP91-CNTF (NCBI GENE ID NO:386607); COX8A (NCBI GENE ID NO:1351); DHX34 (NCBI GENE ID NO:9704); FAM175A (NCBI GENE ID NO:NO 51023); MRPS18C (NCBI GENE ID NO:84142); FAM44C (NCBI GENE ID NO:284257); FBP2 (NCBI GENE ID NO:8789); FLI1 (NCBI GENE ID NO:2313); FREM3 (NCBI GENE ID NO:166752); IFIT5 (NCBI GENE ID NO:24138); LOC399851 (NCBI GENE ID NO:399851); LOC400713 (NCBI GENE ID NO:400713); LOC651610 (NCBI GENE ID NO:651610); PIGP (NCBI GENE ID NO:51227); SH3TC2 (NCBI GENE ID NO:79628); SLC2A3 (NCBI GENE ID NO:6515); STAU2 (NCBI GENE ID NO:27067) TMEFF1 (NCBI GENE ID NO:8577); TMEM217 (NCBI GENE ID NO:221468); TMEM79 (NCBI GENE ID NO:84283); USHBP1 (NCBI GENE ID NO:83878); APH1B (NCBI GENE ID NO:83464); ATP2B2 (NCBI GENE ID NO:491); C13orf39 (NCBI GENE ID NO:196541); C4orf17 (NCBI GENE ID NO:84103); C4orf46 (NCBI GENE ID NO:201725); DDX41 (NCBI GENE ID NO:51428); DKFZp547J222 (NCBI GENE ID NO:84237); FGFR1 (NCBI GENE ID NO:2260); FHDC1 (NCBI GENE ID NO:85462); GNAI2 (NCBI GENE ID NO:2771); GP5 (NCBI GENE ID NO:2814); IL1RN (NCBI GENE ID NO:3557); KIF24 (NCBI GENE ID NO:347240); KNDC1 (NCBI GENE ID NO:85442); LOC100132598 (NCBI GENE ID NO:100132598); LOC151760 (NCBI GENE ID NO:151760); LOC152024 (NCBI GENE ID NO:152024); LOC339833 (NCBI GENE ID NO:339833); LPAR4 (NCBI GENE ID NO:2846); LSG1 (NCBI GENE ID NO:55341); MAP3K5 (NCBI GENE ID NO:4217); PDK3 (NCBI GENE ID NO:5165); PELI2 (NCBI GENE ID NO:57161); RNF103 (NCBI GENE ID NO:7844); SNX31 (NCBI GENE ID NO:169166); TXN2 (NCBI GENE ID NO:25828); or XKR7 (NCBI GENE ID NO:343702).

4. The isolated population of cells of claim 3, wherein at least 50% of the cells in said population of cells are anoikis resistant placental stem cells.

5. The isolated population of cells of claim 3, wherein at least 60%, at least 70%, at least 75%, at least 80%, and least 85%, at least 90%, at least 95%, or at least 99% of the cells in said population of cells are anoikis resistant placental stem cells.

6. The isolated population of cells of any one of claims 3-5, wherein said anoikis resistant placental stem cells in said population are CD10+, CD34-, CD105+, CD200+ placental stem cells.

7. A method of producing anoikis resistant placental stem cells comprising contacting the placental stem cells with an effective amount of modulatory RNA molecules, such that said placental stem cells, after having been contacted with said modulatory RNA molecules (i) express at least one anoikis associated gene at a decreased level as compared to the expression of the same anoikis associated gene in an equivalent amount of placental stem cells not contacted with said modulatory RNA molecules and/or (ii) survive in a low-attachment environment for a longer duration of time than an equivalent amount of placental stem cells not contacted with said modulatory RNA molecules, wherein said modulatory RNA molecules target at least one anoikis associated gene of said placental stem cells, and wherein said anoikis associated gene is AMIGO1 (NCBI GENE ID NO:57463); ARHGAP20 (NCBI GENE ID NO:57569); CD38 (NCBI GENE ID NO:952); CLCC1 (NCBI GENE ID NO:23155); CNTF (NCBI GENE ID NO:1270); ZFP91-CNTF (NCBI GENE ID NO:386607); COX8A (NCBI GENE ID NO:1351); DHX34 (NCBI GENE ID NO:9704); FAM175A (NCBI GENE ID NO:NO 51023); MRPS18C (NCBI GENE ID NO:84142); FAM44C (NCBI GENE ID NO:284257); FBP2 (NCBI GENE ID NO:8789); FLI1 (NCBI GENE ID NO:2313); FREM3 (NCBI GENE ID NO:166752); IFIT5 (NCBI GENE ID NO:24138); LOC399851 (NCBI GENE ID NO:399851); LOC400713 (NCBI GENE ID NO:400713); LOC651610 (NCBI GENE ID NO:651610); PIGP (NCBI GENE ID NO:51227); SH3TC2 (NCBI GENE ID NO:79628); SLC2A3 (NCBI GENE ID NO:6515); STAU2 (NCBI GENE ID NO:27067) TMEFF1 (NCBI GENE ID NO:8577); TMEM217 (NCBI GENE ID NO:221468); TMEM79 (NCBI GENE ID NO:84283); USHBP1 (NCBI GENE ID NO:83878); APH1B (NCBI GENE ID NO:83464); ATP2B2 (NCBI GENE ID NO:491); C13orf39 (NCBI GENE ID NO:196541); C4orf17 (NCBI GENE ID NO:84103); C4orf46 (NCBI GENE ID NO:201725); DDX41 (NCBI GENE ID NO:51428); DKFZp547J222 (NCBI GENE ID NO:84237); FGFR1 (NCBI GENE ID NO:2260); FHDC1 (NCBI GENE ID NO:85462); GNAI2 (NCBI GENE ID NO:2771); GP5 (NCBI GENE ID NO:2814); IL1RN (NCBI GENE ID NO:3557); KIF24 (NCBI GENE ID NO:347240); KNDC1 (NCBI GENE ID NO:85442); LOC100132598 (NCBI GENE ID NO:100132598); LOC151760 (NCBI GENE ID NO:151760); LOC152024 (NCBI GENE ID NO:152024); LOC339833 (NCBI GENE ID NO:339833); LPAR4 (NCBI GENE ID NO:2846); LSG1 (NCBI GENE ID NO:55341); MAP3K5 (NCBI GENE ID NO:4217); PDK3 (NCBI GENE ID NO:5165); PELI2 (NCBI GENE ID NO:57161); RNF103 (NCBI GENE ID NO:7844); SNX31 (NCBI GENE ID NO:169166); TXN2 (NCBI GENE ID NO:25828); or XKR7 (NCBI GENE ID NO:343702).

8. The method of claim 7, wherein said modulatory RNA molecules comprise small interfering RNAs (siRNAs), microRNA inhibitors (miR inhibitors), micro RNA mimics (miR mimics), antisense RNAs, short hairpin RNAs (shRNAs), or any combinations thereof.

9. The method of claim 7 or 8, wherein said anoikis resistant placental stem cells are CD10+, CD34-, CD105+, CD200+ placental stem cells.

10. An isolated anoikis resistant placental stem cell or population of isolated anoikis resistant placental stem cells produced by the method of any one of claims 7 to 9.

11. A composition comprising the isolated anoikis resistant placental stem cell of claim 1.

12. A composition comprising the isolated anoikis resistant placental stem cell or population of isolated anoikis resistant placental stem cells of claim 10.

13. The composition of any one of claims 11 to 12, wherein said anoikis resistant placental stem cells are CD10+, CD34-, CD105+, CD200+ placental stem cells.
